# EUROPEAN PATENT SPECIFICATION

(11) **EP 2 049 508 B1**
(45) Date of publication and mention of the grant of the patent: **09.06.2010**
(21) Application number: 07727569.1
(22) Date of filing: 30.03.2007
(51) Int. Cl.: C07D 295/10, C07D 211/58, C07D 295/18, C07D 295/22, C07D 295/20, C07D 211/46, C07D 213/54, A61K 31/495, A61K 31/4468, A61K 31/5375, A61P 35/00

(54) **N-HYDROXY-3-(4-{3-PHENYL-3-OXO-PROPENYL}-PHENYL)-ACRYLAMIDE DERIVATIVES AND RELATED COMPOUNDS AS HISTONE DEACETYLASE INHIBITORS FOR THE TREATMENT OF CANCER**
N-HYDROXY-3-(4-{3-PHENYL-3-OXOPROPENYL}PHENYL)ACRYLAMIDDERIVATE UND VERWANDTE VERBINDUNGEN ALS HISTONDEACETYLASEINHIBITOREN ZUR BEHANDLUNG VON KREBS
DÉRIVÉS DE N-HYDROXY-3-(4-{3-PHÉNYL-3-OXO-PROPÉNYL}-PHÉNYL)-ACRYLAMIDE ET COMPOSÉS SIMILAIRES EN TANT QU'INHIBITEURS DE L'HISTONE DÉSACÉTYLASE POUR LE TRAITEMENT DU CANCER

(30) Priority: 31.03.2006 IT MI20060621
(43) Date of publication of application: 22.04.2009
(73) Proprietor: DAC S.r.l., 20121 Milano (IT)
(72) Inventor: MAI, Antonello, I-00137 Roma (IT); MINUCCI, Saverio, I-20090 Noverasco Di Opera (IT); THALER, Florian, I-21040 Gerenzano (IT); PAIN, Gilles, I-04021 Castelforte (IT); COLOMBO, Andrea, I-20015 Parabiago (IT); GAGLIARDI, Stefania, I-20059 Vimercate (IT)
(74) Representative: Gervasi, Gemma
(86) International application number: PCT/EP2007/053097
(87) International publication number: WO 2007/113249

(56) References cited:
- WO-A-2005/040101
- WO-A-2005/040161
- WO-A-2006/037761

## Description

### FIELD OF THE INVENTION

The present invention relates to the field of histone deacetylases (HDACs) inhibiting compounds and methods for using the compounds to treat diseases linked to the disregulation of histone deacetylases activity, in particular for the treatment of cancer.

### PRIOR ART

The reversible acetylation of the ε-amino groups of several lysine residues in the N-terminal histone tails mediates important conformational modifications in nucleosomes. These modifications influence the access of transcription factor to DNA and regulate gene expression (Davie, J.R. Curr. Opin. Genet. Dev. 1998, 8, 173-178). Two enzyme classes are involved in the process of acetylation and deacetylation of histones: histone acetyltransferases (HAT), which catalyse histone acetylation by acting as transcriptional co-activators, and histone deacetylases (HDAC).

After their recruitment to the promoter regions induced by transcription repressors and co-repressors such as Sin3, SMRT and N-CoR, histone deacetylases induce the formation of hypoacetylated histones and ultimately lead to transcriptional silencing (Wu, J. et al. Trends Biochem. Sci. 2000, 25, 619-623). The aberrant recruitment of histone deacetylases by oncogene proteins, or the disruption of the equilibrium between the activities of histone acetyltransferases and histone deacetylases are implicated in a series of pathologies, including:
1. Primarily, cancer (Lin, R.J. et al. Oncogene 2001, 20, 7204-7215; Kastner, P. et al. Oncogene 2001, 20, 7186-7203; Pandolfi, P. et al. Oncogene 2001, 20, 3116-3127; Grignani, F. et al. Nature 1998, 391, 815-818; Lutterbach, B. et al. Mol. Cell. Biol. 1998, 18, 7176-7184).
2. Non-tumor diseases:
   - Nervous system: Huntington's disease (Ferrante, R.J. et al. J. Neurosci. 2003, 23, 9418-9427; Hockey, E. et al. Proc. Natl. Acad. Sci. USA 2003, 100, 2041-2046);
   - diseases caused by triplet expansions (Bodai, L. et al. Curr. Med. Chem. 2003, 10, 2577-2587; Hughes, R.E. Curr. Biol. 2002, 12, R141-143);
   - neurodegenerative disorders (Jeong, M.R. et al. FEBS Lett. 2003, 542, 74-78);
   - ischemia (Ming, R. et al. J. Neurochem. 2004, 89, 1358-1367);
   - oxidative stress (Ryu, H. et al. Proc. Natl. Acad. Sci. USA 2003, 100, 4281-4286);
   - inflammatory responses of the nervous system (Suuronen, T. J. Neurochem. 2003, 87, 407-416);
   - epilepsy (Eyal, S. et al. Epilepsia 2004, 45, 737-744; Huang, Y. et al. J. Neurosci. 2002, 22, 8422-8428);
   - diseases caused by protein aggregates (Corcoran, L.J. et al. Curr. Biol. 2004, 14, 488-492);
   - Psychic diseases: bipolar disorders (Williams, R.S.B. et al. Nature 2002, 417, 292-295);
   - cognitive disorders (Levenson, J.M. US20060018921);
   - psychiatric disorders (Costa, E. et al. Crit. Rev. Neurobiol. 2003, 15, 121-142);
   - fragile X syndrome (Chandler, S.P. et al. BMC Mol. Biol. 2003, 4, 3; Chiurazzi, P. et al. Hum. Mol. Genet. 1999, 8, 2317-2323).
   - Infections: HIV (Adam, E. et al. Mol. Cell. Biol. 2003, 23, 6200-6209; Van Lint, C. et al. Embo J. 1996, 15, 1112-1120; Demonte, D. et al. Biochem. Pharmacol. 2004, 68, 1231-1238; Ylisastigui, L. et al. Aids 2004, 18, 1101-1108); malaria, leishmaniasis, infections by protozoa, fungi, phytotoxic agents, viruses and parasites.
   - Immune system: autoimmune diseases (Skov, S. et al. Blood 2003, 101, 1430-1438); chronic immune reactions against the host (Reddy, P. et a/. Proc. Natl. Acad. Sci. USA 2004, 101, 3921-3926).
   - The heart: hypertrophy and cardiac decompensation (Kook, H. et al. J. Clin. Invest. 2003, 112, 863-871; McKinsey, T.A. et al. Novartis Found. Symp. 2004, 259, 132-141, discussion 141-145, 163-169; Hamamori, Y. et a/. J. Clin. Invest. 2003, 112, 824-826).
   - Muscular system: fibrotic skin disease (Rombouts, K. et al. Exp. Cell. Res. 2002, 278, 184-197); fibrosis (Niki, T. et al. Hepatology 1999, 29, 858-867); spinal and bulbar muscular atrophy (Minamiyama, M. et al. Hum. Mol. Genet. 2004, 13, 1183-1192).

Others: arthritis (Chung, Y.L. et al. Mol. Ther. 2003, 8, 707-717); hyperlipidemia (Crestani, M. et al. WO05/105066); kidney diseases (Mishra, N. et al. J. Clin. Invest. 2003, 111, 539-552); psoriasis (McLaughlin, F. et al. Curr. Drug Targets Inflamm. Allergy 2004, 3, 213-219); intestinal and colitic diseases (Saemann, M.D. et al. Wien. Klin. Wochenschr. 2002, 114, 289-300); beta thalassemia (Rodgers, G.P. et al. Expert Opin. Investig. Drugs 2001, 10, 925-934); respiratory diseases (Barnes, P.J. Am. J. Respir. Crit. Care Med. 2003, 167, 813-818), Rubinstein-Taybi syndrome (Alarcon, J.M. et al. Neuron 2004, 42, 947-959).

A number of histone deacetylase inhibitors are known, including natural products (e.g. trichostatin A (TSA), trapoxin (TPX), depsipeptide FK-228), short chain fatty acids (sodium-butyrate, -phenylbutyrate and -valproate) hydroxamates (e.g. suberoylanilide (SAHA), pyroxamide, scriptaid, oxamflatin, NVP-LAQ824) cyclic peptides containing hydroxamic acid (CHAPs) and benzamides (e.g. MS-275). All of them potently induce growth arrest, differentiation and apoptosis in a variety of transformed cells in culture as well in animal models (Marks, P.A. et al. Curr. Opin. Oncol. 2001, 13, 477-483). Several HDAC inhibitors such as, sodium phenylbutyrate (alone or in combination), depsipeptide, SAHA, pyroxamide, NVP-LAQ824 and MS-275 are being evaluated in clinical studies for the treatment of various tumor diseases (Johnstone, R.W Nat. Rev. Drug Discov. 2002, 1, 287-299). Their clinical benefit, however, is limited by toxicity problems (TSA, CHAPs, MS-275), low stability (TSA), low solubility (TSA), poor potency and lack of selectivity (butyrates and analogues) (Vigushin, D. et al. Anti-Cancer Drugs 2002, 13, 1-13).

To overcome these liabilities many derivatives have been synthesised based on the structures of the aforesaid compounds, with some molecular sub-structures hypothesised by certain authors as being useful for the activity and penetration of cellular structures (Miller, T.A. Expert Opin. Ther. Patents 2004, 14, 791-804; Miller, T.A. J. Med. Chem. 2003, 46, 5098-5116; Moradei, O. et al. Curr. Med. Chem. - Anticancer Agents 2005, 5, 529-560; Minucci, S. et al. Nature Reviews Cancer, 2006 6, 38-51). WO 2006/037761 discloses three compounds which have been excluded by disclaimer from the claimed scope.

WO 06/020004 describes HDAC inhibitors with the following general formula 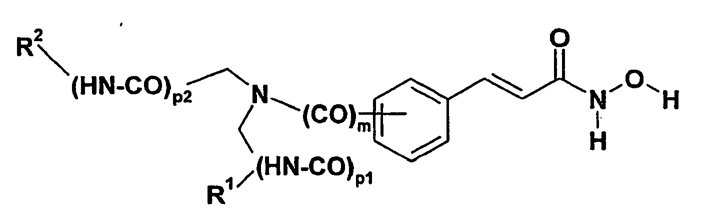 where m, p¹ and p² are 0 or 1, R¹ and R² are, among other groups, C₁-C₁₀ alkyl, aryl, heteroaryl, C₁-C₁₀ alkylaryl or C₁-C₁₀ alkylheteroaryl.

WO 04/063169 describes histone deacetylase inhibitors of general formula 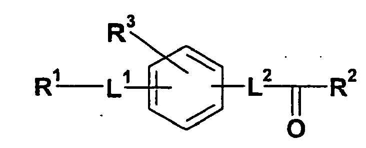 where R¹ is an optionally substituted heterocycle which contains a nitrogen, R² is hydroxylamine, R³ is, among other substituents, hydrogen, L¹ is an optionally substituted -(CH₂)ₙ- group with n being between 0 and 6; L² is an alkenyl chain. WO 03/087066 describes HDAC inhibitors of general formula: 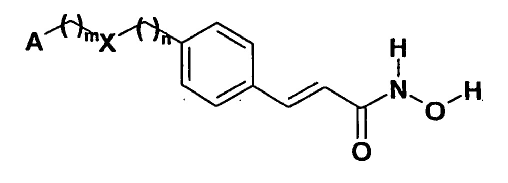 where A is phenyl or an optionally substituted heterocycle; m and n are from 0 to 4; and X can be the following group 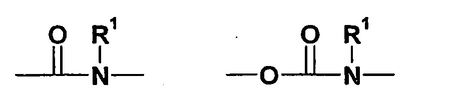 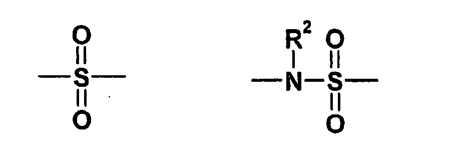 where R¹ and R² are independently hydrogen or an optionally substituted C₁-C₄ alkyl chain.

WO 95/13264 describes N-hydroxypropenamides of general formula 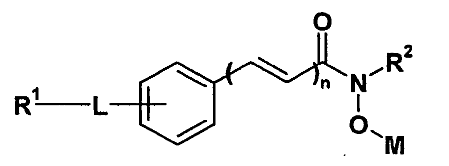 where R¹ is, among other groups, phenyl or aryloxyphenyl; L is a C₁-C₈ alkylene chain, -(CH₂)ₘ-O- (where m is a number from 0 to 4) or -CO-; n is 0 or 1; R² is hydrogen, C₁-C₄ alkyl or arylalkyl; M is, among other groups, hydrogen.

In J. Med. Chem. 2001, 44, 2069-2072, J. Med. Chem. 2002, 45, 1778-1784, J. Med. Chem. 2003, 46, 512-524, J. Med. Chem. 2003, 46, 4826-4829, J. Med. Chem. 2004, 47, 1098-1109, J. Med. Chem. 2004, 47, 1351-1359 and J. Med. Chem. 2005, 48, 3344-3353, Mai *et al.* describe a series of pyrrolyl hydroxyamides as selective histone deacetylase inhibitors.

HDAC inhibitors are also described in patent application PCT/EP2005/054949.

Several lines of research are currently ongoing in the field, focused both on the identification of new inhibitors having a broad-ranging action on all histone deacetylases, or inhibitors having a greater activity towards specific HDAC subclasses.

In addition, based on the clinical and preclinical data of the first HDAC inhibitors and the great therapeutic potential of HDAC inhibition for various pathologies, the need for new inhibitors with improved pharmacological and chemico-physical properties is considerably high.

In particular, compounds endowed with increased inhibitory potency and metabolic stability could be extremely useful therapeutic agents with higher activity and longer duration of effect as compared to known inhibitors.

### SUMMARY

New histone deacetylase inhibitors have now been identified, endowed with HDAC inhibitory activity and favourable pharmacological properties. Said inhibitors have the general formula **(I)** 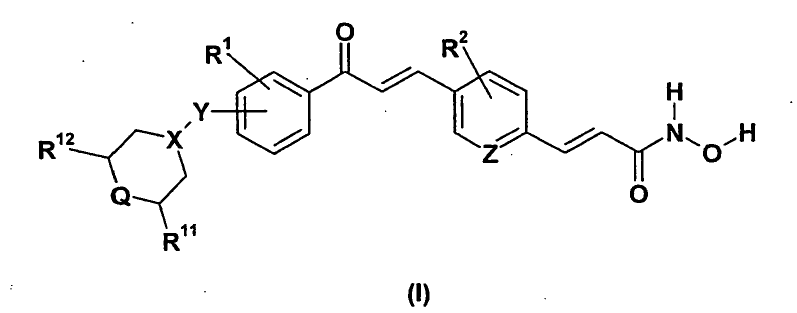 wherein:
- **Q**: is a bond, CH₂, **CH-NR³R⁴, NR⁵** or oxygen;
- **X**: is CH or nitrogen;
- **Y**: is a bond, CH₂, oxygen or **NR⁶;**
- **Z**: is CH or nitrogen;
- **R¹, R²**: are, independently, hydrogen, halogen, C₁-C₆ alkyl or C₁-C₆ haloalkyl;
- **R³, R⁴**: are, independently, hydrogen, C₁-C₆ alkyl, phenyl or benzyl;
- **R⁵**: is hydrogen, C₁-C₆ alkyl, (CO)R⁷, SO₂-C₁-C₆ alkyl, phenyl or benzyl;
- **R⁶**: is hydrogen, C₁-C₆ alkyl or benzyl;
- **R⁷**: is hydrogen, C₁-C₆ alkyl, phenyl, benzyl, **OR⁸** or **NR⁹R¹⁰;**
- **R⁸**: is C₁-C₆ alkyl;
- **R⁹**: is hydrogen, C₁-C₆ alkyl, phenyl or benzyl;
- **R¹⁰**: is hydrogen, C₁-C₆ alkyl or benzyl;
- **R¹¹, R¹²**: are, independently, hydrogen or C₁-C₆ alkyl;
and the pharmaceutically acceptable salts thereof;
with the proviso that when **X** is nitrogen, **Y** cannot be oxygen or **NR⁶;**
and with the exclusion of the following compounds:
(E)-N-Hydroxy-3-(4-{(E)-3-[4-(4-methyl-piperazin-1-yl)-phenyl]-3-oxo-propenyl}-phenyl)-acrylamide;
(E)-N-Hydroxy-3-{4-[(E)-3-(4-morpholin-4-yl-phenyl)-3-oxo- propenyl]-phenyl}-acrylamide;
(E)-3-{3-Fluoro-4-[(E)-3-(4-morpholin-4-yl-phenyl)-3-oxo-propenyl]-phenyl}-N-hydroxy-acrylamide.

### DETAILED DESCRIPTION OF THE INVENTION

In the above described formula **(I)** as well as in the sub-formulas **(Ia), (Ib), (Ic)** herebelow disclosed, the following general definitions apply.

The phenyl or benzyl in **R³, R⁴, R⁵, R⁶, R⁷, R⁹, R¹⁰,** may be optionally substituted with one or more substituents selected from halogen, C₁-C₆ alkyl, C₁-C₆ haloalkyl, C₁-C₆ alkoxy, or C₁-C₆ haloalkoxy.

"Acceptable pharmaceutical salts" comprise salts obtained by salification with inorganic acids (e.g. hydrochloric, hydrobromide, sulfuric or phosphoric acids), or with organic acids (e.g. acetic, propionic, benzoic, cinnamic, mandelic, salicylic, glycolic, lactic, oxalic, malic, maleic, malonic, fumaric, tartaric, citric, p-toluenesulfonic or methanesulfonic acids).

All the "alkyl" chains and alkyl-containing chains (e.g. haloalakyl) can be linear or branched.

"Halogens" are preferably fluorine, chlorine or bromine, being in particular fluorine or chlorine.

The "C₁-C₆ alkyl" group is preferably a linear or branched C₁-C₄ alkyl group, more preferably a C₁-C₂ alkyl group.

The "C₁-C₆ alkoxy" group is preferably a linear or branched C₁-C₄ alkoxy group, more preferably a C₁-C₂ alkoxy group.

The "C₁-C₆ haloalkoxy" group is preferably a linear or branched C₁-C₄ haloalkoxy group, more preferably a C₁-C₂ haloalkoxy group.

The "C₁-C₆ haloalkyl" group is preferably a linear or branched C₁-C₄ haloalkyl group, more preferably a C₁-C₂ haloalkyl group, being in particular CF₃.

The present invention comprises all possible isomers of said formulas **(I), (Ia), (Ib)** or **(Ic)** and mixtures thereof, and the metabolic precursors of formula **(I)** compounds. The term "metabolic precursors" means compounds having a different structure from that of the relevant formulas **(I), (Ia), (Ib)** or **(Ic),** which after administration to the patient are directly or indirectly transformed into a compound of said formula **(I), (Ia), (Ib)** or **(Ic).** Methods for selecting metabolic precursors and their relative preparation are described for example in the book by Bundgaard (Bundgaard, H. ed., "Design of Prodrugs", Elsevier, 1985).

All compounds of present formula **(I)** show useful HDAC inhibiting activity. Furthermore, within the scope of the general formula **(I),** the present inventors have identified three sub-groups of compounds identified by formulas **(Ia), (Ib)** and (Ic) as herebelow defined. The compounds of these three sub-groups are characterised by a particularly high HDAC inhibiting activity, and a high resistance to metabolic inactivation; these three sub-groups represent particular embodiments of the invention.

A first embodiment is thus represented by the compounds of formula (Ia) 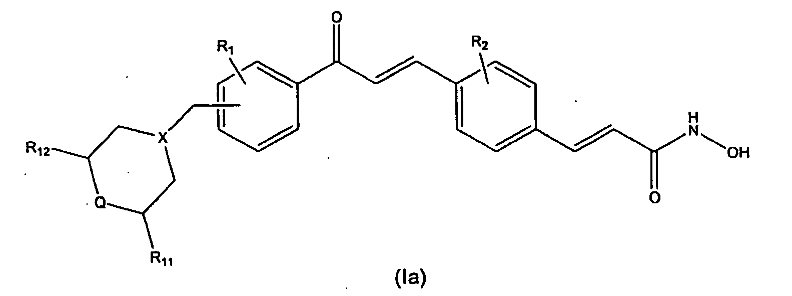 wherein:
- **Q**: is CH₂,CH-**NR³R⁴**, or **NR⁵ ;**
- **X**: is CH or nitrogen;
- **R¹, R²**: are, independently, hydrogen, halogen, C₁-C₄ alkyl, or C₁-C₄ haloalkyl;
- **R³, R⁴**: are, independently, hydrogen or C₁-C₄ alkyl;
- **R⁵**: is hydrogen, C₁-C₄ alkyl, (CO)**R⁷**, phenyl or benzyl;
- **R⁷**: is hydrogen, C₁-C₆ alkyl, phenyl, benzyl, **OR⁸** or **NR⁹R¹⁰;**
- **R⁸**: is C₁-C₄ alkyl;
- **R⁹, R¹⁰**: are, independently, hydrogen or C₁-C₄ alkyl;
- **R¹¹, R¹²**: are, independently, hydrogen or C₁-C₄ alkyl.

As evident from comparison with formula **(I),** in formula **(Ia)** Y is only CH₂ and Z is only CH; further limitations are present with respect to the remaining radicals.

Preferably, within said formula **(Ia),** the shown radicals have the following meanings:
- **Q**: is CH₂, **CH-NR³R⁴** or **NR⁵;**
- **X**: is CH or nitrogen;
- **R¹, R²**: are, independently, hydrogen, fluoro, chloro, or CF₃;
- **R³, R⁴**: are, independently, hydrogen or C₁-C₂ alkyl;
- **R⁵**: is hydrogen, C₁-C₄ alkyl, (CO)R⁷, phenyl or benzyl;
- **R⁷**: is hydrogen, C₁-C₄ alkyl, phenyl, benzyl, **OR⁸** or **NR⁹R¹⁰;**
- **R⁸**: is C₁-C₄ alkyl;
- **R⁹, R¹⁰**: are, independently, hydrogen or C₁-C₂ alkyl;
- **R¹¹, R¹²**: are, independently, hydrogen or C₁-C₂ alkyl.

Even more preferably, within said formula (Ia), the shown radicals have the following meanings:
- **Q**: is **NR⁵;**
- **X**: is nitrogen;
- **R¹, R²**: are, independently, hydrogen, fluoro, chloro or CF₃;
- **R⁵**: is hydrogen, C₁-C₄ alkyl, (CO)**R⁷,** phenyl or benzyl;
- **R⁷**: is hydrogen, C₁-C₄ alkyl, phenyl, benzyl, O**R⁸** or **NR⁹R¹⁰;**
- **R⁸**: is C₁-C₄ alkyl;
- **R⁹, R¹⁰**: are, independently, hydrogen or C₁-C₂ alkyl;
- **R¹¹, R¹²**: are, independently, hydrogen or C₁-C₂ alkyl.

According this last implementation mode, the X/Q containing ring of formula **(I)** is always a piperazine ring.

**A second embodiment** is represented by the compounds of formula **(Ib)** 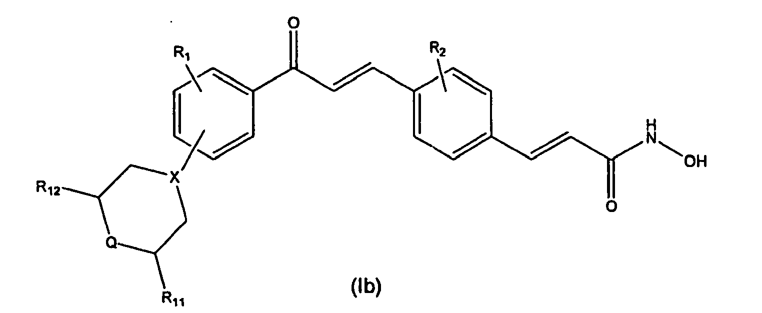 wherein:
- **Q**: is CH₂, **CH-NR³R⁴,** or **NR⁵;**
- **X**: is CH or nitrogen;
- **R¹, R²**: are, independently, hydrogen, halogen, C₁-C₄ alkyl or C₁-C₄ haloalkyl;
- **R³, R⁴**: are, independently, hydrogen or C₁-C₄ alkyl;
- **R⁵**: is hydrogen, C₁-C₄ alkyl, (CO)R⁷, SO₂-C₁-C₄ alkyl, phenyl or benzyl;
- **R⁷**: is hydrogen, C₁-C₆ alkyl, phenyl, benzyl, OR⁸ or **NR⁹R¹⁰;**
- **R⁸**: is C₁-C₄ alkyl;
- **R⁹, R¹⁰**: are, independently, hydrogen or C₁-C₄ alkyl;
- **R¹¹, R¹²**: are, independently, hydrogen or C₁-C₄ alkyl;
with the exclusion of the following compound:
(E)-N-Hydroxy-3-(4-{(E)-3-[4-(4-methyl-piperazin-1-yl)-phenyl]-3-oxo-propenyl}-phenyl)-acrylamide.

As evident from comparison with formula **(I),** in formula **(Ib)** Y is only a bond and Z is only CH; further limitations are present with respect to the remaining radicals. Preferably, within said formula **(Ib),** the group: 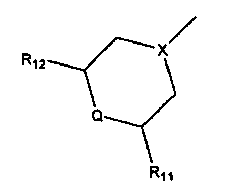 is in ortho or meta position with respect to the 3-oxo-propenyl moiety (i.e. the Y-radical and the 3-oxo-propenyl radical are attached on the R₁-containing ring in ortho or meta relation with each other), and the other radicals have the following meanings:
- **Q**: is CH₂ or **NR⁵;**
- **X**: is CH or nitrogen;
- **R¹, R²**: are, independently, hydrogen, fluoro, chloro or CF₃;
- **R⁵**: is hydrogen, C₁-C₄ alkyl, (CO)R⁷, phenyl or benzyl;
- **R⁷**: is hydrogen, C₁-C₄ alkyl, phenyl, benzyl, **OR⁸** or **NR⁹R¹⁰;**
- **R⁸**: is C₁-C₄ alkyl;
- **R⁹, R¹⁰**: are, independently, hydrogen or C₁-C₂ alkyl;
- **R¹¹, R¹²**: are, independently, hydrogen or C₁-C₂ alkyl.

**A third embodiment** is represented by the compounds of formula (Ic) 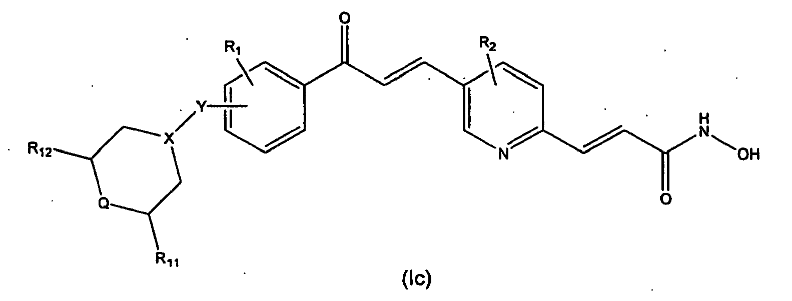 wherein:
- **Q**: is CH₂, CH-**NR³R⁴**, **NR⁵** or oxygen;
- **X**: is CH or nitrogen;
- **Y**: is a bond, CH₂, oxygen or **NR⁶;**
- **R¹, R²**: are, independently, hydrogen, halogen, C₁-C₄ alkyl, or C₁-C₄ haloalkyl;
- **R³, R⁴**: are, independently, hydrogen or C₁-C₄ alkyl;
- **R⁵**: is hydrogen, C₁-C₄ alkyl, (CO)**R⁷**, phenyl or benzyl;
- **R⁶**: is hydrogen or C₁-C₄ alkyl;
- **R⁷**: is hydrogen, C₁-C₆ alkyl, phenyl, benzyl, O**R⁸** or **NR⁹R¹⁰;**
- **R⁸**: is C₁-C₄ alkyl;
- **R⁹,R¹⁰**: are, independently, hydrogen or C₁-C₄ alkyl;
- **R¹¹, R¹²**: are, independently, hydrogen or C₁-C₄ alkyl;
provided that when **X** is nitrogen, **Y** cannot be oxygen or **NR⁶.**

As evident from comparison with formula **(I),** in formula **(Ic)** Z is only nitrogen; further limitations are present with respect to the remaining radicals. Preferably, within said formula **(Ic),** the shown radicals have the following meanings:
- **Q**: is CH₂, **NR⁵** or oxygen;
- **X**: is CH or nitrogen;
- **Y**: is a bond or CH₂;
- **R¹, R²**: are, independently, hydrogen, fluoro, chloro or CF₃;
- **R⁵**: is hydrogen, C₁-C₂ alkyl, (CO)R⁷ , phenyl or benzyl;
- **R⁷**: is hydrogen, C₁-C₄ alkyl, phenyl, benzyl, **OR⁸** or **NR⁹R¹⁰;**
- **R⁸**: is C₁-C₄ alkyl;
- **R⁹, R¹⁰**: are, independently, hydrogen or C₁-C₂ alkyl;
- **R¹¹, R¹²**: are, independently, hydrogen or C₁-C₂ alkyl.

All compounds of said formulas **(I), (Ia), (Ib), (Ic),** possess HDAC inhibitory activity. In particular, as shown in the experimental section, the compounds of formulas **(Ia), (Ib), (Ic)** show surprisingly a remarkable higher HDAC inhibitory activity, and a higher resistance to metabolic inactivation.

Preferred compounds belonging to both formulas **(I)** and **(Ia)** are the following:
(E)-N-Hydroxy-3-(4-{(E)-3-[4-(4-methyl-piperazin-1-yl-methyl)-phenyl]-3-oxo-propenyl}-phenyl)-acrylamide; (example 7)
(E)-3-(4-{(E)-3-[4-(4-Dimethylamino-piperidin-1-yl-methyl)-phenyl]-3-oxo-propenyl}-phenyl)-N-hydroxy-acrylamide; (example 9)
(E)-N-Hydroxy-3-(4-{(E)-3-[4-(1-methyl-piperidin-4-ylmethyl)-phenyl]-3-oxo-propenyl}-phenyl)-acrylamide; (example 23)
(E)-N-Hydroxy-3-{4-[(E)-3-oxo-3-(4-piperazin-1-ylmethyl-phenyl)-propenyl]-phenyl}-acrylamide; (example 26)
(E)-3-(4-{(E)-3-[4-(4-Benzyl-piperazin-1-ylmethyl)-phenyl]-3-oxo-propenyl}-phenyl)-N-hydroxy-acrylamide; (example 27)
(E)-3-(4-{(E)-3-[4-((3R,5S)-3,5-Dimethyl-piperazin-1-ylmethyl)-phenyl]-3-oxo-propenyl}-phenyl)-N-hydroxy-acrylamide; (example 29)
(E)-3-(4-{(E)-3-[4-(4-Acetyl-piperazin-1-ylmethyl)-phenyl]-3-oxo-propenyl}-phenyl)-N-hydroxy-acrylamide; (example 30)
(E)-3-(4-{(E)-3-[4-((3R,5S)-4-Acetyl-3,5-dimethyl-piperazin-1-ylmethyl)-phenyl]-3-oxo-propenyl}-phenyl)-N-hydroxy-acrylamide; (example 31)
(E)-3-(4-{(E)-3-[4-(4-Ethyl-piperazin-1-ylmethyl)-phenyl]-3-oxo-propenyl}phenyl)-N-hydroxy-acrylamide; (example 32)
(E)-N-Hydroxy-3-(4-{(E)-3-[3-(4-methyl-piperazin-1-ylmethyl)-phenyl]-3-oxo-propenyl}-phenyl)-acrylamide; (example 34)
(E)-N-Hydroxy-3-(4-{(E)-3-[2-(4-methyl-piperazin-1-ylmethyl)-phenyl]-3-oxo-propenyl}-phenyl)-acrylamide; (example 35)

Preferred compounds belonging to both formulas **(I)** and **(Ib)** are the following:
(E)-N-Hydroxy-3-(4-{(E)-3-[2-(4-methyl-piperazin-1-yl)-phenyl]-3-oxo-propenyl}-phenyl)-acrylamide (example 1)
(E)-N-Hydroxy-3-(4-{(E)-3-[3-(4-methyl-piperazin-1-yl)-phenyl]-3-oxo-propenyl}-phenyl)-acrylamide; (example 2)
(E)-N-Hydroxy-3-(4-{(E)-3-[4-(4-methylamino-piperidin-1-yl)-phenyl]-3-oxo-propenyl}-phenyl)-acrylamide; (example 3)
(E)-3-(4-{(E)-3-[4-(4-Dimethylamino-piperidin-1-yl)-phenyl]-3-oxo-propenyl}-phenyl)-N-hydroxy-acrylamide; (example 4)
(E)-N-Hydroxy-3-(4-{(E)-3-[4-(1-methyl-piperidin-4-yl)-phenyl]-3-oxo-propenyl}-phenyl)-acrylamide; (example 10)
(E)-N-Hydroxy-3-(4-{(E)-3-[4-(4-isobutyl-piperazin-1-yl)-phenyl]-3-oxo-propenyl}-phenyl)-acrylamide; (example 12)
(E)-3-(4-{(E)-3-[4-(4-Ethyl-piperazin-1-yl)-phenyl]-3-oxo-propenyl}-phenyl)-N-hydroxy-acrylamide; (example 13)
(E)-3-(4-{(E)-3-[4-(4-Benzyl-piperazin-1-yl)-phenyl]-3-oxo-propenyl}-phenyl)-N-hydroxy-acrylamide; (example 14)
(E)-N-Hydroxy-3-{4-[(E)-3-(4-piperazin-1-yl-phenyl)-3-oxo-propenyl]-phenyl}-acrylamide; (example 15)
(E)-3-(4-{(E)-3-[4-(4-Benzoyl-piperazin-1-yl)-phenyl]-3-oxo-propenyl}-phenyl)-N-hydroxy-acrylamide; (example 16)
(E)-3-(4-{(E)-3-[4-(4-Acetyl-piperazin-1-yl)-phenyl]-3-oxo-propenyl}-phenyl)-N-hydroxy-acrylamide; (example 17)
(E)-N-Hydroxy-3-(4-{(E)-3-[4-(4-methanesulfonyl-piperazin-1-yl)-phenyl]-3-oxo-propenyl}-phenyl)-acrylamide; (example 18)
4-(4-{(E)-3-[4-((E)-2-Hydroxycarbamoyl-vinyl)-phenyl]-acryloyl}-pheny)-piperazine-1-carboxylic acid dimethylamide; (example 19)
4-(4-{(E)-3-[4-((E)-2-Hydroxycarbamoyl-vinyl)-phenyl]-acryloyl}-phenyl)-piperazine-1-carboxylic acid amide; (example 20)
4-(4-{(E)-3-[4-((E)-2-Hydroxycarbamoyl-vinyl)-phenyl]-acryloyl}-phenyl)-piperazine carboxylic acid ethyl ester; (example 21)
(E)-N-Hydroxy-3-(4-{(E)-3-oxo-3-[4-((3R,5S)-3,4,5-trimethyl-piperazin-1-yl)-phenyl]-propenyl}-phenyl)-acrylamide; (example 24)
(E)-3-(4-{(E)-3-[3-Chloro-5-(4-methyl-piperazin-1-yl)-phenyl]-3-oxo-propenyl}-phenyl)-N-hydroxy-acylamide; (example 25)
(E)-3-(4-{(E)-3-[5-Chloro-2-(4-methyl-piperazin-1-yl)-phenyl]-3-oxo-propenyl}-phenyl)-N-hydroxy-acrylamide; (example 46)
(E)-3-(4-{(E)-3-[2-Chloro-5-(4-methyl-piperazin-1-yl)-phenyl]-3-oxo-propenyl}-phenyl)-N-hydroxy-acrylamide (example 51)

Preferred compounds belonging to both formulas **(I)** and **(Ic)** are the following:
(E)-N-Hydroxy-3-(5-{(E)-3-[4-(4-methyl-piperazin-1-yl)-phenyl]-3-oxo-propenyl}-pyridin-2-yl)-acrylamide; (example 11)
(E)-N-Hydroxy-3-(5-{(E)-3-[2-(4-methyl-piperazin-1-yl)-phenyl]-3-oxo-propenyl}-pyridin-2-yl)-acrylamide; (example 36)
(E)-N-Hydroxy-3-(5-{(E)-3-[3-(4-rnethyl-piperazin-1-yl)-phenyl]-3-oxo-propenyl}-pyridin-2-yl)-acrylamide; (example 37)
(E)-3-(5-{(E)-3-[4-(4-Benzyl-piperazin-1-yl)-phenyl]-3-oxo-propenyl}-pyridin-2-yl)-N-hydroxy-acrylamide; (example 38)
(E)-N-Hydroxy-3-(5-{(E)-3-oxo-3-[4-((3R,5S)-3,4,5-trimethyl-piperazin-1-yl)-phenyl]-propenyl}-pyridin-2-yl)-acrylamide; (example 39)
(E)-N-Hydroxy-3-{5-[(E)-3-(4-morpholin-4-ylmethyl-phenyl)-3-oxo-propenyl]-pyridin-2-yl}-acrylamide; (example 40)
(E)-3-(5-{(E)-3-[4-(4-Ethyl-piperazin-1-yl)-phenyl]-3-oxo-propenyl}-pyridin-2-yl)-N-hydroxy-acrylamide; (example 41)
(E)-3-(5-{(E)-3-[4-(4-Acetyl-piperazin-1-yl)-phenyl]-3-oxo-propenyl}-pyridin-2-yl)-N-hydroxy-acrylamide; (example 42)
(E)-N-Hydroxy-3-(5-{(E)-3-oxo-3-[3-((3R,5S)-3,4,5-trimethyl-piperazin-1-yl)-phenyl]-propenyl}-pyridin-2-yl)-acrylamide; (example 43)
(E)-N-Hydroxy-3-(5-{(E)-3-[4-(1-methyl-piperidin-4-ylmethyl)-phenyl]-3-oxo-propenyl}-pyridin-2-yl)-acrylamide; (example 44)
(E)-N-Hydroxy-3-{5-[(E)-3-oxo-3-(4-piperazin-1-yl-phenyl)-propenyl]- pyridin-2-yl}-acrylamide; (example 45)
(E)-N-Hydroxy-3-(5-{(E)-3-[2-(4-methyl-piperazin-1-ylmethyl)-phenyl]-3-oxo-propenyl}-pyridin-2-yl)-acrylamide; (example 47)
(E)-N-Hydroxy-3-{5-[(E)-3-oxo-3-(4-piperazin-1-ylmethyl-phenyl)-propenyl]- pyridin-2-yl}-acrylamide; (example 48)
(E)-3-(5-{(E)-3-[4-(4-Acetyl-piperazin-1-ylmethyl)-phenyl]-3-oxo-propenyl}-pyridin-2-yl)-N-hydroxy-acrylamide; (example 49)
(E)-N-Hydroxy-3-(5-{(E)-3-[4-(4-methyl-piperazin-1-ylmethyl)-phenyl]-3-oxo-propenyl}-pyridin-2-yl)-acrylamide (example 50)
(E)-3-(5-{(E)-3-[3-Chloro-5-(4-methyl-piperazin-1-yl)-phenyl]-3-oxo-propenyl}-pyridin-2-yl)-N-hydroxy-acrylamide (example 52)
(E)-N-Hydroxy-3-(5-{(E)-3-[3-(4-methyl-piperazin-1-ylmethyl)-phenyl]-3-oxo-propenyl}-pyridin-2-yl)-acrylamide (example 53).

Further preferred compounds belonging to formula (I) are the following:
(E)-N-Hydroxy-3-(4-{(E)-3-[4-(1-methyl-piperidin-4-yl-amino)-phenyl]-3-oxo-propenyl}-phenyl)-acrylamide (example 5)
(E)-N-Hydroxy-3-[4-(E)-3-{4-[N-methyl-(1-methyl-piperidin-4-yl)-amino]-phenyl}-3-oxo-propenyl)-phenyl]-acrylamide (example 6)
(E)-N-Hydroxy-3-(4-{(E)-3-[4-(1-methyl-piperidin-4-yloxy)-phenyl]-3-oxo-propenyl}-phenyl)-acrylamide (example 22).

The present invention also comprises the process for preparing the compounds of formula **(I), (Ia), (Ib), (Ic).** These compounds can be synthesized by treating a compound of formula (II): 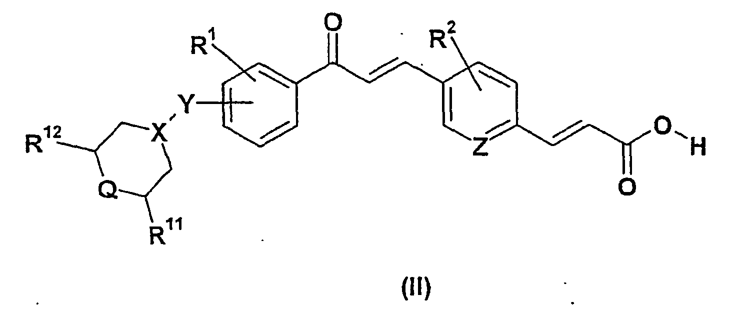 where **Q, X, Y, Z, R¹, R², R¹¹, R¹²** have the aforedescribed meanings, with a protected hydroxylamine such as O-(tetrahydro-2H-pyran-2-yl)hydroxylamine (NH₂OTHP), followed by a deprotection step to give the corresponding hydroxylamine.

The reaction of the compound of formula **(II)** with the protected hydroxylamine can be carried out with condensation agents such as EDC (1-(3-dimethylaminopropyl)-3-ethylcarbodiimide), in the presence of a suitable base (e.g. triethylamine or di-isopropylethylamine) in a suitable solvent (e.g. tetrahydrofuran, dichloromethane or DMF). Generally an activator of the condensation reaction, such as HOBT (1-hydroxybenzotriazole) or HOAT (1-hydroxy-7-aza-benzotriazole), can be added to the reaction mixture. The reaction can be carried out at room temperature for a period lasting between about 2 and 12 hours. Deprotection of the hydroxylamine, in the case of tetrahydropyranyl, can be achieved by known methods, for example using HCl in aprotic solvents (such as THF, diethylether or dioxane).

The compounds of formula **(II)** can be synthesized by treating a compound of formula **(III),** 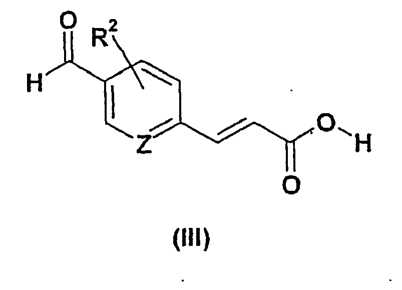 where **R²** and **Z** have the aforesaid meanings, with a compound of formula **(IV),** 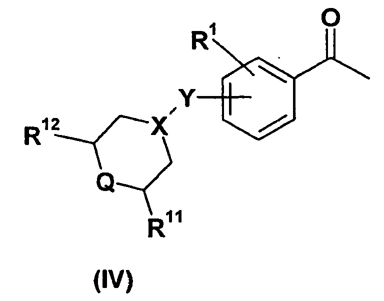 where **Q, X, Y, R¹, R¹¹, R¹²** have the aforesaid meanings.

The compounds of formula **(II)** where Q is NCOR⁷ (where R⁷ is hydrogen, C₁-C₆ alkyl, phenyl, benzyl or O**R⁸** , with **R⁸** as aforedefined) can also be synthesized by treating a compound of formula **(V)** 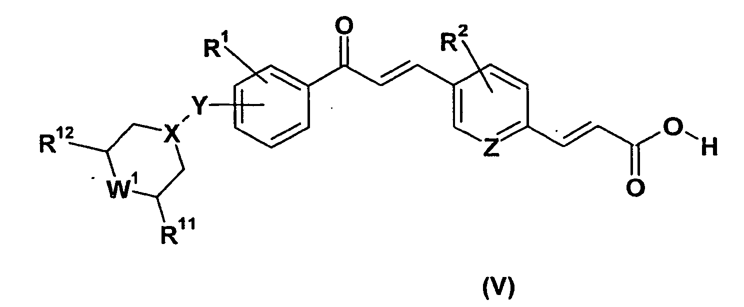 (where **X, Y, R¹, R², R¹¹, R¹²** and **Z** have the aforesaid meanings and **W¹** is NH) with a compound of formula **(VI) R⁷COA,** where **R⁷** is hydrogen, C₁-C₆ alkyl, phenyl, benzyl or **OR⁸** (with **R⁸** as aforedefined) and **A** is a halogen or a O-EWG group where EWG indicates an electron-attracting group, such as a p-toluenesulfonic or methanesulfonic group, or benzotriazole if **R⁷**CO is formyl.

The compounds of formula **(II),** in which Q is equal to **NR⁵,** with **R⁵** equal to C₁-C₆ alkyl or benzyl, can also be synthesized by treating a compound of formula **(V)** with a compound of formula **(VII) R⁵A** or with a compound of formula **(VIII) R¹³**CHO, **where R⁵** is C₁-C₆ alkyl or benzyl, the benzyl being optionally substituted by one or more substituents chosen from halogen, C₁-C₆ alkyl, halo-C₁-C₆ alkyl, C₁-C₆ alkoxy or halo-C₁-C₆ alkoxy, **A** is a halogen or a O-EWG group, where EWG indicates an electron-attracting group, such as a p-toluenesulfonic or methanesulfonic group, and **R¹³** is a C₁-C₅ alkyl or phenyl, the phenyl being optionally substituted by one or more substituents chosen from halogen, C₁-C₆ alkyl, halo-C₁-C₆ alkyl, C₁-C₆ alkoxy or halo-C₁-C₆ alkoxy.

To obtain compounds of formula **(II)** with **Q** equal to **NCONR⁹R¹⁰,** where **R⁹** has the aforementioned meanings and R¹⁰ is hydrogen, a compound of formula **(V)** is treated with a compound of formula **(IX) R⁹N=C=O,** where **R⁹** has the aforelisted meanings; otherwise, to obtain compounds of formula **(II)** with Q equal to **NCONR⁹R¹⁰,** where **R⁹** has the aforesaid meanings and **R¹⁰** is different from hydrogen, a compound of formula **(V)** is first treated with a compound of formula **(IX)** and then with a compound of formula **(X) R¹⁰A,** where **R¹⁰** has the aforelisted meanings and **A** is a halogen or a O-EWG group, where EWG indicates an electron-attracting group, such as a p-toluenesulfonic or methanesulfonic group.

The compounds of formula **(II)** where Q is **CH-NR³R⁴,** where **R³** and **R⁴** have the aforesaid meanings, can also be synthesized by treating a compound of formula **(XI)** 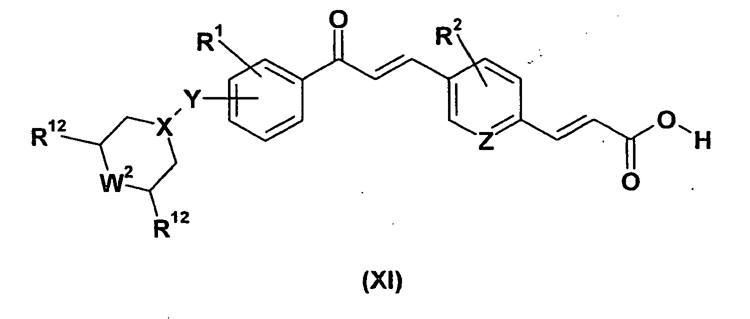 where **X, Y, R¹, R², R¹¹, R¹²** and **Z** have the aforesaid meanings and **W²** is CO, with a compound **HNR³R⁴,** where **R³** and **R⁴** have the aforesaid meanings.

The reaction between a compound of formula **(III)** and one of formula **(IV),** with **Q** being different from **N(CO)R⁷,** can be carried out in the presence of an inorganic base in a protic solvent, such as water, methanol or ethanol, at a temperature between 0°C and 25°C and for a reaction time between about 2 and 36 hours. The reaction between a compound of formula **(V)** and one of formula **(VI)** can be carried out in the presence of a suitable base (such as triethylamine, di-isopropylethylamine) in a suitable solvent (e.g. tetrahydrofuran) at a temperature between about 0°C and room temperature.

The reaction between a compound of formula **(V)** and one of formula **(VII)** is an alkylation process and can be carried out in a suitable organic solvent (e.g. tetrahydrofuran, dichloromethane or diethylether) in the presence of a suitable base (such as triethylamine, di-isopropylethylamine) at a temperature between about 0°C and 50°C. The preferred halogen is bromine or iodine.

The reactions between the compound of formula **(V)** and the compound of formula **(VIII)** and between the compound of formula **(XI)** and **HNR³R⁴** are reductive amination processes and can be carried out, preferably under nitrogen atmosphere, in a suitable organic solvent (e.g. methanol, ethanol or tetrahydrofuran) at a temperature between about 0 and 70°C in the presence of a reducing agent such as NaBH₄, Na(CH₃CO₂)₃BH or NaBH₃CN. If necessary titanium tetraisopropylate or molecular sieves can be added to facilitate the reaction.

The reaction between the compound of formula **(V)** and the compound of formula **(IX)** can be carried out in a suitable organic solvent (e.g. tetrahydrofuran, dichloromethane or diethylether) at a temperature between about 0°C and room temperature.

The alkylation of the product of the reaction of a compound of formula **(V)** with a compound of formula **(IX)** and with a compound of formula **(X)** can be carried out in a suitable organic solvent (e.g. tetrahydrofuran, dichloromethane or diethylether) at a temperature between about 0°C and 50°C. The preferred halogen is bromine or iodine.

The compounds of formula **(V)** and the compounds of formula **(XI)** can be prepared in a similar method to that previously described for the reaction between compounds of formula **(III)** and compounds of formula **(IV),** starting from compounds of formula **(III)** and from compounds of formula **(XII)** or formula **(XIII).** 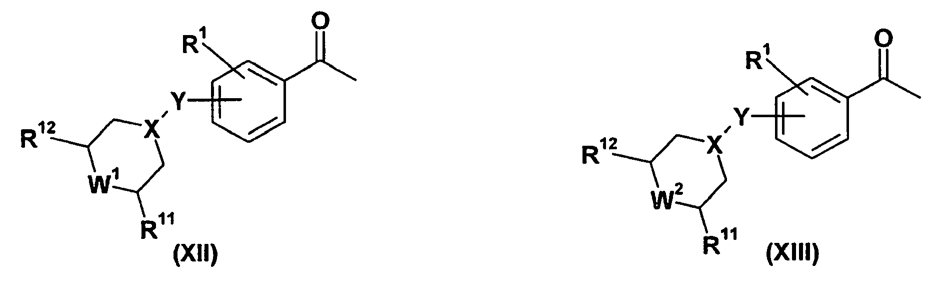 where **W¹, W², X, Y, R¹, R¹¹, R¹²** have the aforesaid meanings.

The compounds of formula **(III)** are commercial products or can be synthesized by treating a compound of formula **(XIV),** 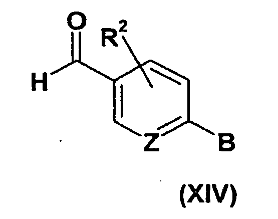 (where **Z** and **R²** have the aforesaid meanings and **B** is halogen, in particular bromine or iodine) with tert-butylacrylate according the Heck reaction. The reaction conditions are described for example in the book by Larhed and Hallberg (Larhed, M.; Hallberg, A. "Handbook of Organopalladium Chemistry for Organic Synthesis", Negishi, E., Ed.; Wiley-Interscience, 2002). The reaction can be carried out in a suitable organic solvent (e.g. DMF) in the presence of palladium salts (e.g. palladium acetate), organic or inorganic bases (e.g. triethylamine, 1,4-diazabicyclo[2,2,2]-octane, sodium or potassium carbonate) and phosphine ligand derivatives, such as triphenylphosphine, at a temperature between room temperature and about 140°C.

Otherwise, the compounds of formula **(III),** where **Z** is nitrogen, can be synthesized by treating a compound of formula **(XV)** 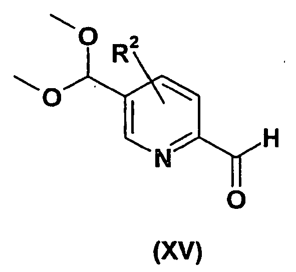 where **R²** has the aforesaid meaning, with tert-butyl diethylphosphono acetate in the presence of an inorganic base, e.g. NaH, in an aprotic solvent, such as tetrahydrofuran, at a temperature between about 0°C and room temperature. The deprotection of the tert-butyl group can be achieved by known methods.

The compounds of formula **(XV)** can be synthesized by treating a compound of formula **(XVI)** 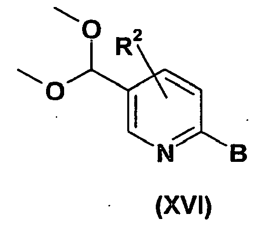 where **B and R²** have the aforesaid meaning, firstly with alkyl lithium, e.g. n-butyllithium, then with DMF in an aprotic solvent (e.g. THF) at a temperature between about -78°C and room temperature between 1 and 3 hours.

The compounds of formula **(IV)** are known products or can be obtained by treating a compound of formula **(XVII)** 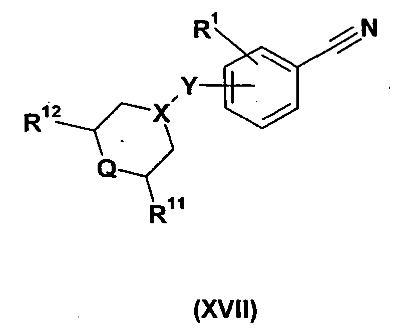 where **Q, X, Y, R¹, R¹¹, R¹²** have the aforesaid meanings provided that **Q** is different from N(CO)R⁷, with methyl magnesium bromide.

The compounds of formula (**IV**) can also be synthesized by treating a compound of formula (XVIII) 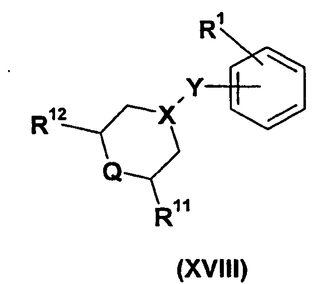 where **Q, X, Y, R¹, R¹¹, R¹²** have the aforesaid meanings, with acetyl chloride in the presence of a Lewis acid (AlCl₃).

The compounds of formula **(IV)** with **Q** being equal to **NCOR⁷,** where **R⁷** is hydrogen, C₁-C₆ alkyl, phenyl, benzyl or **OR⁸,** with **R⁸** as aforedefined, can also be synthesized by treating a compound of formula **(XII)** with a compound of formula **(VI) R⁷COA**, where **R⁷** is C₁-C₆ alkyl, phenyl, benzyl or **OR⁸** and **A** is a halogen or a O-EWG group where EWG indicates an electron-attracting group, such as a p-toluenesulfonic or methanesulfonic group, or benzotriazole if **R⁷CO** is formyl.

The compounds of formula **(IV)** where Q is **NR⁵,** with **R⁵** being equal to C₁-C₆ alkyl or benzyl, can also be synthesized by treating a compound of formula **(XII)** with a compound of formula **(VII).**

The compounds of formula **(IV)** where Q is **CHNR³R⁴,** where **R³** and **R⁴** have the aforesaid meanings, can also be synthesized by treating a compound of formula **(XIII)** with a compound **HNR³R⁴,** where **R³** and **R⁴** have the aforesaid meanings. The reaction between the compound of formula **(XVII)** and methyl magnesium bromide can be carried out under inert atmosphere in a suitable organic solvent (e.g. tetrahydrofuran or diethylether) at a temperature between about 0°C and the boiling point of the chosen solvent.

The reaction between the compound of formula **(XVIII)** and acetyl chloride can be carried out in the presence of a stoichiometric quantity of a Lewis acid (e.g. AlCl₃) under inert atmosphere and in a suitable organic solvent (e.g. dichloromethane or hexane) at a temperature between about 0°C and the boiling point of the chosen solvent.

The reactions between the compound of formula **(XII)** and the compound of formula **(VI)** or with a compound of formula **(VII)** can be carried out under the same conditions as the reaction between a compound of the aforedescribed formula **(V)** and a compound of the aforedescribed formula **(VI)** or **(VII).**

The reaction between the compound of formula **(XIII)** and **HNR³R⁴ can** be carried out under the same conditions as the reaction between a compound of the aforedescribed formula **(XI)** and **HNR³R⁴.**

The compounds of formula **(XVII)** are known products or, if **Y** is a bond and **X** is **N,** they can be obtained by treating a compound of formula **(XIX)** 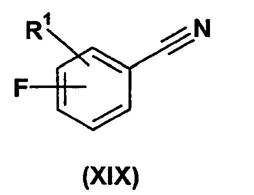 where **R¹** has the aforesaid meaning and F is a fluorine atom, with a compound of formula **(XX)** 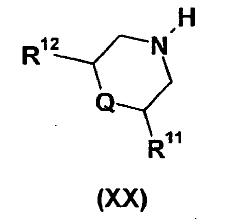 where **R¹¹, R¹²** and **Q** have the aforesaid meaning.

Otherwise, to obtain compounds of formula **(XVII),** where **Y** is CH₂ and **X** is **N,** a compound of formula **(XXI)** 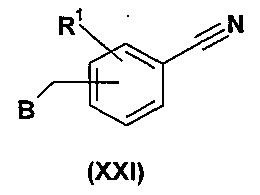 where **R¹** has the aforesaid meaning and **B** is a chlorine, bromine, or iodine atom, is treated with a compound of formula **(XX).**

The reaction between the compound of formula **(XIX)** and the compound of formula **(XX)** can be carried out in the presence of a base (e.g. potassium carbonate) in a suitable organic solvent (e.g. DMSO) at a temperature between about room temperature and 150°C.

The reaction between the compound of formula **(XXI)** and the compound of formula **(XX)** can be carried out under the same conditions as for the reaction between a compound of formula **(V)** and a compound of formula **(VII).** Alternatively, a compound of formula **(IV),** wherein **Y** is CH₂ and **X** is CH, can be obtained by treating a compound of formula **(XXII)** with methyl magnesium bromide and then by reducing it with hydrogen using Pd/C as a catalyst. 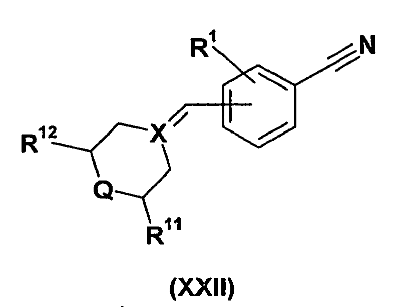 wherein **R¹, R¹¹, R¹²** and **Q** are as defined above and **X** is C.

Compound of formula **(XXII)** can be obtained by treating a compound of formula **(XXI)** with triethyl phosphite and then with a compound of formula **(XXIII)** 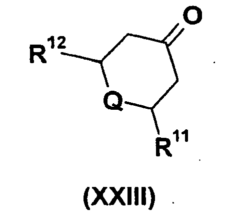 wherein **R¹¹, R¹²** and **Q** are as defined above.

The reaction between a compound of formula **(XXII)** and methylmagnesium bromide can be carried out in an inert atmosphere in an appropriate organic solvent, such as tetrahydrofuran or diethylether, at a temperature ranging from room temperature to the boiling point of the solvent. The hydrogenation can be carried out in an appropriate organic solvent, such as an alcohol, in the presence of a catalyst, for example Pd/C at a pressure ranging from atmospheric pressure to 100 bars.

The reaction of a compound of formula **(XXI)** and triethyl phosphite can be carried out in an appropriate solvent, for example THF at a temperature ranging from room temperature to the boiling point of the solvent. The subsequent Horner-Emmons reaction with a compound of formula (XXIII) can be carried out in an appropriate solvent, for example THF or dioxan and using an appropriate base, for example NaH or BuLi, under an inert atmosphere.

Alternatively, a compound of formula **(IV)** with **Y** equal to oxygen can be obtained by treating a compound of formula **(XXIV)** 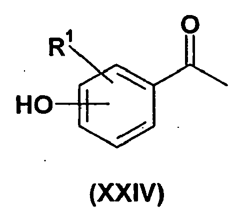 wherein **R¹** is defined above, with a compound of formula **(XXV)** 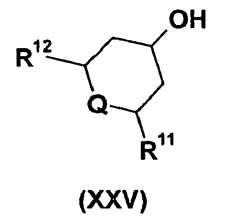 wherein **R¹¹, R¹²** and **Q** are as defined above, in the presence of PPh₃ and diethylazodicarboxylate in a suitable solvent, for example THF or toluene, at a temperature ranging from 0°C to the boiling point of the solvent.

Alternatively a compound of formula **(II),** wherein **Q, Z, R², R¹¹, R¹²** are as defined above and **R¹** is hydrogen, **X** CH₂ and **Y** NH, can be prepared treating a compound of formula **(XXVI)** 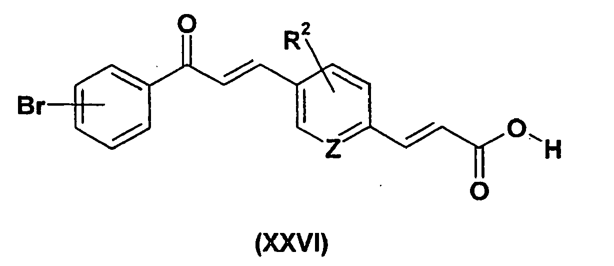 wherein **R²** and **Z** are as defined above, with a compound of formula (XX) 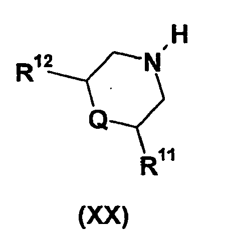 wherein **R¹¹, R¹²** and **Q** are as defined above, in presence of a catalyst, for example Pd₂(dba)₃, in an appropriate solvent, for example toluene, at a temperature ranging from room temperature to the boiling point of the solvent. Alternatively, a compound of formula **(XXVII)**, which forms a compound of formula **(I)** by deprotecting the THP moiety following to the procedure described before, 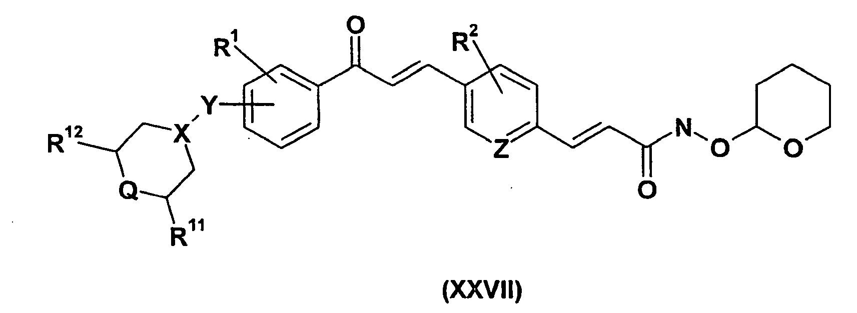 wherein **Q, Z, R¹, R², R¹¹** and **R¹²** are as defined above, and when Y = CH₂ and when **X = N,** can be obtained by treating a compound of formula **(XXVIII)** 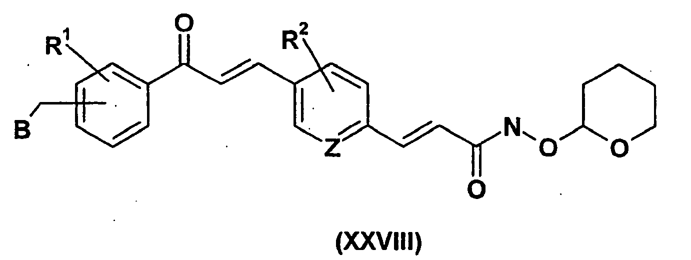 wherein **Z, R¹, R²** and **B** are as defined above, with a compound of formula **(XX),** following the experimental procedures described for the reaction between compounds of formula **(XXI)** and **(XX).**

**HNR³R⁴** and the compounds of formula **(VI), (VII), (VIII), (IX), (X), (XVI), (XVIII), (XIX), (XX), (XXI), (XXIII), (XXIV), (XXV), (XXVI), and (XXVIII)** are known products or can be obtained with known methods by starting from known compounds.

Should the protection of a chemical group of a compound of the present invention and/or an intermediate thereof become necessary, before carrying out one of the aforedescribed reactions, said chemical group can be protected and deprotected according to known methods. References to protection/deprotection steps can be found for example in the book by Greene and Wuts (Greene, T.W.; Wuts, P.G.M. "Protective Groups in Organic Synthesis", John Wiley & Sons Inc., 1991) or the book by Kocienski (Kocienski, P.J. "Protecting Groups", George Thieme Verlag, 1994).

Salification of the compounds of formula **(I), (Ia), (Ib), (Ic)**, and the preparation of compounds of formula **(I), (Ia), (Ib), (Ic),** free of their salts can be carried out by known conventional methods.

The compounds of formula **(I), (Ia), (Ib), (Ic),** have an inhibitory action on histone deacetylases and are therefore useful in the treatment of diseases linked to the disregulation of histone deacetylase activity.

The invention therefore provides compounds of formula **(I), (Ia), (Ib), (Ic),** as previously defined, for use in therapy, particularly for treating diseases linked to the disregulation of histone deacetylase activity.

The invention also comprises the use of one or more compounds of formula **(I), (Ia), (Ib), (Ic),** as previously defined, in the preparation of a drug for preventing and/or treating diseases linked to the disregulation of histone deacetylase activity.

The invention also discloses a method for preventing and/or treating diseases linked to the disregulation of histone deacetylase activity characterized by administering, to a patient requiring it, a pharmacologically useful quantity of one or more compounds of formula **(I)**, **(Ia)**, **(Ib)**, **(Ic)**, as previously defined.

The aforesaid uses and methods also include the possibility of co-administration, simultaneously with or delayed with respect to the adminstration of the compound of formula **(I)**, **(Ia)**, **(Ib)**, **(Ic)**, of additional therapeutic agents.

Diseases linked to the disregulation of histone deacetylase activity at which the present treatment is aimed are particularly tumor type diseases: e.g. leukemias and myeloid and lymphoid lymphomas, myelodysplastic syndromes, multiple myeloma, mammary tumors, pulmonary tumors and pleural mesotheliomas, skin tumors including basal cell carcinomas (basaliomas), melanomas, osteosarcomas, fibrosarcomas, rhabdomyosarcomas, neuroblastomas, glioblastomas, cerebral tumors, testicular and ovarian tumors, endometrial and prostate tumors, thyroid carcinomas, colorectal tumors, gastric tumors and gastrointestinal adenocarcinomas, hepatic carcinomas, pancreatic carcinomas, renal tumors, teratocarcinomas and embryonic carcinomas.

Non-tumor type diseases linked to the disregulation of histone deacetylase activity are for example Huntington's disease, diseases caused by triplet expansion, degenerative diseases, ischemia, oxidative stress, inflammatory responses of the nervous system, epilepsy, diseases caused by protein aggregates, HIV infections, malaria, leishmaniasis, infections by protozoa, fungi, phytotoxic agents, viruses and parasites, autoimmune diseases, chronic immune reactions against the host, hypertrophy and cardiac decompensation, fibrotic diseases of the skin, fibrosis, spinal and bulbar muscular atrophy, bipolar disorders, psychiatric disorders, fragile X syndrome, arthritis, renal diseases, psoriasis, intestinal and colitic diseases, beta thalassemia, respiratory diseases, Rubinstein-Taybi syndrome.

In the aforesaid uses and methods, the dosage of the compounds of formula (I), (Ia), (Ib), (Ic), can vary on the basis of patient type and condition, the degree of disease severity, administration route selected and the number of daily administrations given etc. As an indication, they can be administered within a dose range of between 0.001 and 1000 mg/kg/day.

The invention also comprises pharmaceutical compositions characterized by containing one or more active principles of formula (**I**), (**Ia**), (**Ib**), (**Ic**), in association with pharmaceutically acceptable excipients and diluents.

The compounds of formula (**I**), (**Ia**), (**Ib**), (**Ic**) can also be used in combination with additional anti-tumor agents and differentiating agents, for non-exclusive example retinoic acid, either by separate administrations, or by including the two active principles in the same pharmaceutical formulation.

The compounds of formula **(I)**, **(Ia)**, **(Ib)**, **(Ic)** can be pharmaceutically formulated according to known methods. The pharmaceutical compositions can be chosen on the basis of the treatment requirements. Such compositions are prepared by blending and are suitably adapted to oral or parenteral administration, and as such can be administered in the form of tablets, capsules, oral preparations, powders, granules, pills, injectable or infusible liquid solutions, suspensions or suppositories.

Tablets and capsules for oral administration are normally presented in unit dose form and contain conventional excipients such as binders, fillers, diluents, tableting agents, lubricants, detergents, disintegrants, coloring agents, flavoring agents and wetting agents. The tablets can be coated with methods well known in the art.

Suitable fillers include cellulose, mannitol, lactose and other similar agents. Suitable disintegrants include, polyvinylpyrrolidone and starch derivatives such as sodium glycolate starch. Suitable lubricants include, for example, magnesium stearate. Suitable wetting agents include sodium lauryl sulfate.

These oral solid compositions can be prepared by conventional methods of blending, filling or tableting. The blending operation can be repeated to distribute the active principle throughout compositions containing large quantities of fillers. Such operations are conventional.

Oral liquid preparations can be in the form of, for example, aqueous or oily suspensions, solutions, emulsions, syrups or elixirs, or can be presented as a dry product for reconstitution with water or with a suitable vehicle before use. Such liquid preparations can contain conventional additives such as suspending agents, for example sorbitol, syrup, methyl cellulose, gelatin, hydroxyethyl cellulose, carboxymethyl cellulose, aluminium stearate gel, or hydrogenated edible fats; emulsifying agents, such as lecithin, sorbitan monooleate, or acacia; non-aqueous vehicles (which can include edible oils), such as almond oil, fractionated coconut oil, oily esters such as esters of glycerine, propylene glycol, or ethyl alcohol; preservatives, such as methyl or propyl p-hydroxybenzoate or sorbic acid, and if desired, conventional flavoring or coloring agents.

Oral formulations also include conventional slow-release formulations such as enterically coated tablets or granules.

For parenteral administration, fluid unit dosages can be prepared, containing the compound and a sterile vehicle. The compound can be either suspended or dissolved, depending on the vehicle and concentration. The parenteral solutions are normally prepared by dissolving the compound in a vehicle and filter sterilizing before filling suitable vials or ampoules and sealing them. Advantageously, adjuvants such as local anaesthetics, preservatives and buffering agents can also be dissolved in the vehicle. To increase stability, the composition can be frozen after having filled the vials and removed the water *in vacuo.* Parenteral suspensions are prepared in substantially the same manner, except that the compound can be suspended in the vehicle instead of being dissolved, and sterilized by exposure to ethylene oxide before suspending in the sterile vehicle. Advantageously, a surfactant or wetting agent can be included in the composition to facilitate uniform distribution of the compound of the invention.

Another means of administering the compounds of the invention regards topical treatment. Topical formulations can contain for example ointments, creams, lotions, gels, solutions, pastes and/or can contain liposomes, micelles and/or microspheres. Examples of ointments include oleaginous ointments such as vegetable oils, animal fats, semisolid hydrocarbons, emulsifiable ointments such as hydroxystearin sulfate, anhydrous lanolin, hydrophilic petrolatum, cetyl alcohol, glycerol monostearate, stearic acid, water soluble ointments containing polyethylene glycols of various molecular weights. A reference for the formulations is the book by Remington ("Remington: The Science and Practice of Pharmacy", Lippincott Williams & Willcins, 2000). Creams, as known to formulation experts, are viscous liquids or semisolid emulsions, and contain an oil phase, an emulsifier and an aqueous phase. The oil phase generally contains petrolatum and an alcohol such as cetyl or stearic alcohol. The emulsifier in a cream formulation is chosen from non-ionic, anionic, cationic or amphoteric surface-active agents. The monophasic gels contain the organic molecules uniformly distributed in the liquid, which is generally aqueous, but they also preferably contain an alcohol and optionally an oil. Preferred gelling agents are cross-linked acrylic acid polymers (e.g. carbomer-type polymers, such as carboxypolyalkylenes, which are commercially available under the Carbopol^{™} trademark). Hydrophilic polymers are also preferred, such as polyoxyethylene, polyoxyethylene-polyoxypropylene copolymers and polyvinyl alcohol; cellulose polymers such as hydroxypropyl cellulose, hydroxyethyl cellulose, hydroxypropyl methylcellulose, hydroxypropyl methylcellulose phthalate and methylcellulose; gums, such as xanthan gum and tragacanth gum; sodium alginate; and gelatin. Dispersing agents such as alcohol or glycerin can be added for gel preparation. The gelling agent can be dispersed by finely chopping and/or mixing.

A further method of administering the compounds of the invention regards transdermal delivery. Typical transdermal formulations comprise conventional aqueous and non-aqueous vectors, such as creams, oils, lotions or pastes or can be in the form of membranes or medicated patches. One formulation provides that a compound of the invention is dispersed within a pressure sensitive patch which adheres to the skin. This formulation enables the compound to diffuse from the patch to the patient through the skin. For a constant release of the drug through the skin, natural rubber and silicon can be used as pressure sensitive adhesives.

As is common practice, the compositions are normally accompanied by written or printed instructions for use in the treatment in question.

The invention is described hereinafter by means of the following examples.

### EXPERIMENTAL PART

### 1. CHEMICAL SYNTHESIS

### Methods

Unless otherwise indicated, all the starting reagents were found to be commercially available and were used with no further purification. Specifically, the following abbreviations may have been used in the descriptions of the experimental methods.

| | |
|---|---|
| g (grams) | NMR (Nuclear Magnetic Resonance) |
| mg (milligrams) | ¹H (proton) |
| ml (millilitres) | MHz (Megahertz) |
| M (molarity) | Hz (Hertz) |
| µl (microlitres) | LC-MS (Liquid Chromatography Mass Spectrum) |
| mmol (millimoles) | rt (retention time in minutes) |
| THF (tetrahydrofuran) | TEA (triethylamine) |
| EtOAc (ethyl acetate) | NH₂OTH (O-(tetrahydro-2H-pyran-2-yl)hydroxylamine) |
| MeOH (methanol) | HOBT (1-hydroxybenzotriazole) |
| EtOH (ethanol) | AcOH (acetic acid) |
| DCM (dichloromethane) | Pd(OAc)₂ (palladium acetate) |
| DMF (dimethylformamide) | DMSO-d₆ (deuterated dimethyl sulfoxide) |
| EDC (1-3(dimethylaminopropyl)-3-ethylcarbodiimide hydrochloride) | BOC (tert-butoxycarbonyl) |
| Et₂O (diethyl ether) | n-BuLi (*n-*butyllithium) |

Except where indicated otherwise, all temperatures are expressed in °C (degrees centigrade).

The ¹H-NMR spectra were acquired with a Brucker 300 MHz. The chemical shifts are expressed in parts per million (ppm, δ units). The coupling constants are expressed in Hertz (Hz) and the splitting patterns are described as s (singlet), d (doublet), t (triplet), q (quartet), quint (quintet), m (multiplet), bs (broad singlet). The LC-MS analyses were carried out according to the following methods:
METHOD A: Waters Acquity UPLC, Micromass ZQ single quadruple (Waters). Atlantis dC18 Column (100x2,1 mm, 3 µm);
   flow: 0.3 ml/min splitting ratio MS:waste/1:4;
   mobile phase: phase A = H₂O/CH₃ (95/5, v/v) + 0.1% TFA; phase B = H₂O/CH₃ (5/95, v/v) + 0.1% TFA. 0-0.5 min (A: 95%, B: 5%), 0.5-7 min (A: 0%, B: 100%), 7-8 min (A: 0%, B: 100%), 8-8.10 min (A: 95%, B: 5%), 8.10-9.50 min (A: 95%, B: 5%); UV wavelength 254 nm or BPI; injection volume: 5 µl
METHOD B: Waters Acquity UPLC, Micromass ZQ single quadruple (Waters). Acquity UPLC-BEH C18 Column (50x2.1 mm, 1.7 µm);
   flow: 0.4 ml/min splitting ratio MS:waste/1:4;
   mobile phase: phase A = H₂O/CH₃CN (95/5, v/v) + 0.1% TFA; phase B= H₂O/CH₃CN (5/95, v/v) + 0.1 % TFA. 0-0.25 min (A: 98%, B: 2%), 0.25-4.0 min (A: 0%, B: 100%), 4.0-5.0 min (A: 0%, B: 100%), 5-5.10 min (A: 98%, B: 2%), 5.10-6 min (A: 98%; B: 2%); UV wavelength 254 nm or BPI; injection volume: 5 µl
METHOD C: Waters Acquity UPLC, Micromass ZQ Single quadrupole (Waters). Column Acquity UPLC-BEH C18 (50x2.1 mm, 1.7 µm);
   Flow rate: 0.6 ml/min splitting ratio MS: waste/1:4;
   Mobile phase: A phase= water/CH₃CN 95/5 + 0.1% TFA; B phase= water/CH₃CN 5/95 +0.1% TFA. 0-0.25 min (A: 98%, B: 2%), 0.25-3.30 min (A: 0%, B: 100%), 3.30-4.00 min (A: 0%, B: 100%), 4.00-4.10 min (A: 98%, B: 2%);4.10-5.00 min (A: 98%, B: 2%) UV detection wavelength 254 nm or BPI; Injection volume: 5µl
METHOD D: Waters Acquity UPLC, Micromass ZQ Single quadrupole (Waters). Column Ascentis (100x2.1 mm, 3 µm);
   Flow rate: 0.3 ml/min splitting ratio MS: waste/1:4;
   Mobile phase: A phase= water/CH₃CN 95/5 +0.1% TFA; B phase= water/CH₃CN 5/95 + 0.1% TFA. 0-0.5 min (A: 95%, B: 5%), 0.5-7 min (A: 0%, B: 100%), 7.00-8.00 min (A: 0%, B: 100%), 8.00-8.10 min (A: 95%, B: 5%);8.10-9.50 min (A: 95%, B: 5%) UV detection wavelength 254 nm or BPI; Injection volume: 5µl
METHOD E: Waters Acquity UPLC, Micromass ZQ Single quadrupole (Waters). Column Acquity UPLC-BEH C18 (50x2.1 mm, 1.7 µm);
   Flow rate: 0.6 ml/min splitting ratio MS: waste/1:4;
   Mobile phase: A phase= water/CH₃CN 95/5 + 0.1% TFA; B phase= water/CH₃CN 5/95 +0.1% TFA. 0-0.5 min (A: 98%, B: 2%), 0.5-6 min (A: 0%, B: 100%), 6.00-7.00 min (A: 0%, B: 100%), 7.00-7.10 min (A: 98%, B: 2%);7.10-8.50 min (A: 98%, B: 2%) UV detection wavelength 254 nm or BPI; Injection volume: 5µl
METHOD F: Waters Acquity UPLC, Micromass ZQ Single quadrupole (Waters). Column Acquity UPLC-BEH C18 (50x2.1 mm, 1.7 µm);
   Flow rate: 0.6 ml/min splitting ratio MS: waste/1:4;
   Mobile phase: A phase= water/CH₃CN 95/5 + 0.1% TFA; B phase= water/CH₃CN 5/95 + 0.1% TFA. 0-0.25min (A: 95%, B: 5%), 0.25-3.30 min (A: 0%, B: 100%), 3.30-4.00 min (A: 0%, B: 100%), 4.00-4.10 min (A: 95%, B: 5%);4.10-5.00 min (A: 95%, B: 5%) UV detection wavelength 254 nm or BPI; Injection volume: 5µl

All the mass spectra were acquired with the ESI mode.

Most of the reactions were monitored by thin layer chromatography (TLC) with 0.2 mm Merck silica gel plates (60F-254), visualized with UV light (254 nm). The chromatographic columns were packed with Merck silica gel 60 (0.04-0.063 mm).

### Example 1: (E)-N-Hydroxy-3-(4-{(E)-3-[2-(4-methyl-piperazin-1-yl)-phenyl]-3-oxo-propenyl}-phenyl)-acrylamide

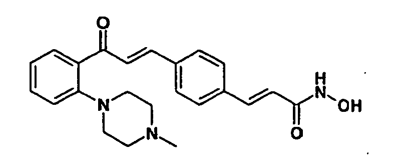

### STEP A

A mixture of 2-fluoro benzonitrile (2.28 g, 18.84 mmol), 1-methyl piperazine (3.14 ml, 28.26 mmol) and finely ground K₂CO₃ (3.19 g, 23 mmol) in DMSO (50 ml) was heated to 120°C for 24 hours.

The mixture was then diluted with H₂O and extracted twice with AcOEt. The pooled organic phases were dried over Na₂SO₄ and evaporated *in vacuo.*

The crude product was purified by flash chromatography (DCM:MeOH:NH₃ 97:3:0.3), the product obtained was dissolved in DCM and treated with HCl in Et₂O.

The resulting precipitate was filtered off and washed with DCM to obtain 3.15 g of 2-(4-methyl-piperazin-1-yl)-benzonitrile hydrochloride. Y = 70%

### STEP B

A solution of 2-(4-methyl-piperazin-1-yl)-benzonitrile hydrochloride (2.16 g, 9.1 mmol) in H₂O was brought to basic conditions with NH₄OH and extracted with DCM. The organic phase was dried over Na₂SO₄, and evaporated *in vacuo.* The product obtained (1.77 g, 8.80 mmol) was dissolved in 30 ml of toluene and added under nitrogen atmosphere to a solution of 3 M methyl magnesium bromide in diethyl ether (8.79 ml, 26.38 mmol). The resulting suspension was heated under reflux for 4 hours. The reaction was cooled down to 0°C, acidified with 10% HCl, and then heated under reflux for 1 hour. The two phases were separated and the aqueous phase was extracted with AcOEt, then brought to basic conditions with NH₄OH and extracted with DCM. The organic phase was dried over Na₂SO₄ and concentrated *in vacuo* to dryness. The crude product was purified by flash chromatography (DCM:MeOH:NH₃ 98:2:0.2) to obtain 1.62 g of 1-[2-(4-methyl-piperazin-1-yl)-phenyl]-ethanone.
Y=84%

### STEP C

A mixture of 1-[2-(4-methyl-piperazin-1-yl)-phenyl]-ethanone (542 mg, 2.48 mmol), 4-formyl cinnamic acid (438 mg, 2.48 mmol) and 1.7 M KOH (2.92 ml) in H₂O (5 ml) and EtOH (5 ml) was stirred at room temperature for 24 hours.

The mixture was then acidified with 10% HCl and the resulting yellow precipitate was filtered off through a Buchner funnel to obtain 0.93 g of (E)-3-(4-{(E)-3-[2-(4-methyl-piperazin-1-yl)-phenyl]-3-oxo-propenyl}-phenyl)-acrylic acid hydrochloride. Y = 90%

### STEP D

A mixture of (E)-3-(4-{(E)-3-[2-(4-methyl-piperazin-1-yl)-phenyl]-3-oxo-propenyl}-phenyl)-acrylic acid hydrochloride (250 mg, 0.608 mmol), NH₂OTHP (85.4 mg, 0.73 mmol), EDC (232 mg, 1.22 mol), HOBT (164 mg, 1.22 mmol) and TEA (253 µl, 1.82 mmol) in THF (5 ml) and DMF (5 ml) was stirred for 24 hours at room temperature. The mixture was then diluted with water and extracted with AcOEt. The organic phase was washed with water, then with a saturated NaCl solution, dried over Na₂SO₄ and evaporated *in vacuo* to dryness.

The crude product was purified by flash chromatography (DCM:MeOH:NH₃ 98:2:0.2). The product obtained was dissolved in DCM and treated with HCl in Et₂O for 1 hour to obtain precipitation of a yellow solid. The solid was then filtered off through a Buchner funnel to obtain 115 mg of (E)-N-hydroxy-3-(4-{(E)-3-[2-(4-methyl-piperazin-1-yl)-phenyl]-3-oxo-propenyl}-phenyl)-acrylamide hydrochloride.
Y = 44%
LC-MS: METHOD A, rt=3.34; (ES+) MH⁺: 392.2
¹H-NMR (DMSO-d₆) δ: 10.90 (bs, 1H); 7.83 (d, 2H); 7.64 (d, 2H); 7.64-7.52 (m, 5H); 7.27 (d, 1H); 7.20 (dd, 1H); 6.56 (d, 1H); 3.50-3.13 (m, 6H); 2.89 (bs, 2H); 2.66 (d, 3H).

The compounds in table 1 were obtained by following the aforedescribed procedure (steps A-D or C-D when the intermediates were found to be commercially available).

**Table 1**

| Ex no | structure | Compound name | MH⁺ | ¹H-NMR (DMSO-d₆) δ: |
|---|---|---|---|---|
| 2 | 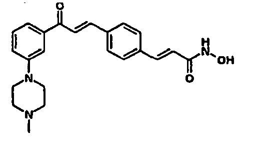 | (E)-N-Hydroxy-3-(4-{(E)-3-[3-(4-methyl-piperazin-1-yl)-phenyl]-3-oxo-propenyl}-phenyl)-acrylamide | 392.3 | 11.08 (bs, 1H); 7.94 (d, 1H); 7.93 (d, 2H); 7.74 (d, 1H); 7.66 (m, 4H); 7.50 (d, 1H); 7.47 (dd, 1H); 7.33 (dd, 1H); 6.60 (d, 1H); 3.96 (d br, 2H); 3.51 (d br 2H); 3.20 (m, 4H); 2.82 (d, 3H) |
| 3 | 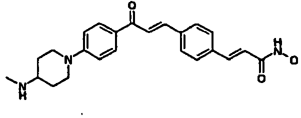 | (E)-N-Hydroxy-3-(4-{(E)-3-[4-(4-methylamino-piperidin-1-yl)-phenyl]-3-oxo-propenyl}-phenyl)-acrylamide | 406.50 | |
| 4 | 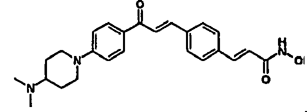 | (E)-3-(4-{(E)-3-[4-(4-Dimethylamino-piperidin-1-yl)-phenyl]-3-oxo-propenyl}-phenyl)-N-hydroxy-acrylamide | 420.3 | 10.89 (bs, 1H); 8.07 (d, 2H); 7.95 (d, 1H); 7.91 (d, 2H); 7.66 (d, 1H); 7.63 (d, 2H); 7.49 (d, 1H); 7.07 (d, 2H); 6.59 (d, 1H); 4.16 (d br, 2H); 3.40 (m, 1H); 2.91 (dd, 2H); 2.72 (d, 6H); 2.14 (d br, 2H); 1.69 (m, 2H) |
| 5 | 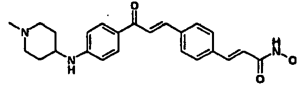 | (E)-N-Hydroxy-3-(4-{(E)-3-[4-(1-methyl-piperidin-4-yl-amino)-phenyl]-3-oxo-propenyl}-phenyl)-acrylamide | 406.50 | |
| 6 | 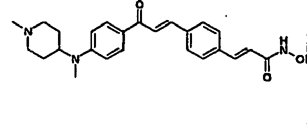 | (E)-N-Hydroxy-3-[4-(E)-3-{4-[N-methyl-(1-methyl-piperidin-4-yl)-amino]-phenyl}-3-oxo-propenyl)-phenyl]-acrylamide | 420.3 | |

The compound of **Example 5** can be alternatively prepared by the following procedure:

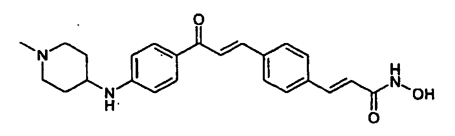

### STEP A

A mixture of 1-(4-bromo-phenyl)-ethanone (1 g, 5.02 mmol), *tert*-butyl 4-formyl cinnamate (1.16 g, 5.02 mmol), KOH (560 mg, 1.04 mmol) in EtOH (20 ml) and H₂O (5 ml), was stirred at room temperature for 4 h. The mixture was then diluted with water and the resulting precipitate was filtered to give 1.7 g of tert-butyl (E)-3-{4-[(E)-3-(4-bromo-phenyl)-3-oxo-propenyl]-phenyl}-acrylate as yellow powder. Y=81 %

### STEP B

A mixture of (±)BINAP (435 mg, 0.7 mmol) and Pd₂(dba)₃ (320 mg, 0.35 mmol) in toluene (10 ml) was heated to 80 °C for 1 h under N₂. Then the mixture was cooled down to room temperature and tert-butyl (E)-3-{4-[(E)-3-(4-bromo-phenyl)-3-oxo-propenyl]-phenyl}-acrylate (726 mg, 1.75 mmol), 1-methyl-piperidin-4-ylamine (0.200, 1.75 mmol) and NaOtBu (252 mg, 2.6 mmol) were added. The reaction was refluxed overnight under N₂, then the slurry was filtrated (Celite) and the organic filtrate was evaporated *in vacuo.* The crude reaction mixture was purified by column chromatography (eluent: petroleum ether/AcOEt 1:1). The resulting product was dissolved in DCM (1 ml) and TFA (1 ml) and the solution was stirred at room temperature for 4 h. The solvent was removed *in vacuo* to give 280 mg of (E)-3-(4-{(E)-3-[4-(1-methyl-piperidin-4-ylamino)-phenyl]-3-oxo-propenyl}-phenyl)-acrylic acid as its trifluoroacetate salt.
Y=32%

### STEP C

A mixture of (E)-3-(4-{(E)-3-[4-(1-methyl-piperidin-4-ylamino)-phenyl]-3-oxo-propenyl}-phenyl)-acrylic acid trifluoroacetate (126 mg, 0.25 mmol), HOBT (40 mg, 0.30 mmol), EDC (58 mg, 0.30 mmol), TEA (0.10 ml, 0.75 mmol) and NH₂OTHP (30 mg, 0.25 mmol) in THF (5 ml) and DMF (1 ml), was stirred at room temperature overnight and then partitioned between water and AcOEt. The organic extract was dried over Na₂SO₄ and evaporated *in vacuo.* The crude product was triturated in CH₃CN/MeOH 9:1 and filtered to give a brown powder that was dissolved in DCM and treated with HCl/Et₂O for 2 h. The precipitate was filtered off to give 65 mg of (E)-N-hydroxy-3-(4-{(E)-3-[4-(1-methyl-piperidin-4-ylamino)-phenyl]-3-oxo-propenyl}-phenyl)-acrylamide as its hydrochloride salt.
Y= 60%
LC-MS: Method C, rt=1.29; (ES+) MH+: 406.24
1H NMR (DMSO-d₆) δ (ppm): 10.42 (bs, 1 H), 10.22 (s, 1 H), 7.99 (m, 2 H), 7.90 (d, 1 H), 7.87 (m, 2 H), 7.56 - 7.70 (m, 3 H), 7.48 (d, 1H), 6.70 (m, 2 H), 6.56 (d, 1 H), 3.56 - 3.69 (m, 1 H), 3.36 - 3.53 (m, 2 H), 2.92 - 3.33 (m, 2 H), 2.74 (d, 3 H), 2.00-2.19 (m, 2 H), 1.67- 1.94 (m, 2 H).

### Example 7: (E)-N-Hydroxy-3-(4-{(E)-3-[4-(4-methyl-piperazin-1-yl-methyl)-phenyl]-3-oxo-propenyl}-phenyl)-acrylamide

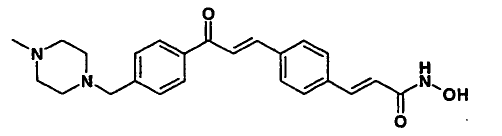

### STEP A

1-methyl piperazine (805 µl, 7.6 mmol) was added to a solution of 4-(bromomethyl)benzonitrile (1 g, 5.1 mmol) and TEA (1.4 ml, 10.2 mmol) in DCM (15 ml) and the resulting mixture was stirred at room temperature for 24 hours.

The solution was then diluted with DCM, washed with a 5% NaHCO₃ solution and then with H₂O. The organic phase was dried over Na₂SO₄ and evaporated to dryness to give 0.73 g of 4-(4-methyl-piperazin-1-yl)-benzonitrile as a white solid. Y = 67%

### STEP B

0.73 g of 4-(4-methyl-piperazin-1-yl-methyl)-benzonitrile (3.40 mmol) were dissolved in toluene (13 ml) and added to a solution of 3 M methyl magnesium bromide in diethyl ether (3.4 ml, 10.2 mmol) under nitrogen atmosphere. The resulting suspension was heated under reflux for 4 hours. The reaction was cooled down to 0°C, acidified with 10% HCl and then heated under reflux for 1 hour. The two phases were separated and the aqueous phase rinsed with AcOEt, then brought to basic conditions with NH₄0H and extracted with DCM. The organic phase was dried over Na₂SO₄ and concentrated *in vacuo* to dryness to obtain 0.71 g of 1-[4-(4-methyl-piperazin-1-yl-methyl)-phenyl]-ethanone as a yellow oil.
Y = 90%

### STEP C

A mixture of 1-[4-(4-methyl-piperazin-1-yl-methyl)-phenyl]-ethanone (392 mg, 1.69 mmol), 4-formyl cinnamic acid (300 mg, 1.69 mmol) and 1.7 M KOH (2.0 ml, 3.4 mmol) in H₂O (5 ml) and EtOH (5 ml) was stirred at room temperature for 24 hours.

The mixture was then acidified with 10% HCl and the resulting yellow precipitate was filtered off through a Buchner funnel to obtain 542 mg of (E)-3-(4-{(E)-3-[4-(4-methyl-piperazin-1-yl-methyl)-phenyl]-3-oxo-propenyl}-phenyl)-acrylic acid dihydrochloride.
Y = 69%

### STEP D

A mixture of (E)-3-(4-{(E)-3-[4-(4-methyl-piperazin-1-yl-methyl)-phenyl]-3-oxo-propenyl}-phenyl)-acrylic acid dihydrochloride (542 mg, 1.17 mmol), NH₂OTHP (164 mg, 1.4 mmol), EDC (447 mg, 2.34 mol), HOBT (316 mg, 2.34 mmol) and TEA (488 µl, 3.51 mmol) in THF (5 ml) and DMF (5 ml) was stirred for 24 hours at room temperature. The mixture was then diluted with water and extracted with AcOEt. The organic phase was washed with water, then with a saturated NaCl solution, dried over Na₂SO₄ and evaporated *in vacuo* to dryness.

The crude product was purified by flash chromatography (DCM:MeOH:NH₃ 98:2:0.2). The product obtained was dissolved in DCM and treated with HCl in Et₂O for 1 hour to obtain precipitation of a yellow solid. The solid was then filtered off through a Buchner funnel to obtain 300 mg of (E)-N-hydroxy-3-(4-{(E)-3-[4-(4-methyl-piperazin-1-yl-methyl)-phenyl]-3-oxo-propenyl}-phenyl)-acrylamide dihydrochloride.
Y = 53%
LC-MS: METHOD A, rt= 3.02; (ES+) MH⁺: 406.2
¹H-NMR (DMSO-d₆) δ: 11.74 (bs, 1 H); 10.12 (bs, 1H); 8.23 (d, 2H); 7.99 (d, 1 H); 7.94 (d, 2H); 7.86 (d, 2H); 7.77 (d, 1H); 7.65 (d, 2H); 7.49 (d, 1H); 6.59 (d, 1H); 4.45 (d, 2H); 3.70-3.17 (m, 8H); 2.81 (s, 3H).

The compounds in Table 2 were obtained by following the aforedescribed procedure.

**Table 2**

| Ex no | Structure | Compound name | MH⁺ | ¹H-NMR (DMSO-d₆) δ: |
|---|---|---|---|---|
| 8 | 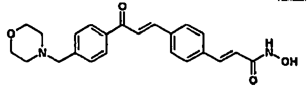 | (E)-N-Hydroxy-3-{4-[(E)-3-(4-morpholin-4-yl-methyl-phenyl)-3-oxo-propenyl]-phenyl}-acrylamide | 393.2 | 11.25 (bs, 1H); 10.83 (bs, 1H); 8.24 (d, 2H); 7.99 (d, 1H); 7.94 (d, 2H); 7.82 (d, 2H); 7.77 (d, 1H); 7.66 (d, 2H); 7.49 (d, 1H); 6.58 (d, 1H); 4.44 (s, 2H); 4.03-3.72 (m, 4H); 3.32-3.00 (m, 4H). |
| 9 | 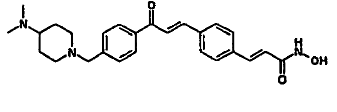 | (E)-3-(4-{(E)-3-[4-(4-Dimethylamino-piperidin-1-yl-methyl)-phenyl]-3-oxo-propenyl}-phenyl)-N-hydroxy-acrylamide | 434.5 | (DMSO-d6 + TFA): 11.15 (bs, 1H); 10.88 (bs, 1H); 8.25 (d, 2H); 8.00 (d, 1H); 7.95 (d, 2H); 7.82 (d, 2H); 7.77 (d, 1H); 7.66 (d, 2H); 7.50 (d, 1H); 6.60 (d, 1H); 4.42 (s, 2H); 3.58-3.26 (m, 2H); 3.01 (m, 2H); 2.87-2.62 (m, 7H); 2.32-202 (m, 4H). |

### Example 10: (E)-N-Hydroxy-3-(4-{(E)-3-[4-(1-methyl-piperidin-4-yl)-phenyl]-3-oxo-propenyl}-phenyl)-acrylamide

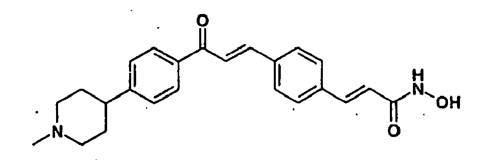

### STEP A

A mixture of 4-phenyl piperidine hydrochloride (1.23 g, 6.2 mmol), formaldehyde (36.5% in H₂O, 0.702 ml, 9.3 mmol), NaBH(OAC)₃ (2.63 g, 12.42 mmol) and AcOH (0.71 ml, 12.42 mmol) in DCM (30 ml) was stirred for 24 hours at room temperature. The mixture was then brought to basic conditions with a 5% NaHCO₃ solution and extracted twice with DCM. The pooled organic phases were dried over Na₂SO₄ and evaporated to dryness. The residue was dissolved in DCM and treated with HCl in Et₂O observing the precipitation of a white solid. The solid was filtered off through a Buchner funnel to obtain 1 g of 1-methyl-4-phenylpiperidine hydrochloride as a white solid.
Y=77%

### STEP B

Acetyl chloride (0.634 ml, 8.92 mmol) and AlCl₃ (991 mg, 7.43 mmol) were added to a solution of 1-methyl-4-phenylpiperidine hydrochloride (786 mg, 3.7 mmol) in DCM (16 ml). The resulting mixture was heated to reflux under nitrogen atmosphere for 5 hours during which further 0.53 ml of acetyl chloride and 991 mg of AlCl₃ were added.

The reaction was then cooled down to room temperature and slowly quenched with H₂O. The mixture was brought to basic conditions with a 5% K₂CO₃ solution and extracted with DCM. The organic phase was dried over Na₂SO₄ and evaporated to dryness.

The crude product was purified by flash chromatography (DCM:MeOH:NH₃ 98:2:0.2).

The resulting oil was dissolved in DCM and treated with HCl in Et₂O observing the precipitation of a white solid. The solid was filtered through a Buchner funnel to give 873 mg of 1-[4-(1-methyl-piperidin-4-yl)-phenyl]-ethanone hydrochloride as a white solid.
Y = 93%

### STEP C

A mixture of 1-[4-(1-methyl-piperidin-4-yl)-phenyl]-ethanone hydrochloride (544 mg, 2.15 mmol), 4-formylcinnamic acid tert-butyl ester (500 mg, 2.15 mmol) and 1.7 M KOH (3.8 ml, 6.46 mmol) in EtOH (10 ml) was stirred at room temperature for 6 hours. During the reaction, the formation of a precipitate was observed. The solid was then filtered off through a Buchner funnel to obtain 270 mg of (E)-3-(4-{(E)-3-[4-(1-methyl-piperidin-4-yl)-phenyl]-3-oxo-propenyl}-phenyl)-acrylic acid tert-butyl ester as a yellow solid.
Y=29%

### STEP D

2 ml of trifluoroacetic acid were added to a solution of (E)-3-(4-{(E)-3-[4-(1-methyl-piperidin-4-yl)-phenyl]-3-oxo-propenyl}-phenyl)-acrylic acid tert-butyl ester (270 mg, 0.63 mmol) in 10 ml of DCM. The solution was stirred at room temperature for 2 hours. The solvent was then removed until dryness and 300 mg of (E)-3-(4-{(E)-3-[4-(1-methyl-piperidin-4-yl)-phenyl]-3-oxo-propenyl}-phenyl)-acrylic acid trifluoro acetate were obtained as a yellow solid.
Y = 98%

### STEP E

A mixture of (E)-3-(4-{(E)-3-[4-(1-methyl-piperidin-4-yl)-phenyl]-3-oxo-propenyl}-phenyl)-acrylic acid trifluoro acetate (310 mg, 0.634 mmol), NH₂OTH (89 mg, 0.76 mmol), EDC (242 mg, 1.268 mmol), HOBT (172 mg, 1.268 mmol) and TEA (176 µl, 1.268 mmol) in THF (5 ml) and DMF (5 ml), was stirred for 24 hours at room temperature. The mixture was then diluted with water and extracted with AcOEt. The organic phase was then washed with water, with a saturated NaCl solution and then dried over Na₂SO₄ and evaporated *in vacuo* to dryness.

The crude product was purified by flash chromatography (DCM:MeOH:NH₃ 98:2:0.2). The product obtained was dissolved in DCM and treated with HCl in Et₂O for 1 hour to obtain the precipitation of a yellow solid. The solid was then filtered off through a Bucher funnel and purified by preparative HPLC-MS to obtain 20 mg of (E)-N-Hydroxy-3-(4-{(E)-3-[4-(1-methyl-piperidin-4-yl)-phenyl]-3-oxo-propenyl}-phenyl)-acrylamide trifluoro acetate.
Y = 6%
LC-MS: METHOD A, rt= 3.31; (ES+) MH⁺: 391.2
¹H-NMR (DMSO-d₆) δ: 10.78 (bs, 1H); 9.35 (bs, 1H); 9.06 (s, 1H); 8.15 (d, 2H); 7.96 (d, 1H); 7.92 (d, 2H); 7.74 (d, 1H); 7.66 (d, 2H); 7.50 (d, 1H); 7.45 (d, 2H); 6.57 (d, 1H); 3.55 (m, 2H); 3.09 (m, 2H); 2.93 (m, 1H); 2.84 (s, 3H); 2.15-1.76 (m, 4H).

### Example 11: (E)-N-Hydroxy-3-(5-{(E)-3-[4-(4-methyl-piperazin-1-yl)-phenyl]-3-oxo-propenyl}-pyridin-2-yl)-acrylamide

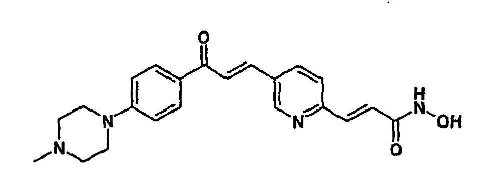

### STEP A

A solution of 6-bromopyridine 3-carbaldehyde (3.07 g, 16.5 mmol), p-toluenesulfonic acid (386 mg, 2.02 mmol) and trimethyl orthoformate (1.97 ml, 18 mmol) in MeOH (80 ml) was stirred at room temperature for 48 hours. The mixture was then brought to basic conditions with a 5% NaHCO₃ solution and extracted twice with diethyl ether. The pooled organic phases were dried over Na₂SO₄ and evaporated to dryness to give 3.66 g of 2-bromo-5-dimethoxymethyl-pyridine as a pale yellow oil.
Y= 95%

### STEP B

A solution of 2.5 M BuLi in hexane (7.6 ml) was added drop-wise, under a N₂ atmosphere, to a solution of 2-bromo-5-dimethoxymethyl-pyridine (3.66 g, 15.84 mmol) in THF (60 ml) at -70°C. After 15 minutes, DMF (1.82 ml, 23.5 mmol) was added drop-wise and the mixture was stirred for 30 minutes at -70°C and then allowed to reach room temperature. The reaction was diluted with H₂O and extracted with DCM. The organic phase was dried over Na₂SO₄ and evaporated to dryness *in vacuo.* The crude product was purified by flash chromatography (petroleum ether: AcOEt 7:3) to obtain 1.54 g of 5-dimethoxymethyl-pyridine-2-carbaldehyde as a yellow oil.
Y=54%

### STEP C

A solution of 5-dimethoxymethyl-pyridine-2-carbaldehyde (1.54 g, 8.50 mmol) in 20 ml of THF was added drop-wise under nitrogen to a mixture of tert-butyl diethyl phosphonoacetate (2.36 g, 9.36 mmol) and NaH (60%, 442 mg, 11.06 mmol) in THF (20 ml). The resulting solution was stirred at room temperature for 1 hour, then slowly diluted with H₂O and extracted twice with diethyl ether. The pooled organic phases were dried over Na₂SO₄ and evaporated to dryness. The crude product was purified by flash chromatography (petroleum ether: AcOEt 92:8) to obtain 1.76 g of (E)-3-(5-dimethoxymethyl-pyridin-2-yl)-acrylic acid tert-butyl ester as a pale yellow oil.
Y = 74%

### STEP D

A mixture of (E)-3-(5-dimethoxymethyl-pyridin-2-yl)-acrylic acid tert-butyl ester (1.76 mg, 6.30 mmol) in THF (30 ml) and 1 M HCl (25 ml) was stirred for 4 hours at room temperature, then brought to basic conditions with a 5% NaHCO₃ solution and extracted twice with Et₂O. The pooled organic phases were dried over Na₂SO₄ and evaporated *in vacuo* to dryness to give 1.45 g of (E)-3-(5-formyl-pyridin-2-yl)-acrylic acid tert-butyl ester as a white solid.
Y = 98%

### STEP E

A mixture of (E)-3-(5-formyl-pyridin-2-yl)-acrylic acid tert-butyl ester (261 mg, 1.12 mmol), 1-[4-(4-methyl-piperazin-1-yl)-phenyl]-ethanone (246 mg, 1.2 mmol) and KOH (125 mg, 2.24 mmol) in 10 ml of EtOH was stirred at room temperature for 24 hours, observing the formation of a precipitate. The solid was then filtered off through a Buchner funnel to obtain 222 mg of (E)-3-(5-{(E)-3-[4-(4-methyl-piperazin-1-yl)-phenyl]-3-oxo-propenyl}-pyridin-2-yl)-acrylic acid tert-butyl ester.
Y = 45%

### STEP F

A mixture of (E)-3-(5-{(E)-3-[4-(4-methyl-piperazin-1-yl)-phenyl]-3-oxo-propenyl}-pyridin-2-yl)-acrylic acid tert-butyl ester (222 mg, 0.513 mmol) and trifluoro acetic acid (2 ml) in DCM (5 ml) was stirred at room temperature for 5 hours.

The solvent was evaporated *in vacuo* to dryness to give 330 mg of (E)-3-(5-{(E)-3-[4-(4-methyl-piperazin-1-yl)-phenyl]-3-oxo-propenyl}-pyridin-2-yl)-acrylic acid bis trifluoroacetate.
Y = >99%

### STEP G

A mixture of (E)-3-(5-{(E)-3-[4-(4-methyl-piperazin-1-yl)-phenyl]-3-oxo-propenyl}-pyridin-2-yl)-acrylic acid bis trifluoroacetate (330 mg, 0.54 mmol), NH₂OTHP (78 mg, 0.67 mmol), EDC (155 mg, 0.81 mmol), HOBT (109 mg, 0.80 mmol) and TEA (280 µl, 2 mmol) in THF (5 ml) and DMF (5 ml), was stirred for 72 hours at room temperature. The mixture was then diluted with water and extracted with AcOEt. The organic phase was washed with water, then with a saturated NaCl solution, dried over Na₂SO₄ and evaporated *in vacuo* to dryness.

The crude product was purified by flash chromatography (DCM:MeOH:NH₃ 96:4:0.2). The product obtained was dissolved in DCM and treated with HCl in Et₂O for 1 hour to obtain precipitation of a dark brown solid. The solid was then filtered off through a Buchner funnel, washed with DCM to obtain 156 mg of (E)-N-hydroxy-3-(5-{(E)-3-[4-(4-methyl-piperazin-1-yl)-phenyl]-3-oxo-propenyl}-pyridin-2-yl)-acrylamide dihydrochloride.
Y = 62%
LC-MS: METHOD B, rt=1.36; (ES+) MH⁺: 393.3
¹H-NMR (DMSO-d₆) δ: 11.38 (bs, 1H); 9.09 (d, 1H); 8.52 (dd, 1H); 8.19-8.09 (m, 3H); 7.81 (d, 1H); 7.72 (d, 1H); 7.56 (d, 1H); 7.12 (d, 2H); 7.07 (d, 1H); 4.13 (m, 2H); 3.49 (m, 2H); 3.35 (m, 2H); 3.13 (m, 2H); 2.80 (d, 3H).

### Example 12: (E)-N-Hydroxy-3-(4-{(E)-3-[4-(4-isobutyl-piperazin-1-yl)-phenyl]-3-oxo-propenyl}-phenyl)-acrylamide

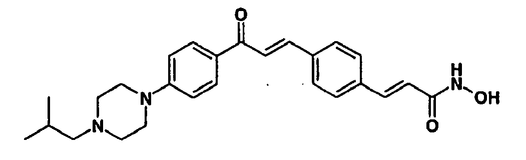

### STEP A

Isobutylaldehyde (0.230 ml, 2.94 mmol) and Na(CH₃CO₂)₃BH (620 mg, 2.94 mmol) were added at 5°C to a solution of 4-piperazino-acetophenone (500 mg, 2.45 mmol) in 1,2- dichloroethane (10 ml). The resulting mixture was stirred for 4 hours at room temperature.

The mixture was then concentrated *in vacuo,* brought to basic conditions with a 5% NaHCO₃ solution and extracted with AcOEt. The organic phase was dried over Na₂SO₄ and evaporated *in vacuo* to dryness.

The crude product was ground in isopropyl ether, filtered off and then oven dried to obtain 1-[4-(4-isobutyl-piperazin-1-yl)-phenyl]-ethanone.
Y = 80%

### STEP B

A mixture of 1-[4-(4-isobutyl-piperazin-1-yl)-phenyl]-ethanone (520 mg, 2 mmol), 4-formylcinnamic acid (360 mg, 2 mmol) and 1.7 M KOH (2.5 ml) in EtOH (10 ml) was stirred at room temperature for 12 hours.

The product was filtered off and oven dried to give 450 mg of (E)-3-(4-{(E)-3-[4-(4-isobutyl-piperazin-1-yl)-phenyl]-3-oxo-propenyl}-phenyl)-acrylic acid as potassium salt.
Y = 50%

### STEP C

A mixture of (E)-3-(4-{(E)-3-[4-(4-isobutyl-piperazin-1-yl)-phenyl]-3-oxo-propenyl}-phenyl)-acrylic acid as potassium salt (450 mg, 1 mmol), NH₂OTHP (117 mg, 1 mmol), EDC (230 mg, 1.2 mmol), HOBT (160 mg, 1.2 mmol) and TEA (0.420 ml, 3 mmol) in THF (5 ml) and DMF (5 ml) was stirred for 24 hours at room temperature. The mixture was then diluted with water and extracted with AcOEt. The organic phase was washed with water, then with a saturated NaCl solution, dried over Na₂SO₄ and evaporated *in vacuo* to dryness. The crude product was purified by flash chromatography (DCM:MeOH:NH₃ 98:2:0.2). The product was then dissolved in DCM and a few drops of HCl in ether were added. The mixture was stirred for 12 hours at room temperature. The precipitate was then filtered and ground in acetonitrile to obtain 300 mg of (E)-N-hydroxy-3-(4-{(E)-3-[4-(4-isobutyl-piperazin-1-yl)-phenyl]-3-oxo-propenyl}-phenyl)-acrylamide hydrochloride.
Y= 64 %
LC-MS: METHOD B, rt= 2.24; (ES+) MH⁺: 434.5
¹H-NMR (DMSO-d₆) δ: 10.83 (bs, 1H); 10.49 (bs, 1H); 8.12 (d, 2H); 7.97 (d, 1H); 7.91 (d, 2H); 7.68 (d, 1H); 7.64 (d, 2H); 7.48 (d, 1H); 7.11 (d, 2H); 6.59 (d, 1H); 4.08 (m, 2H); 3.62-3.44 (m, 4H); 3.12 (m, 2H); 3.01 (m, 2H); 2.15 (m, 1H); 1.02 (d, 6H).

The compounds in Table 3 were obtained by following the aforedescribed process.

**Table 3**

| Ex no | structure | Compound name | MH⁺ | ¹H-NMR (DMSO-dₛ) δ: |
|---|---|---|---|---|
| 13 | 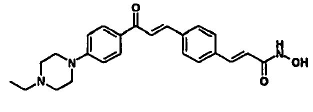 | (E)-3-(4-{(E)-3-[4-(4-Ethyl-piperazin-1-yl)-phenyl]-3-oxo-propenyl}-phenyl)-N-hydroxy-acrylamide | 406.50 | (DMSO-d₆ + TFA): 8.08 (d, 2H); 7.84 (d, 2H); 7.83 (d, 1H); 7.66 (d, 1H); 7.62 (d, 2H); 7.50 (d, 1H); 7.01 (d, 2H); 6.62 (d br, 1H); 4.12-3.24 (m br, 8H); 3.21 (q, 2H); 1.33 (t, 3H). |
| 14 | 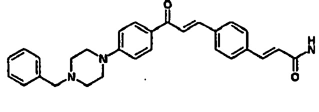 | (E)-3-(4-{(E)-3-[4-(4-Benzyl-piperazin-1-yl)-phenyl]-3-oxo-propenyl}-phenyl)-N-hydroxy-acrylamide | 468,1 | 10.79 (s, 1H); 10.52 (bs, 1H); 9.05 (bs, 1H); 8.11 (d, 2H); 7.96 (d, 1H); 7.90 (d, 2H); 7.74-7.37 (m, 9H); 7.08 (d, 2H); 6.55 (d, 1H); 4.41 (s, 2H); 4.14 (m, 2H); 3.41-3.07 (m, 6H). |

### Example 15: (E)-N-Hydroxy-3-{4-[(E)-3-(4-piperazin-1-yl-phenyl)-3-oxo-propenyl]-phenyl}-acrylamide

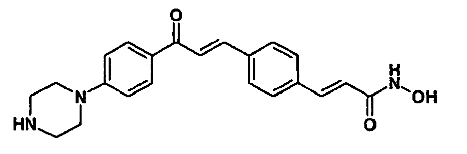

### STEP A

A solution of 4-piperazino-acetophenone (2 g, 9.8 mmol), 4-formyl cinnamic acid (1.72, 9.8 mmol) and 1.7 M KOH (10 ml) in EtOH (20 ml) and H₂O (5 ml) was stirred for 12 hours at room temperature.

10% HCl (30 ml) was then added to the mixture and the precipitate was filtered off and dried *in vacuo* to obtain 3.8 g of (E)-3-{4-[(E)-3-oxo-3-(4-piperazin-1-yl-phenyl)-propenyl]-phenyl}-acrylic acid as hydrochloride.
Y = 97%

### STEP B

A mixture of (E)-3-{4-[(E)-3-(4-piperazin-1-yl-phenyl)-3-oxo-propenyl]-phenyl}-acrylic acid hydrochloride (550 mg, 1.38 mmol), BOC anhydride (361 mg, 1.65 mmol) and triethylamine (0.390 ml, 2.8 mmol) in 1.4-dioxane (5 ml) and H₂O (5 ml) was stirred for 12 hours at room temperature.

The solvent was evaporated *in vacuo* and the residue was ground in di-isopropylether. The solid was then filtered off and oven dried to obtain 638 mg of 4-(4-{(E)-3-[4-((E)-2-carboxy-vinyl)-phenyl]-acryloyl}-phenyl)-piperazino-1-carboxylic acid tert-butyl ester.
Y = quantitative.

### STEP C

A mixture of 4-(4-{(E)-3-[4-((E)-2-carboxy-vinyl)-phenyl]-acryloyl}-phenyl)-piperazino-1-carboxylic acid tert-butyl ester (460 mg, 1 mmol), NH₂OTH (117 mg, 1 mmol), EDC (230 mg, 1.2 mmol), HOBT (160 mg, 1.2 mmol) and TEA (0.420 ml, 3 mmol) in THF (5 ml) and DMF(5 ml) was stirred for 24 hours at room temperature. The mixture was then diluted with water and extracted with AcOEt. The organic phase was washed with water, then with a saturated NaCl solution, dried over Na₂SO₄ and evaporated *in vacuo* to dryness.

The crude product was then dissolved in DCM and a few drops of HCl in ether were added to the solution. The mixture was stirred for 12 hours at room temperature and the resulting precipitate was then filtered off to obtain 180 mg of (E)-N-hydroxy-3-{4-[(E)-3-oxo-3-(4-piperazin-1-yl-phenyl)-propenyl]-phenyl}-acrylamide hydrochloride.
Y = 43%
LC-MS: METHOD A, rt= 3.22; (ES+) MH⁺: 378.1
¹H-NMR (DMSO-d₆) δ: 10.79 (bs, 1H); 9.23 (s, 2H); 8.11 (d, 2H); 7.97 (d, 1H); 7.91 (d, 2H); 7.68 (d, 1H); 7.64 (d, 2H); 7.49 (d, 1H); 7.10 (d, 2H); 6.57 (d, 1H); 3.63 (m, 4H); 3.23 (m, 4H).

### Preparation 1: 1-[4-(1-Methyl-piperidin-4-yloxy)-phenyl]-ethanone

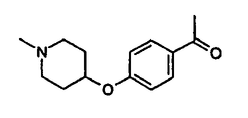

Diethyl azodicarboxylate (1.27 g, 7.35 mmol) was added to a stirred mixture of 1-(4-hydroxy-phenyl)-ethanone (1 g, 7.35 mmol), 1-methyl-piperidin-4-ol (845 mg, 7.35 mmol) and PPh₃ (1.92 g, 7.35 mmol) in THF (50 ml) at 0°C. The resulting brown solution was stirred at 0°C for 1 h and then at room temperature for 4 h. The mixture was partitioned between water and AcOEt and the organic phase was extracted with 1 M HCl. The aqueous phase was brought to basic conditions with NH₄OH and extracted with AcOEt.

The organic phase was dried over Na₂SO₄ and evaporated *in vacuo.* The crude mixture was purified by silica gel chromatography to give 351 mg of 1-[4-(1-methyl-piperidin-4-yloxy)-phenyl]-ethanone.
Y= 20%

### Preparation 2: 1-[4-(1-methyl-piperidin-4-ylmethyl)-phenyl]-ethanone

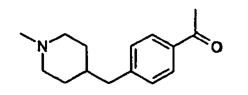

### STEP A

1-Methyl-piperidin-4-one (500 mg, 4.42 mmol) was dissolved in THF (10 ml) and added dropwise to a stirred mixture of (4-cyano-benzyl)-phosphonic acid diethyl ester (1.12 g, 4.42 mmol) and NaH (60% oil dispersion, 212 mg, 5.30 mmol) in THF (10 ml) under N₂. The solution was stirred at room temperature for 2 h, then partitioned between water and AcOEt. The organic phase was dried over Na₂SO₄ and evaporated *in vacuo.* The crude mixture was purified by silica gel chromatography (DCM/MeOH/NH₄O 95:5:0.2). The collected fractions gave 392 mg of 4-(1-methyl-piperidin-4-ylidenemethyl)-benzonitrile as a yellow oil.
Y= 42%

### STEP B

A solution of 4-(1-methyl-piperidin-4-ylidenemethyl)-benzonitrile (392 mg, 1.85 mmol) in toluene (5 ml) was added dropwise to a stirred solution of MeMgBr (3 M in Et₂O, 1.85 ml, 5.54 mmol) in toluene (10 ml) under N₂ atmosphere.

The resulting mixture was heated to 80°C for 1 h then treated with 10% HCl and stirred at room temperature for 1 h. The phases were separated and the aqueous layer was washed with AcOEt and then brought to basic conditions with NH₄OH. The resulting slurry was extracted with AcOEt and the organic phase was dried over Na₂SO₄ and evaporated *in vacuo* to give 401 mg of 1-[4-(1-methyl-piperidin-4-ylidenemethyl)-phenyl]-ethanone.
Y= 94%

### STEP C

1-[4-(1-methyl-piperidin-4-ylidenemethyl)-phenyl]-ethanone (401 mg, 1.75 mmol) was dissolved in EtOH (20 ml) and 5% Pd/C (40 mg) was added to the resulting solution. The mixture was hydrogenated in a Parr apparatus at 40 psi for 2 h. The catalyst was then filtered and the solvent was removed *in vacuo.* The residue was taken up with DCM and treated with HCl/Et₂O until reaching a pH value of 1. The solvent was removed *in vacuo* to give an oil which was allowed to crystallize. The solid was triturated in Et₂O and filtered to obtain 426 mg of 1-[4-(1-methyl-piperidin-4-ylmethyl)-phenyl]-ethanone as its hydrochloride salt.
Y=91%

### Preparation 3: 1-[4-((3R,5S)-3,4,5-trimethyl-piperazin-1-yl)-phenyl]-ethanone

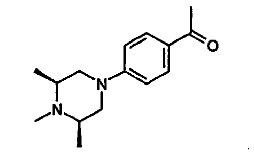

### STEP A

A mixture of 4-fluoro-benzonitrile (1.12 g, 9.25 mmol), (2R,6S)-2,6-dimethylpiperazine (1.58 g, 13.9 mmol) and K₂CO₃ (3.20 g, 23.12 mmol) in DMSO (50 ml) was stirred at 130°C for 24 h. The mixture was then partitioned between water and AcOEt and the organic phase was washed twice with water. The organic layer was then dried over Na₂SO₄ and evaporated *in vacuo.* The residue was taken up with Et₂O, treated with HCl/Et₂O and the resulting precipitate was filtered to give 2.2 g of 4-((3R,5S)-3,5-dimethyl-piperazin-1-yl)-benzonitrile hydrochloride as a yellow powder.
Y= 94%

### STEP B

4-((3R,5S)-3,5-dimethyl-piperazin-1-yl)-benzonitrile hydrochloride (1 g, 3.97 mmol) was dissolved in DCM (25 ml) and TEA (1.1 ml, 7.94 mmol). NaBH₃CN (374 mg, 5.96 mmol) and CH₂O (37% water solution, 0.446 ml, 5.96 mmol) were added to the resulting solution. The slurry was stirred overnight at room temperature, then further CH₂O (0.297 ml, 3.97 mmol) and NaBH₃CN (249 mg, 3.97 mmol) were added. The slurry was stirred at room temperature for 4 h, then brought to basic conditions with 5% NaHCO₃ and extracted twice with DCM. The collected organic phases were dried over Na₂SO₄ and evaporated *in vacuo.* The resulting oil was taken up with DCM and treated with HCl/Et₂O. The precipitate was filtered and washed with di-isopropylether to give 1 g of 4-((3R,5S)-3,4,5-trimethyl-piperazin-1-yl)-benzonitrile hydrochloride.
Y= 95%

### STEP C

A solution of 4-((3R,5S)-3,4,5-trimethyl-piperazin-1-yl)-benzonitrile (506 mg, 2.21 mmol) in toluene (10 ml) was added dropwise to a stirred solution of MeMgBr (3 M in Et₂O 2.2 ml, 6.63 mmol) in toluene (10 ml) under N₂ atmosphere.

The resulting mixture was heated to 80°C for 4 h, then treated with 10% HCl and heated to 80°C for 1 h. The phases were separated and the aqueous layer was washed with AcOEt and then brought to basic conditions with NH₄OH. The resulting slurry was extracted with AcOEt and the organic phase was dried over Na₂SO₄ and evaporated *in vacuo* to give 481 mg of 1-[4-((3R,5S)-3,4,5-trimethyl-piperazin-1-yl)-phenyl]-ethanone as a yellow oil.
Y= 88%

### Preparation 4: 1-[3-Chloro-5-(4-methyl-piperazin-1-yl)-phenyl]-ethanone

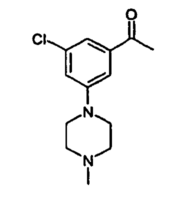

### STEP A

A mixture of 3-chloro-5-fluoro-benzonitrile (1 g, 6.45 mmol), 1-methyl-piperazine (0.715 ml, 6.45 mmol), and K₂CO₃ (2.64 g, 19.3 mmol) in DMF (5 ml) was heated to 140°C for 40 min in a microwave apparatus. The resulting slurry was filtered and the solvent was removed *in vacuo* to give 1 g of 3-chloro-5-(4-methyl-piperazin-1-yl)-benzonitrile. The crude reaction mixture was used in the next step without any further purification.

### STEP B

A solution of 3-chloro-5-(4-methyl-piperazin-1-yl)-benzonitrile (1 g, crude mixture from step A) in toluene (5 ml) was added dropwise to a stirred solution of MeMgBr (3 M in Et₂O, 4.25 ml, 12.7 mmol) in toluene (5 ml) under N₂.

The resulting mixture was heated to 100°C for 6 h, then cooled down to 0°C and treated with 10% HCl. The mixture was refluxed for 1 h and then stirred at room temperature for 12 h. The phases were separated and the aqueous layer was brought to basic conditions with NH₄OH and extracted with DCM. The organic phase was dried over Na₂SO₄ and evaporated *in vacuo.* The crude material was purified by column chromatography (eluent: AcOEt/petroleum ether 9:1) to give 250 mg of 1-[3-chloro-5-(4-methyl-piperazin-1-yl)-phenyl]-ethanone as a yellow oil.

### Preparation 5: Methanesulfonic acid 4-((E)-3-{4-[(E)-2-(tetrahydro-pyran-2-yloxycarbamoyl)-vinyl]-phenyl}-acryloyl)-benzyl ester

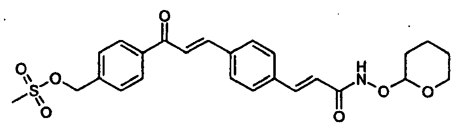

### STEP A

A mixture of 1-(4-hydroxymethyl-phenyl)-ethanone (1 g, 6.67 mmol), 4-formylcinnamic acid (1.17 g, 6.67 mmol) and 1.7 M KOH (5.89 ml) in EtOH (60 ml) was stirred at room temperature overnight. The resulting precipitate was filtered and washed with EtOH to give 1.39 g of (E)-3-{4-[(E)-3-(4-hydroxymethyl-phenyl)-3-oxo-propenyl]-phenyl}-acrylic acid as its potassium salt.
Y=60%

### STEP B

A mixture of (E)-3-{4-[(E)-3-(4-hydroxymethyl-phenyl)-3-oxo-propenyl]-phenyl}-acrylic acid potassium salt (1.39 g, 4.01 mmol), EDC (1.53 g, 8.03 mmol), HOBT (1.08 g, 8.03 mmol), TEA (1.11 ml, 8.03 mmol), NH₂OTH (939 mg, 8.03 mmol) in THF (20 ml) and DMF (20 ml) was stirred at room temperature overnight and then partitioned between water and AcOEt. The organic phase was washed with water, dried over Na₂SO₄ and evaporated *in vacuo.* The resulting solid was triturated with *di*-isopropylether and filtered to give 1.15 g of (E)-3-{4-[(E)-3-(4-hydroxymethylphenyl)-3-oxo-propenyl]-phenyl}-N-(tetrahydro-pyran-2-yloxy)-acrylamide.
Y=70%

### STEP C

Methanesulfonyl chloride (388 mg, 3.39 mmol) was added to a stirred solution of (E)-3-{4-[(E)-3-(4-hydroxymethyl-phenyl)-3-oxo-propenyl]-phenyl}-N-(tetrahydropyran-2-yloxy)-acrylamide (1.15 g, 2.82 mmol) and TEA (1.18 ml, 8.46 mmol) in DCM (20 ml) and DMF (20 ml). The mixture was stirred at room temperature for 1 h and then additional methanesulfonyl chloride (258 mg, 2.25 mmol) and TEA (0.393 ml, 2.82 mmol) were added. After stirring for additional 1 h the solution was diluted with water and brought to basic conditions with 5% NaHCO₃. The resulting slurry was extracted with AcOEt and the organic phase was washed with water, dried over Na₂SO₄ and evaporated *in vacuo.* The crude reaction mixture was purified by column chromatography (eluent: petroleum ether/AcOEt 1:1) to give 277 mg of the title compound and 423 mg of (E)-3-{4-[(E)-3-(4-chloromethyl-phenyl)-3-oxo-propenyl]-phenyl}-N-(tetrahydro-pyran-2-yloxy)-acrylamide.

### Preparation 6: 1-((2R,6S)-2,6-Dimethyl-piperazin-1-yl)-ethanone

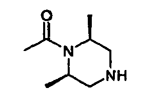

### STEP A

A solution of BOC₂O (1.05 g, 4.8 mmol) in DCM (10 ml) was added dropwise to a stirred solution of (2R,6S)-2,6-dimethyl-piperazine (500 mg, 4.38 mmol) and TEA (1.22 ml, 8.75 mmol) in DCM (20 ml) at 0°C. The mixture was stirred at room temperature for 4 h, the solvent was evaporated and the residue was partitioned between water and Et₂O. The organic phase was dried over Na₂SO₄, evaporated *in vacuo* and the crude reaction mixture was purified by column chromatography (eluent: DCM/MeOH/NH₄OH 97:3:0.1) to give 840 mg of (3S,5R)-3,5-dimethylpiperazine-1-carboxylic acid tert-butyl ester as a yellow oil.
Y=89%

### STEP B

Acetyl chloride (0.216 ml, 3.04 mmol) was added to a stirred solution of (3S,5R)-3,5-dimethyl-piperazine-1-carboxylic acid tert-butyl ester (500 mg, 2.34 mmol) and TEA (0.49 ml, 3.51 mmol) in DCM (20 ml). The mixture was stirred at room temperature overnight, then the solvent was evaporated and the residue was partitioned between Et₂O and 5% citric acid. The organic phase was dried over Na₂SO₄ and evaporated *in vacuo* to give 545 mg of (3S,5R)-4-acetyl-3,5-dimethylpiperazine-1-carboxylic acid tert-butyl ester as a colourless oil.
Y= 90%

### STEP C

(3S,5R)-4-Acetyl-3,5-dimethyl-piperazine-1-carboxylic acid tert-butyl ester (450 mg, 1.75 mmol) was dissolved in DCM (10 ml) and acidified with HCl/Et₂O. The mixture was stirred at room temperature for two days. The resulting solid was filtered to give 190 mg of 1-((2R,6S)-2,6-dimethyl-piperazin-1-yl)-ethanone hydrochloride. The mother liquors were treated with HCl/Et₂O for 4 h and the resulting solid was filtered to give additional 43 mg of the title compound as hydrochloric salt.
Y=69%

### Preparation 7: Methanesulfonic acid 3-((E)-3-{4-[(E)-2-(tetrahydro-pyran-2-yloxycarbamoyl)-vinyl]-phenyl}-acryloyl)-benzyl ester

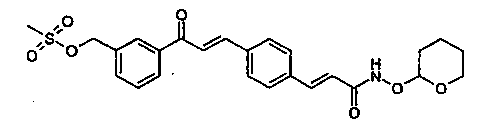

### STEP A

A mixture of 3-acetyl-benzaldehyde (850 mg, 5.74 mmol) and NaBH(OAc)₃ (2.44 g, 11.48 mmol) in toluene (15 ml) was stirred at 80°C for 4 h. The resulting solution was brought to basic conditions with 2 N NaOH and extracted with AcOEt. The organic phase was dried over Na₂SO₄ and evaporated *in vacuo* to give 850 mg of crude mixture of 1-(3-hydroxymethyl-phenyl)-ethanone as a pale yellow oil.

### STEP B

A mixture of 1-(3-hydroxymethyl-phenyl)-ethanone (810 mg), 4-formylcinnamic acid (950 mg, 5.4 mmol) and 1.7 M KOH (6.4 ml) in EtOH (40 ml) was stirred at room temperature for 18 h. The resulting precipitate was filtered and washed with EtOH to give 1.25 g of (E)-3-{4-[(E)-3-(3-hydroxymethyl-phenyl)-3-oxo-propenyl]-phenyl}-acrylic acid as its potassium salt.

### STEP C

A mixture of (E)-3-{4-[(E)-3-(3-hydroxymethyl-phenyl)-3-oxo-propenyl]-phenyl}-acrylic acid potassium salt (1.25 g, 3.6 mmol), EDC (828 mg, 4.32 mmol), HOBT (584 mg, 4.32 mmol), TEA (1.0 ml, 7.2 mmol), NH₂OTHP (421 mg, 3.60 mmol) in THF (20 ml) and DMF (20 ml) was stirred at room temperature for 12 h and then partitioned between water and AcOEt. The organic phase was washed with water, dried over Na₂SO₄ and evaporated *in vacuo.* The crude reaction mixture was purified by column chromatography (eluent: petroleum ether/AcOEt 2:8) to give 1.2 g of (E)-3-{4-[(E)-3-(3-hydroxymethyl-phenyl)-3-oxo-propenyl]-phenyl}-N-(tetrahydro-pyran-2-yloxy)-acrylamide as a yellow powder.
Y=81%

### STEP D

Methanesulfonyl chloride (0.41 ml, 5.3 mmol) was added to a stirred solution of (E)-3-{4-[(E)-3-(3-hydroxymethyl-phenyl)-3-oxo-propenyl]-phenyl}-N-(tetrahydropyran-2-yloxy)-acrylamide (1.08 g, 2.68 mmol) and TEA (1.47 ml, 10.6 mmol) in DCM (18 ml) and DMF (12 ml). The mixture was stirred at room temperature for 30 min and then brought to basic conditions with 5% NaHCO₃. The resulting slurry was extracted with Et₂O and the organic phase was washed with water, dried over Na₂SO₄ and evaporated *in vacuo.* The crude reaction mixture was purified by column chromatography (eluent: petroleum ether/AcOEt 1:1) to give 600 mg of a mixture of the title compound and (E)-3-{4-[(E)-3-(3-chloromethyl-phenyl)-3-oxo-propenyl]-phenyl}-N-(tetrahydro-pyran-2-yloxy)-acrylamide as the main product.

### Preparation 8:1-[3-((3R,5S)-3,4,5-Trimethyl-piperazin-1-yl)-phenyl]-ethanone

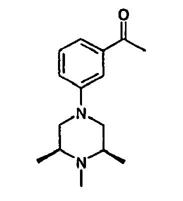

### STEP A

An oven-dried MW tube was charged with Pd₂(dba)₃ (592 mg, 0.65 mmol), K₃PO₄ (1.92 g, 9.06 mmol) and (2'-dicyclohexylphosphanyl-biphenyl-2-yl)-dimethyl-amine (127 mg, 0.32 mmol). The tube was purged and backfilled with N₂ and then 1-(3-chloro-phenyl)-ethanone (1 g, 6.47 mmol), (2R,6S)-2,6-dimethyl-piperazine (886 mg, 7.76 mmol) and DME (10 ml) were added. The mixture was heated in a MW apparatus for 4 h at 100°C and then further Pd₂(dba)₃ (592 mg, 0.65 mmol) and (2'-dicyclohexylphosphanyl-biphenyl-2-yl)-dimethyl-amine (127 mg, 0.32 mmol) were added. The reaction mixture was heated to 100°C for additional 10 h and then the solid was filtered off over a Celite pad. The filtrate was diluted with AcOEt and extracted with 1 M HCl. The aqueous phase was brought to basic conditions with NH₄OH and extracted with AcOEt. The organic phase was dried over Na₂SO₄ and evaporated *in vacuo.* The crude reaction mixture was purified by column chromatography (eluent: AcOEt/MeOH 8:2) to give 226 mg of 1-[3-((3R,5S)-3,5-dimethyl-piperazin-1-yl)-phenyl]-ethanone.
Y= 15%

### STEP B

A mixture of 1-[3-((3R,5S)-3,5-dimethyl-piperazin-1-yl)-phenyl]-ethanone (226 mg, 0.974 mmol), NaBH(OAc)₃ (309 mg, 1.46 mmol) and CH₂O (37% water solution, 0.087 ml, 1.17 mmol) in DCM (10 ml) was stirred at room temperature overnight. The resulting solution was diluted with water, brought to basic conditions with NH₄OH and extracted with DCM. The organic phase was dried over Na₂SO₄ and evaporated *in vacuo.* The crude reaction mixture was purified by SCX cartridge (eluent: MeOH and then 3% NH₄OH in MeOH) to give 188 mg of 1-[3-((3R,5S)-3,4,5-trimethyl-piperazin-1-yl)-phenyl]-ethanone.
Y= 78%

### Preparation 9: 4-(4-Acetyl-phenyl)-piperazine-1-carboxylic acid tert-butyl ester

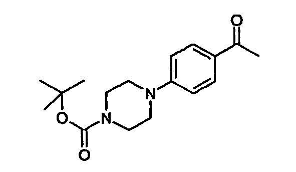

A mixture of 1-(4-piperazin-1-yl-phenyl)-ethanone (513 mg, 2.51 mmol), BOC₂O (820 mg, 3.76 mmol) and TEA (0.698 ml, 5.02 mmol) in DCM (20 ml) was stirred at room temperature overnight. The resulting solution was concentrated *in vacuo* and then partitioned between water and AcOEt. The organic phase was dried over Na₂SO₄, evaporated *in vacuo* and the crude reaction mixture was purified by column chromatography (eluent: petroleum ether/AcOEt) to give 701 mg of 4-(4-acetyl-phenyl)-piperazine-1-carboxylic acid tert-butyl ester.
Y= 92%

### Preparation 10: 1-[5-Chloro-2-(4-methyl-piperazin-1-yl)-phenyl]-ethanone

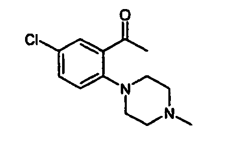

### STEP A

A mixture of 5-chloro-2-fluoro-berizonitrile (500 mg, 3.21 mmol), K₂CO₃ (1.32 g, 9.64 mmol) and 1-methyl-piperazine (482 mg, 4.81 mmol) in DMSO (6.4 ml) was stirred at 100°C for 6 h and then partitioned between water and Et₂O. The organic phase was dried over Na₂SO₄ and evaporated *in vacuo.* The crude mixture was purified by column chromatography (eluent: DCM/MeOH/NH₄OH 98:2:0.1) to give 660 mg of 5-chloro-2-(4-methyl-piperazin-1-yl)-benzonitrile.
Y= 87%

### STEP B

A solution of 5-chloro-2-(4-methyl-piperazin-1-yl)-benzonitrile (660 mg, 2.80 mmol) in dry toluene (6 ml) was added dropwise to a stirred solution of MeMgBr (3 M in Et₂O, 2.8 ml, 8.42 mmol) under N₂ atmosphere.

The resulting mixture was heated to 80°C for 4 h, then treated with 10% HCl until reaching a pH value of 1 and extracted with AcOEt. The phases were separated and the aqueous layer was brought to basic conditions with Na₂CO₃ and extracted with AcOEt. The organic phase was dried over Na₂SO₄ and evaporated *in vacuo* to give 570 mg of 1-[5-chloro-2-(4-methyl-piperazin-1-yl)-phenyl]-ethanone as a yellow powder.
Y= 81%

### Preparation 11: 1-[2-(4-Methyl-piperazin-1-ylmethyl)-phenyl]-ethanone

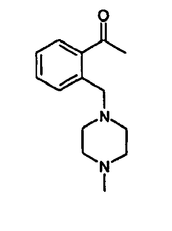

A mixture of 2-acetyl-benzaldehyde (1 g, 6.75 mmol), N-methyl piperazine (878 mg, 8.76 mmol) and NaBH(OAc)₃ (2.14 g, 10.12 mmol) in DCM (50 ml) was stirred at room temperature for 1 h and then acetic acid (526 mg, 8.76 mmol) was added. The resulting solution was stirred at room temperature overnight and then diluted with DCM and washed with 1 M Na₂CO₃. The organic phase was dried over Na₂SO₄ and evaporated *in vacuo.* The crude mixture was purified by column chromatography (eluent: DCM/MeOH/NH₄OH 95:5:0.2) to give 1.06 g of 1-[2-(4-methyl-piperazin-1-ylmethyl)-phenyl]-ethanone.
Y= 67%

### Preparation 12: 4-(4-Acetyl-benzyl)-piperazine-1-carboxylic acid tert-butyl ester

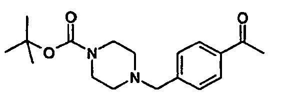

### STEP A

Methanesulfonyl chloride (0.335 ml, 5.05 mmol) was added dropwise to a stirred solution of 1-(4-hydroxymethyl-phenyl)-ethanone (500 mg, 3.37 mmol) and TEA (0.928 ml, 6.74 mmol) in DCM (25 ml). The resulting mixture was stirred at room temperature for 3 h and then the solvent was removed *in vacuo.* The residue was partitioned between water and Et₂O and the organic phase was dried over Na₂SO₄ and evaporated *in vacuo* to give 580 mg of a 1:1 mixture of 1-(4-methanesulfonylmethyl-phenyl)-ethanone and 1-(4-chloromethyl-phenyl)-ethanone, which was used in the next step without further purification.

### STEP B

N-Boc piperazine (485 mg, 2.61 mmol) was added to a stirred solution of a mixture of 1-(4-methanesulfonylmethyl-phenyl)-ethanone, 1-(4-chloromethyl-phenyl)-ethanone (obtained in STEP A, 580 mg) and TEA (0.522 ml, 3.75 mmol) in CH₃CN (5 ml). The resulting mixture was stirred at room temperature overnight and then further TEA (0.250 ml, 1.80 mmol) and N-Boc piperazine (100 mg, 0.53 mmol) were added. After 4 h the solution was partitioned between water and AcOEt, the organic phase was dried over Na₂SO₄ and evaporated *in vacuo.* The crude mixture was purified by column chromatography (eluent: petroleum ether/AcOEt 7:3 to 6:4) to give 700 mg of 4-(4-acetyl-benzyl)-piperazine-1-carboxylic acid tert-butyl ester.

### Preparation 13: 1-[2-Chloro-5-(4-methyl-piperazin-1-yl)-phenyl]-ethanone

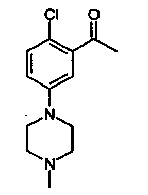

### STEP A

A mixture of 2-chloro-5-fluoro-benzonitrile (1 g, 6.43 mmol), K₂CO₃ (2.66 g, 19.3 mmol) and 1-methyl-piperazine (1.02 g, 10.26 mmol) in DMSO (14 ml) was stirred at 100°C overnight and then further 1-methyl-piperazine (1.02 g, 10.26 mmol) was added. The mixture was stirred at 100°C overnight and then partitioned between water and Et₂O. The organic phase was extracted with 1 M HCl and the aqueous layer was brought to basic conditions with NH₄OH and extracted with DCM. The organic phase was dried over Na₂SO₄ and evaporated *in vacuo* to give 1 g of 2-chloro-5-(4-methyl-piperazin-1-yl)-benzonitrile. Y= 66%

### STEP B

A solution of 2-chloro-5-(4-methyl-piperazin-1-yl)-benzonitrile (1 g, 4.24 mmol) in dry toluene (12 ml) was added dropwise to a stirred solution of MeMgBr (3 M in Et₂O, 4.24 ml, 12.73 mmol) in dry toluene (8 ml) under N₂ atmosphere.

The mixture was heated to 80°C for 4 h, and then acidified with 10% HCl. The resulting mixture were stirred for 2 h at room temperature and then separated. The aqueous phase was brought to basic conditions with NH₄OH and extracted with AcOEt. The organic phase was dried over Na₂SO₄ and evaporated *in vacuo* to give 1.02 g of 1-[2-chloro-5-(4-methyl-piperazin-1-yl)-phenyl]-ethanone.
Y= 94%

### Preparation 14: 1-[3-(4-Methyl-piperazin-1-ylmethyl)-phenyl]-ethanone

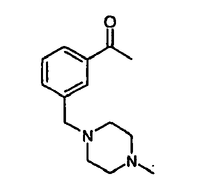

### STEP A

A mixture of 3-formyl-benzonitrile (1.5 g, 11.45 mmol), N-methyl piperazine (1.49 g, 14.9 mmol) and NaBH(OAc)₃ (3.63 g, 17.18 mmol) in DCM (75 ml) and CH₃COOH (0.851 ml, 14.9 mmol) was stirred overnight at room temperature, then diluted with DCM and washed with 1 M Na₂CO₃. The organic phase was dried over Na₂SO₄ and evaporated *in vacuo.* The crude mixture was purified by column chromatography (eluent: DCM/MeOH/NH_{4O}H 97:3:0.5) to give 1.7 g of 3-(4-methyl-piperazin-1-ylmethyl)-benzonitrile.
Y= 70%

### STEP B

A solution of 3-(4-methyl-piperazin-1-ylmethyl)-benzonitrile (1.7 g, 7.91 mmol) in dry toluene (20 ml) was added dropwise to a stirred solution of MeMgBr (3 M in Et₂O, 7.91 ml, 23.72 mmol) under N₂ atmosphere at 0°C.

The mixture was heated to 80 °C for 6 h and then kept at room temperature overnight. The resulting slurry was treated with 10% HCl and ice for 1 h and than brought to basic conditions with 1 M NaOH and extracted with AcOEt. The organic phase was dried over Na₂SO₄ and evaporated *in vacuo.* The crude mixture was purified by column chromatography (eluent: DCM/MeOH/NH₄OH from 97:3:0.1 to 95:5:0.2) to give 1.69 g of 1-(3-(4-methyl-piperazin-1-ylmethyl)-phenyl]-ethanone.
Y=92%

### Example 16: (E)-3-(4-{(E)-3-[4-(4-Benzoyl-piperazin-1-yl)-phenyl]-3-oxo-propenyl}-phenyl)-N-hydroxy-acrylamide

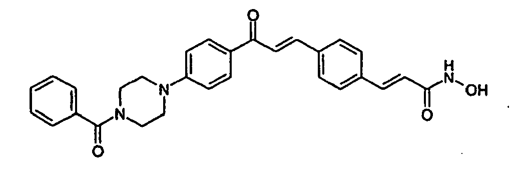

### STEP A

Benzoyl chloride (0.341 ml, 2.94 mmol) was added dropwise to a stirred mixture of 1-(4-piperazin-1-yl-phenyl)-ethanone (500 mg, 2.45 mmol) and TEA (0.681 ml, 4.9 mmol) in DCM (25 ml). The resulting solution was stirred overnight at room temperature, then diluted with DCM and washed with water, with NaHCO₃ (5% in H₂O) and finally with citric acid (20% in H₂O). The organic phase was dried over Na₂SO₄ and evaporated *in vacuo.* The resulting solid was triturated with di-isopropylether and filtered to give 687 mg of 1-[4-(4-benzoyl-piperazin-1-yl)-phenyl]-ethanone.
Y= 91%

### STEP B

1-[4-(4-Benzoyl-piperazin-1-yl)-phenyl]-ethanone (500 mg, 1.62 mmol) was dissolved in 1,4-dioxane (3 ml) and added to a stirred solution of 4-formylcinnamic acid (286 mg, 1.62 mmol) and 1.7 M KOH (1.9 ml) in EtOH (5 ml) and water (5 ml). The resulting mixture was stirred overnight at room temperature and then heated to 40°C for 4 h. The reaction was then quenched with 10% HCl, the resulting precipitate was filtered and washed with EtOH. The resulting green solid was dried *in vacuo* to give 240 mg of (E)-3-(4-{(E)-3-[4-(4-benzoyl-piperazin-1-yl)-phenyl]-3-oxo-propenyl}-phenyl)-acrylic acid as its hydrochloride salt.
Y= 30%

### STEP C

A mixture of (E)-3-(4-{(E)-3-[4-(4-benzoyl-piperazin-1-yl)-phenyl]-3-oxo-propenyl}-phenyl)-acrylic acid hydrochloride (200 mg, 0.398 mmol), HOBT (107.4 mg, 0.796 mmol), EDC (152 mg, 0.796 mmol), TEA (0.111 ml, 0.796 mmol) and NH₂OTHP (56 mg, 0.477 mmol) in THF (5 ml) and DMF (5 ml) was stirred overnight at room temperature and then partitioned between water and AcOEt. The organic phase was dried over Na₂SO₄ and evaporated *in vacuo.* The crude mixture was purified by silica gel chromatography (eluent: DCM/MeOH 98:2) and the resulting product was dissolved in DCM and treated with HCl/Et₂O for 2 h. The precipitate was filtered giving 115 mg of (E)-3-(4-{(E)-3-[4-(4-benzoyl-piperazin-1-yl)-phenyl]-3-oxo-propenyl}-phenyl)-N-hydroxy acrylamide as its hydrochloride salt.
Yield = 56%
LC-MS: Method C, rt=1.94; (ES+) MH⁺: 482.1
'1H NMR (DMSO-d₆, +TFA) δ (ppm): 8.08 (d, 2 H), 7.95 (d, 1 H), 7.90 (d, 2 H), 7.66 (d, 1 H), 7.63 (d, 2 H), 7.33 - 7.57 (m, 6 H), 7.04 (d, 2 H), 6.59 (d, 1 H), 3.41 - 3.72 (m, 8 H).

### Example 17: (E)-3-(4-{(E)-3-[4-(4-Acetyl-piperazin-1-yl)-phenyl]-3-oxo-propenyl}-phenyl)-N-hydroxy-acrylamide

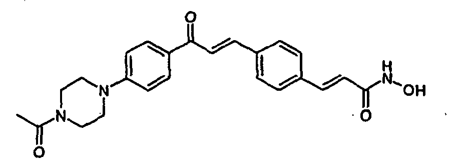

### STEP A

A mixture of 1-[4-(4-acetyl-piperazin-1-yl)-phenyl]-ethanone (prepared following the procedure described in Example 16 STEP A, 533 mg, 2.16 mmol), 4-formylcinnamic acid (381 mg, 2.16 mmol) and 1.7 M KOH (2.54 ml) in EtOH (15 ml) and water (3 ml) was stirred overnight at room temperature. The resulting precipitate was filtered to get 260 mg of (E)-3-(4-{(E)-3-[4-(4-acetyl-piperazin-1-yl)-phenyl]-3-oxo-propenyl}-phenyl)-acrylic acid as its potassium salt. The mother liquors were acidified with 10% HCl and the resulting green precipitate was filtered to give 240 mg of (E)-3-(4-{(E)-3-[4-(4-acetyl-piperazin-1-yl)-phenyl]-3-oxo-propenyl}-phenyl)-acrylic acid hydrochloride. The mixture of both, the potassium salt and of the hydrochloric salt, was used in the next step without any further purification.

### STEP B

(E)-3-(4-{(E)-3-[4-(4-Acetyl-piperazin-1-yl)-phenyl]-3-oxo-propenyl}-phenyl)-acrylic acid (mixture of potassium salt and hydrochloric salt, 200 mg) was dissolved in THF (5 ml), DMF (5 ml) and TEA (0.126 ml, 0.908 mmol), then HOBT (122 mg, 0.908 mmol), EDC (173 mg, 0.908 mmol) and NH₂OTHP (63.7 mg, 0.545 mmol) were added to the resulting solution. The mixture was stirred overnight at room temperature and then partitioned between water and AcOEt. The organic phase was dried over Na₂SO₄ and evaporated *in vacuo.* The crude mixture was purified by silica gel chromatography (eluent: DCM/MeOH/NH₄OH 96:4:0.2). The resulting product was dissolved in DCM and treated with HCl/Et₂O for 1 h. The precipitate was filtered and crystallized from H₂O/CH₃CN/DMSO to give 20 mg of (E)-3-(4-{(E)-3-[4-(4-acetyl-piperazin-1-yl)-phenyl]-3-oxo-propenyl}-phenyl)-N-hydroxy-acrylamide hydrochloride.
LC-MS: Method E, rt=1.54; (ES+) MH⁺: 420.2
'1H NMR (DMSO-d₆) δ (ppm): 10.77 (bs, 1 H), 8.08 (m, 2 H), 7.95 (d, 1 H), 7.90 (d, 2 H), 7.66 (d, 1 H), 7.60 - 7.66 (m, 2 H), 7.49 (d, 1 H), 7.03 (m, 2 H), 6.55 (d, 1 H), 3.56 - 3.67 (m, 4 H), 3.43 - 3.46 (m, 2 H), 3.36 - 3.41 (m, 2 H), 2.05 (s, 3 H).

### Example 18: (E)-N-Hydroxy-3-(4-{(E)-3-[4-(4-methanesulfonyl-piperazin-1-yl)-phenyl]-3-oxo-propenyl}-phenyl)-acrylamide

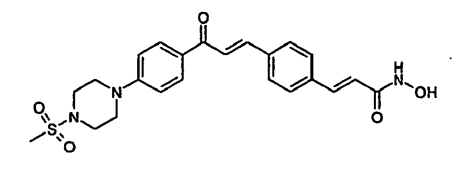

### STEP A

1-[4-(4-Methanesulfonyl-piperazin-1-yl)-phenyl]-ethanone (obtained following the experimental procedure described in Example 16 STEP A, 500 mg, 1.77 mmol) was dissolved in 1,4-dioxane (3 ml) and added to a stirred solution of 4-formylcinnamic acid (312 mg, 1.77 mmol) and 1.7 M KOH (2 ml) in EtOH (5 ml) and water (5 ml). The resulting mixture was stirred overnight at room temperature and then heated to 40°C for 6 h. The resulting precipitate was filtered to give 470 mg of (E)-3-(4-{(E)-3-[4-(4-methanesulfonyl-piperazin-1-yl)-phenyl]-3-oxo-propenyl}-phenyl)-acrylic acid as its potassium salt.
Y= 55%

### STEP B

(E)-3-(4-{(E)-3-[4-(4-Methanesulfonyl-piperazin-1-yl)-phenyl]-3-oxo-propenyl}-phenyl)-acrylic acid potassium salt (190 mg, 0.398 mmol) was dissolved in THF (5 ml), DMF (5 ml) and TEA (0.111 ml, 0.796 mmol). HOBT (107.4 mg, 0.796 mmol), EDC (152 mg, 0.796 mmol) and NH₂OTH (56 mg, 0.477 mmol) were added to the resulting solution. The mixture was stirred overnight at room temperature and then partitioned between water and AcOEt. The organic phase was dried over Na₂SO₄ and evaporated *in vacuo.* The crude mixture was purified by silica gel chromatography (eluent: DCM/MeOH 98:2). The resulting product was dissolved in DCM and treated with HCl/Et₂O for 2 h. The precipitate was filtered and washed with DCM to give 20 mg of (E)-N-hydroxy-3-(4-{(E)-3-[4-(4-methanesulfonyl-piperazin-1-yl)-phenyl]-3-oxo-propenyl}-phenyl)-acrylamide hydrochloride.
Yield = 11%
LC-MS: Method C, rt=1.75; (ES+) MH⁺: 456.0
¹H NMR (DMSO-d₆) δ (ppm): 8.09 (m, 2 H), 7.95 (d, 1 H), 7.90 (d, 2 H), 7.67 (d, 1 H), 7.63 (d, 2 H), 7.49 (d, 1 H), 7.08 (m, 2 H), 6.56 (d, 1 H), 3.48 - 3.55 (m, 4 H), 3.20 - 3.31 (m, 4 H), 2.92 (s, 3 H).

### Example 19: 4-(4-{(E)-3-[4-((E)-2-Hydroxycarbamoyl-vinyl)-phenyl]-acryloyl}-phenyl)-piperazine-1-carboxylic acid dimethylamide

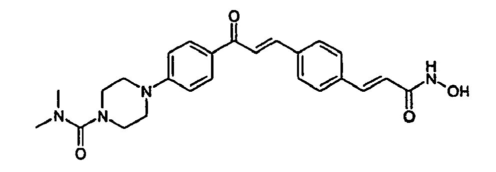

The product was obtained starting from 4-(4-acetyl-phenyl)-piperazine-1-carboxylic acid dimethylamide (obtained following the procedure in Example 16 STEP A) and 4-formylcinnamic acid, following the experimental procedure described in Example 17 STEP A and B. The title compound was obtained as its hydrochloride salt.
LC-MS: Method C, rt=2.62; (ES+) MH⁺: 449.1
¹H NMR (DMSO-d₆) δ (ppm): 8.07 (m, 2 H), 7.95 (d, 1 H), 7.90 (d, 2 H), 7.66 (d, 1 H), 7.63 (d, 2 H), 7.48 (d, 1 H), 7.03 (m, 2 H), 6.57 (d, 1 H), 3.37 - 3.47 (m, 4 H), 3.20 - 3.33 (m, 4 H), 2.79 (s, 6 H).

### Example 20: 4-(4-{(E)-3-[4-((E)-2-Hydroxycarbamoyl-vinyl)-phenyl]-acryloyl}-phenyl)-piperazine-1-carboxylic acid amide

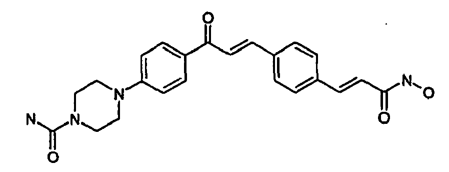

### STEP A

1-(4-Piperazin-1-yl-phenyl)-ethanone (500 mg, 2.45 mmol) was dissolved in DCM (20 ml) and NaOCN (318.6 mg, 4.90 mmol) and AcOH (0.28 ml, 4.90 mmol) were added to the resulting solution. The mixture was stirred at room temperature for 36 h and the resulting precipitate was filtered off, washed with DCM and water to give 675 mg of 4-(4-acetyl-phenyl)-piperazine-1-carboxylic acid amide (crude compound was used without further purification in the next step).

### STEP B

A mixture of 4-(4-acetyl-phenyl)-piperazine-1-carboxylic acid amide (crude mixture from STEP A, 300 mg), 4-formylcinnamic acid (214 mg, 1.21 mmol) and 1.7 M KOH (1.42 ml) in MeOH (10 ml) was stirred at room temperature overnight and then heated to 50°C for 8 h. After stirring at room temperature for additional 72 h the mixture was heated to 50°C for 4 h then quenched with 10% HCl. The resulting precipitate was filtered off and washed with MeOH to give 316 mg of (E)-3-(4-{(E)-3-[4-(4-carbamoyl-piperazin-1-yl)-phenyl]-3-oxo-propenyl}-phenyl)-acrylic acid hydrochloride.

### STEP C

(E)-3-(4-{(E)-3-[4-(4-Carbamoyl-piperazin-1-yl)-phenyl]-3-oxo-propenyl}-phenyl)-acrylic acid hydrochloride (316 mg, 0.716 mmol) was suspended in DMF (5 ml). HOBT (193 mg, 1.43 mmol), EDC (273 mg, 1.43 mmol), TEA (0.200 ml, 1.43 mmol) and NH₂OTHP (100 mg, 0.86 mmol) were added. The mixture was stirred at room temperature for 72 h and then partitioned between water and hot AcOEt. The organic extract was dried over Na₂SO₄, evaporated *in vacuo* and the crude product was crystallized from DCM/Et₂O. The resulting solid was suspended in DCM and treated with HCl/Et₂O for 1 h. The precipitate was filtered off, giving 103 mg of 4-(4-{(E)-3-[4-((E)-2-hydroxycarbamoyl-vinyl)-phenyl]-acryloyl}-phenyl)-piperazine-1-carboxylic acid amide as its hydrochloride salt.
Yield = 31 %
LC-MS: Method C, rt=1.44; (ES+) MH⁺: 421.06
¹H NMR (DMSO-d₆ + TFA) δ (ppm): 8.06 (d, 2 H), 7.94 (d, 1 H), 7.89 (d, 2 H), 7.65 (d, 1 H), 7.63 (d, 2 H), 7.48 (d, 1 H), 7.04 (d, 2 H), 6.57 (d, 1 H), 3.43 - 3.51 (m, 4 H), 3.32 - 3.42 (m, 4 H).

### Example 21: 4-(4-{(E)-3-[4-((E)-2-Hydroxycarbamoyl-vinyl)-phenyl]-acryloyl}-phenyl)-piperazine-1-carboxylic acid ethyl ester

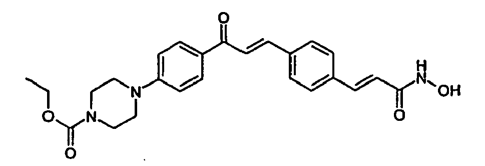

The product was obtained starting from 4-(4-acetyl-phenyl)-piperazine-1-carboxylic acid ethyl ester (obtained following the experimental procedure described in Example 16 STEP A) and 4-formylcinnamic acid, following the experimental procedure described in Example 16 STEP B and C. The title compound was obtained as its hydrochloride salt.
LC-MS: Method C, rt=1.99; (ES+) MH⁺: 450.1
¹H NMR (DMSO-d₆) δ (ppm): 8.07 (d, 2 H), 7.95 (d, 1 H), 7.90 (d, 2 H), 7.66 (d, 1 H), 7.63 (d, 2 H), 7.49 (d, 1 H), 7.03 (d, 2 H), 6.56 (d, 1 H), 4.08 (q, 2 H), 3.48 - 3.56 (m, 4 H), 3.36 - 3.46 (m, 4 H), 1.21 (t, 3 H).

### Example 22: (E)-N-Hydroxy-3-(4-{(E)-3-[4-(1-methyl-piperidin-4-yloxy)-phenyl]-3-oxo-propenyl}-phenyl)-acrylamide

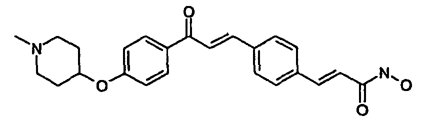

The product was obtained starting from 1-[4-(1-methyl-piperidin-4-yloxy)-phenyl]-ethanone (prepared as described in Preparation 1) and 4-formylcinnamic acid, following the experimental procedure described for Example 1 (STEP C and D). The title compound was purified by preparative LC-MS and was obtained as its trifluoroacetate salt.
LC-MS: Method C, rt=1.74; (ES+) MH⁺: 407.4
¹H NMR (DMSO-d₆) δ (ppm): 10.47 (bs, 1 H), 9.30 (bs, 1 H), 8.14 (m, 2 H), 7.80 - 7.91 (m, 3 H), 7.69 (d, 1 H), 7.64 (d, 2 H), 7.50 (d, 1 H), 7.16 (m, 2 H), 6.62 (d, 1 H), 4.68 - 4.98 (m, 1 H), 2.96 - 3.57 (m, 6 H), 2.86 (s, 3 H), 1.97 - 2.26 (m, 2 H).

### Example 23: (E)-N-Hydroxy-3-(4-{(E)-3-[4-(1-methyl-piperidin-4-ylmethyl)-phenyl]-3-oxo-propenyl}-phenyl)-acrylamide

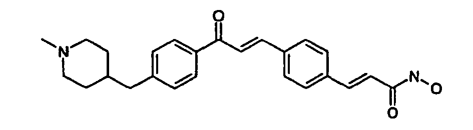

### STEP A

A mixture of 1-[4-(1-rriethyl-piperidin-4-ylmethyl)-phenyl]-ethanone hydrochloride (prepared as described in Preparation 2, 426 mg, 1.59 mmol), 4-formylcinnamic acid (280 mg, 1.59 mmol) and 1.7 M KOH (2.8 ml) in EtOH (7 ml) was stirred at room temperature overnight. The mixture was acidified with 10% HCl and the resulting precipitate was filtered off to give 388 mg of (E)-3-(4-{(E)-3-[4-(1-methyl-piperidin-4-ylmethyl)-phenyl]-3-oxo-propenyl}-phenyl)-acrylic acid hydrochloride.
Y= 57%

### STEP B

(E)-3-(4-{(E)-3-[4-(1-methyl-piperidin-4-ylmethyl)-phenyl]-3-oxo-propenyl}-phenyl)-acrylic acid hydrochloride (160 mg, 0.376) was dissolved in THF (5 ml), and then HOBT (66.5 mg, 0.493 mmol), EDC (94.6 mg, 0.493 mmol), TEA (0.171 ml, 1.233 mmol) and NH₂OTHP (47.6 mg, 0.411 mmol) were added to the resulting solution. The mixture was stirred overnight at room temperature and then partitioned between water and AcOEt. The organic extracts were dried over Na₂SO₄ and evaporated *in vacuo.* The crude mixture was dissolved in DCM and treated with HCl/Et₂O for 2 h. The precipitate was filtered off to give 90 mg (E)-N-hydroxy-3-(4-{(E)-3-[4-(1-methyl-piperidin-4-ylmethyl)-phenyl]-3-oxo-propenyl}-phenyl)-acrylamide hydrochloride.
Yield = 54%
LC-MS: Method C, rt=1.45; (ES+) MH⁺: 405.2
¹H NMR (DMSO-d₆ + TFA) δ (ppm): 9.72 (bs, 1 H), 8.11 (d, 2 H), 7.96 (d, 1 H), 7.92 (d, 2 H), 7.73 (d, 1 H), 7.65 (d, 2 H), 7.49 (d, 1 H), 7.40 (d, 2 H), 6.57 (d, 1 H), 3.29 - 3.44 (m, 2 H), 2.72 - 2.94 (m, 2 H), 2.70 (d, 3 H), 2.65 (d, 2 H), 1.66 - 1.95 (m, 3 H), 1.33 - 1.64 (m, 2 H).

### Example 24: (E)-N-Hydroxy-3-(4-{(E)-3-oxo-3-[4-((3R,5S)-3,4,5-trimethyl-piperazin-1-yl)-phenyl]-propenyl}-phenyl)-acrylamide

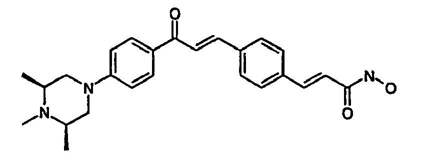

The product was obtained starting from 1-[4-((3R,5S)-3,4,5-trimethyl-piperazin-1-yl)-phenyl]-ethanone (prepared as described in Preparation 3) and 4-formylcinnamic acid, following the experimental procedure described for Example 1 (STEP C and D). The title compound was obtained as its hydrochloride salt. LC-MS: Method D, rt=3.28; (ES+) MH⁺: 420.2
¹H NMR (DMSO-d₆) δ (ppm): 10.95 (bs, 1 H), 8.10 (d, 2 H), 7.96 (d, 1 H), 7.91 (d, 2 H), 7.68 (d, 1 H), 7.64 (d, 2 H), 7.49 (d, 1 H), 7.15 (d, 2 H), 6.58 (d, 1 H), 4.18 (d, 2 H), 3.26 - 3.49 (m, 2 H), 3.10 - 3.25 (m, 2 H), 2.80 (d, 3 H), 1.44 (d, 6 H).

### Example 25: (E)-3-(4-{(E)-3-[3-Chloro-5-(4-methyl-piperazin-1-yl)-phenyl]-3-oxo-propenyl}-phenyl)-N-hydroxy-acrylamide

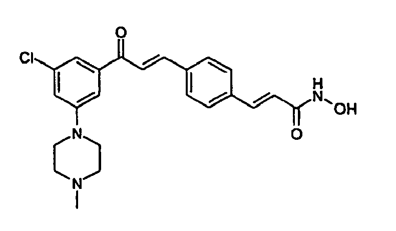

### STEP A

A mixture of 1-[3-chloro-5-(4-methyl-piperazin-1-yl)-phenyl]-ethanone (prepared as described in Preparation 4, 300 mg, 1.19 mmol), KOH (133 mg, 2.38 mmol) and (E)-3-(4-formyl-phenyl)-acrylic acid *tert*-butyl ester (276 mg, 1.19 mmol) in EtOH (10 ml) and H₂O (2 ml) was stirred at -20°C for 1 h and then at room temperature for 12 h. The resulting mixture was partitioned between water and AcOEt and the organic phase was dried over Na₂SO₄ and evaporated *in vacuo.* The crude reaction mixture was purified by column chromatograpy (eluent: AcOEt/MeOH 9:1). The collected fractions were evaporated *in vacuo* and the resulting powder was dissolved in DCM (5 ml) and TFA (1 ml). The solution was stirred at room temperature for 12 h and then the solvent was removed to give 40 mg (E)-3-(4-{(E)-3-[3-chloro-5-(4-methyl-piperazin-1-yl)-phenyl]-3-oxo-propenyl}-phenyl)-acrylic acid as its trifluoroacetate salt.
Y= 6%

### STEP B

(E)-3-(4-{(E)-3-[3-Chloro-5-(4-methyl-piperazin-1-yl)-phenyl]-3-oxo-propenyl}-phenyl)-acrylic acid trifluoroacetate (40 mg, 0.076 mmol) was suspended in THF (5 ml). HOBT (16 mg, 0.116 mmol), EDC (22.3 mg, 0.116 mmol), TEA (0.04 ml, 0.291 mmol) and NH₂OTHP (12 mg, 0.097 mmol) were added to the resulting mixture. The reaction was stirred at room temperature overnight and then partitioned between water and AcOEt. The organic phase was dried over Na₂SO₄, evaporated *in vacuo* and the crude product was purified by column chromatography (eluent: AcOEt/MeOH 9:1). The collected fractions were evaporated and the resulting product was dissolved in DCM and treated with HCl/Et₂O for 1 h. The precipitate was filtered and purified by preparative LC-MS to give 20 mg of (E)-3-(4-{(E)-3-[3-Chloro-5-(4-methyl-piperazin-1-yl)-phenyl]-3-oxo-propenyl}-phenyl)-N-hydroxy-acrylamide as its trifluoroacetate salt.
Y= 48%
LC-MS: Method C, rt=1.57; (ES+) MH⁺: 426.31
¹H NMR (DMSO-d₆) δ (ppm): 10.79 (bs, 1 H), 9.69 (bs, 1 H), 7.96 (m, 2 H), 7.94 (d, 1 H), 7.77 (d, 1 H), 7.62 - 7.71 (m, 3 H), 7.56 - 7.60 (m, 1 H), 7.51 (d, 1 H), 7.38 (d, 1 H), 6.56 (d, 1 H), 3.98 - 4.13 (m, 2 H), 3.43 - 3.62 (m, 2 H), 2.94 - 3.25 (m, 4 H), 2.87 (s, 3 H).

### Example 26: (E)-N-Hydroxy-3-{4-[(E)-3-oxo-3-(4-piperazin-1-ylmethyl-phenyl)-propenyl]-phenyl}-acrylamide

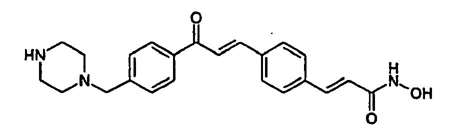

N-Boc-piperazine (46 mg, 0.246 mmol) was added to a stirred mixture of methanesulfonic acid 4-((E)-3-{4-[(E)-2-(tetrahydro-pyran-2-yloxycarbamoyl)-vinyl]-phenyl}-acryloyl)-benzyl ester (prepared as described in Preparation 5, 100 mg, 0.206 mmol) and TEA (0.057 ml, 0.412 mmol) in DMF (1 ml) and DCM (1 ml). The resulting solution was stirred at room temperature for 1 h. The mixture was diluted with water and extracted with AcOEt. The organic phase was washed with water, dried over Na₂SO₄ and evaporated *in vacuo.* The crude mixture was purified by column chromatography (eluent: DCM/MeOH/NH₄OH 99:1:0.2). The resulting product was dissolved in DCM and treated with HCl/Et₂O for 4 h. The precipitate was filtered off and rinsed with DCM to give 42.6 mg of (E)-N-hydroxy-3-{4-[(E)-3-oxo-3-(4-piperazin-1-ylmethyl-phenyl)-propenyl]-phenyl}-acrylamide as its bis-hydrochloride salt.
Y= 44%
LC-MS: Method C, rt=1.16; (ES+) MH⁺: 392.19
¹H NMR (DMSO-d₆ + TFA) δ (ppm): 9.52 (bs, 3 H), 8.24 (d, 2 H), 8.00 (d, 1 H), 7.94 (m, 2 H), 7.85 (m, 2 H), 7.77 (d, 1 H), 7.66 (d, 2 H), 7.49 (d, 1 H), 6.58 (d, 1 H), 4.48 (s, 2 H), 3.23 - 3.56 (m, 8 H).

### Example 27: (E)-3-(4-{(E)-3-[4-(4-Benzyl-piperazin-1-ylmethyl)-phenyl]-3-oxo-propenyl}-phenyl)-N-hydroxy-acrylamide

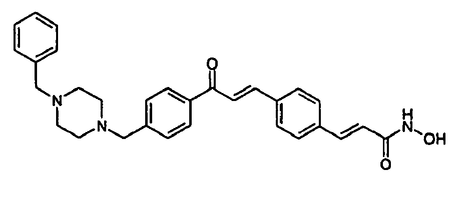

1-Benzyl-piperazine (40 mg, 0.22 mmol) was added to a stirred mixture of methanesulfonic acid 4-((E)-3-(4-[(E)-2-(tetrahydro-pyran-2-ylooycarbamoyl)-vinyl]-phenyl)-acryloyl)-benzyl ester (prepared as described in Preparation 5, 90 mg, 0.185 mmol) and TEA (0.052 ml, 0.37 mmol) in DMF (2 ml). The resulting solution was stirred at room temperature for 1 h. The mixture was diluted with water and extracted with hot AcOEt. The organic phase was washed with water, dried over Na₂SO₄ and evaporated *in vacuo.* The crude reaction mixture was triturated with di-isopropylether/DCM, filtered and the resulting powder was suspended in DCM and treated with HCl/Et₂O for 1 h. The solid was filtered and washed with DCM to give 28.1 mg of (E)-3-(4-{(E)-3-[4-(4-benzyl-piperazin-1-ylmethyl)-phenyl]-3-oxo-propenyl}-phenyl)-N-hydroxy-acrylamide as its bis-hydrochloride salt.
Y=27%
LC-MS: Method C, rt=1.41; (ES+) MH⁺: 482.19
¹H NMR (DMSO-d₆ + TFA 353 K) δ (ppm): 8.13 (m, 2 H), 7.86 (m, 2 H), 7.83 (d, 1 H), 7.68 - 7.76 (m, 3 H), 7.63 (m, 2 H), 7.54 - 7.60 (m, 2 H), 7.49 (d, 1 H), 7.40 - 7.46 (m, 3 H), 6.64 (d, 1 H), 4.24 (s, 2 H), 4.18 (s, 2 H), 3.08 - 3.40 (m, 8 H).

### Example 28: (E)-3-(4-{(E)-3-[4-((2R,6S)-2,6-Dimethyl-morpholin-4-ylmethyl)-phenyl]-3-oxo-propenyl}-phenyl)-N-hydroxy-acrylamide

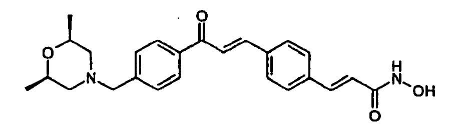

(2S,6R)-2,6-Dimethyl-morpholine (32 mg, 0.277 mmol) was added to a stirred mixture of methanesulfonic acid 4-((E)-3-{4-[(E)-2-(tetrahydro-pyran-2-yloxycarbamoyl)-vinyl]-phenyl}-acryloyl)-benzyl ester (prepared as described in Preparation 5, 112 mg, 0.231 mmol) and TEA (0.064 ml, 0.462 mmol) in DMF (1 ml). The resulting solution was stirred at room temperature overnight. The mixture was diluted with water and extracted with AcOEt. The organic phase was washed with water, dried over Na₂SO₄ and evaporated *in vacuo.* The crude mixture was purified by column chromatography (eluent: DCM/MeOH/NH₄OH 99:1:0.2). The resulting oil was dissolved in DCM and treated with HCl/Et₂O for 1 h. The precipitate was filtered and washed with DCM to give 58.6 mg of (E)-3-(4-{(E)-3-[4-((2R,6S)-2,6-dimethyl-morpholin-4-ylmethyl)-phenyl]-3-oxo-propenyl}-phenyl)-N-hydroxy-acrylamide hydrochloride.
Y=56%
LC-MS: Method C, rt=1.33; (ES+) MH⁺: 421.19
¹H NMR (DMSO-d₆ + TFA 353 K) δ (ppm): 8.16 (m, 2 H), 7.80 - 7.92 (m, 5 H), 7.73 (d, 1 H), 7.63 (m, 2 H), 7.49 (d, 1 H), 6.64 (d, 1 H), 4.39 (s, 2 H), 3.90 - 4.19 (m, 2 H), 3.25 (d, 2 H), 2.68 (dd, 2 H), 1.14 (d, 6 H).

### Example 29: (E)-3-(4-{(E)-3-[4-((3R,5S)-3,5-Dimethyl-piperazin-1-ylmethyl)-phenyl]-3-oxo-propenyl}-phenyl)-N-hydroxy-acrylamide

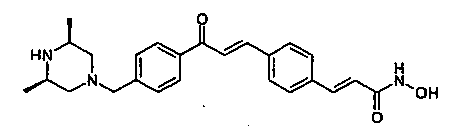

The product was obtained starting from (E)-3-{4-[(E)-3-(4-chloromethyl-phenyl)-3-oxo-propenyl]-phenyl}-N-(tetrahydro-pyran-2-yloxy)-acrylamide (obtained as described in Preparation 5) and (2S,6R)-2,6-dimethyl-piperazine following the experimental procedure described for Example 28. The title compound was obtained as its bis-hydrochloride salt.
LC-MS: Method C, rt=1.16 (ES+) MH⁺: 420.25
¹H NMR (DMSO-d₆ + TFA 353 K) δ (ppm): 8.12 (m, 2 H), 7.86 (m, 2 H), 7.84 (d, 1 H), 7.72 (d, 1 H), 7.60 - 7.69 (m, 4 H), 7.50 (d, 1 H), 6.63 (d, 1 H), 4.02 (s, 2 H), 3.39 - 3.66 (m, 2 H), 3.15 - 3.24 (m, 2 H), 2.57 (dd, 2 H), 1.27 (d, 6 H).

### Example 30: (E)-3-(4-{(E)-3-[4-(4-Acetyl-piperazin-1-ylmethyl)-phenyl]-3-oxo-propenyl}-phenyl)-N-hydroxy-acrylamide

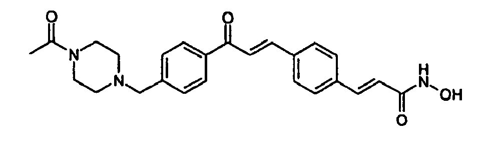

The product was obtained starting from a mixture of methanesulfonic acid 4-((E)-3-{4-[(E)-2-(tetrahydro-pyran-2-yloxycarbamoyl)-vinyl]-phenyl}-acryloyl)-benzyl ester and (E)-3-{4-[(E)-3-(4-chloromethyl-phenyl)-3-oxo-propenyl]-phenyl}-N-(tetrahydro-pyran-2-yloxy)-acrylamide (both obtained as described in Preparation 5) and 1-piperazin-1-yl-ethanone, following the experimental procedure described for Example 28. The title compound was obtained as its hydrochloride salt. LC-MS: Method C, rt=1.19 (ES+) MH⁺: 434.19
¹H NMR (DMSO-d₆ + TFA 353 K) δ (ppm): 8.17 (m, 2 H), 7.68 - 7.96 (m, 6 H), 7.64 (m, 2 H), 7.49 (d, 1 H), 6.64 (d, 1 H), 4.42 (s, 2 H), 3.62 - 3.89 (m, 4 H), 3.04 - 3.32 (m, 4 H), 2.04 (s, 3 H).

### Example 31: (E)-3-(4-{(E)-3-[4-((3R,5S)-4-Acetyl-3,5-dimethyl-piperazin-1-ylmethyl)-phenyl]-3-oxo-propenyl}-phenyl)-N-hydroxy-acrylamide

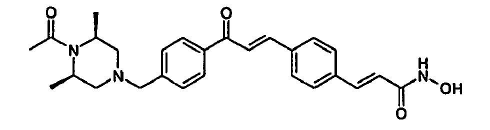

The product was obtained starting from a mixture of methanesulfonic acid 4-((E)-3-{4-[(E)-2-(tetrahydro-pyran-2-yloxycarbamoyl)-vinyl]-phenyl}-acryloyl)-benzyl ester and (E)-3-{4-[(E)-3-(4-chloromethyl-phenyl)-3-oxo-propenyl]-phenyl}-N-(tetrahydro-pyran-2-yloxy)-acrylamide (both obtained as described in Preparation 5) and 1-((2R,6S)-2,6-dimethyl-piperazin-1-yl)-ethanone (obtained as described in Preparation 6), following the experimental procedure described for Example 28. The title compound was obtained as its hydrochloride salt.
LC-MS: Method C, rt=1.28 (ES+) MH⁺: 462.21
¹H NMR (DMSO-d₆ + TFA 353 K) δ (ppm): 8.14 (m, 2 H), 7.68 - 7.96 (m, 6 H), 7.63 (d, 2 H), 7.49 (d, 1 H), 6.64 (d, 1 H), 4.37 - 4.54 (m, 2 H), 4.22 (bs, 2 H), 3.10 (d, 2 H), 2.86 (bs, 2 H), 2.04 (s, 3 H), 1.38 (d, 6 H).

### Example 32: (E)-3-(4-{(E)-3-[4-(4-Ethyl-piperazin-1-ylmethyl)-phenyl]-3-oxo-propenyl}-phenyl)-N-hydroxy-acrylamide

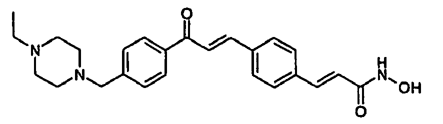

The product was obtained starting from methanesulfonic acid 4-((E)-3-{4-[(E)-2-(tetrahydro-pyran-2-yloxycarbamoyl)-vinyl]-phenyl}-acryloyl)-benzyl ester (obtained as described in Preparation 5) and 1-ethyl-piperazine following the experimental procedure described for Example 28. The title compound was purified by preparative LC-MS and was obtained as its bis-trifluoroacetate salt.
LC-MS: Method C, rt=1.18 (ES+) MH⁺: 420.25
¹H NMR (DMSO-d₆) δ (ppm): 10.79 (bs, 1 H), 9.20 (bs, 1 H), 8.16 (m, 2 H), 7.97 (d, 1 H), 7.93 (m, 2 H), 7.75 (d, 1 H), 7.66 (m, 2 H), 7.55 (m, 2 H), 7.50 (d, 1 H), 6.56 (d, 1 H), 3.77 (s, 2 H), 3.27 - 3.57 (m, 2 H), 3.13 (q, 2 H), 3.02 (bs, 4 H), 2.44 (bs, 2 H), 1.21 (t, 3 H).

### Example 33: (E)-N-Hydroxy-3-{4-[(E)-3-(3-morpholin-4-ylmethyl-phenyl)-3-oxo-propenyl]-phenyl}-acrylamide

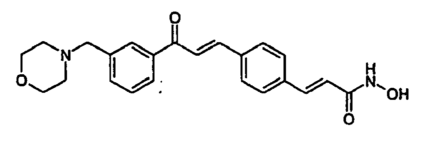

The product was obtained starting from (E)-3-{4-[(E)-3-(3-chloromethyl-phenyl)-3-oxo-propenyl]-phenyl}-N-(tetrahydro-pyran-2-yloxy)-acrylamide (obtained as described in Preparation 7) and morpholine, following the experimental procedure described for the Example 28. The title compound was purified by preparative LC-MS and was obtained as its trifluoroacetate salt.
LC-MS: Method C, rt=1.31 (ES+) MH⁺: 393.08
¹H NMR (DMSO-d₆ 353K +TFA) δ (ppm): 8.19 - 8.26 (m, 2 H), 7.87 (m, 2 H), 7.76 - 7.82 (m, 3 H), 7.61 - 7.74 (m, 3 H), 7.50 (d, 1 H), 6.64 (d, 1 H), 4.47 (s, 2 H), 3.71 - 3.99 (m, 4 H), 3.25 (t, 4 H).

### Example 34: (E)-N-Hydroxy-3-(4-{(E)-3-[3-(4-methyl-piperazin-1-ylmethyl)-phenyl]-3-oxo-propenyl}-phenyl)-acrylamide

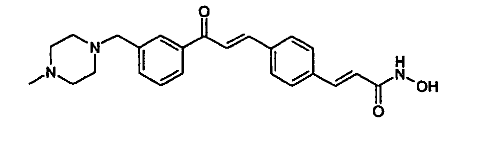

The product was obtained starting from (E)-3-{4-[(E)-3-(3-chloromethyl-phenyl)-3-oxo-propenyl]-phenyl}-N-(tetrahydro-pyran-2-yloxy)-acrylamide (obtained as described in Preparation 7) and N-methylpiperazine, following the experimental procedure described for the Example 28. The title compound was purified by preparative LC-MS and was obtained as its bis-trifluoroacetate salt.
LC-MS: Method C, rt=1.25 (ES+) MH⁺: 406.13
¹H NMR (DMSO-d₆ 353K +TFA) δ (ppm): 8.05 - 8.12 (m, 2 H), 7.73 - 7.90 (m, 4 H), 7.61 - 7.72 (m, 3 H), 7.58 (t, 1 H), 7.50 (d, 1 H), 6.63 (d, 1 H), 3.86 (s, 2 H), 3.25 (t, 4 H), 2.81 - 2.89 (m, 4 H), 2.80 (s, 3 H).

### Example 35: (E)-N-Hydroxy-3-(4-{(E)-3-[2-(4-methyl-piperazin-1-ylmethyl)-phenyl]-3-oxo-propenyl}-phenyl)-acrylamide

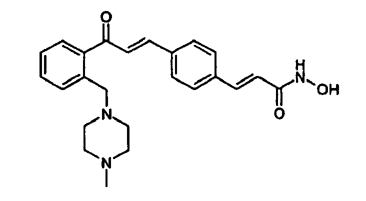

### STEP A

A mixture of 4-formylcinnamic acid (189 mg, 1.077 mmol), 1-[2-(4-methyl-piperazin-1-ylmethyl)-phenyl]-ethanone (obtained as described in Preparation 11, 250 mg, 1.077 mmol) and 1.7 M KOH (1.26 ml) in EtOH (5 ml) and H₂O (5ml) was stirred at room temperature overnight and then acidified with 10% HCl. The resulting precipitate was filtered to give 350 mg of (E)-3-(4-{(E)-3-[2-(4-methyl-piperazin-1-ylmethyl)-phenyl]-3-oxo-propenyl}phenyl)-acrylic acid bis-hydrochloride.
Y= 70%

### STEP B

A mixture of (E)-3-(4-{(E)-3-[2-(4-methyl-piperazin-1-ylmethyl)-phenyl]-3-oxo-propenyl}-phenyl)-acrylic acid bis-hydrochloride (350 mg, 0.756 mmol), HOBT (204 mg, 1.51 mmol), EDC (288 mg, 1.51 mmol), TEA (0.210ml, 1.51 mmol) and NH₂OTHP (106 mg, 0.907 mmol) in DMF (8 ml) was stirred overnight at room temperature and then partitioned between water and AcOEt. The phases were separated and the aqueous layer was brought to basic conditions with NH₄OH and extracted with DCM. The collected organic extracts were dried over Na₂SO₄ and evaporated *in vacuo.* The crude mixture was purified by silica gel chromatography (eluent: DCM/MeOH/NH₄OH 95:5:0.2) and the resulting product was dissolved in DCM and treated with HCl/Et₂O for 1 h. The hygroscopic precipitate was filtered and freeze dried to give 229 mg of (E)-N-Hydroxy-3-(4-{(E)-3-[2-(4-methyl-piperazin-1-ylmethyl)-phenyl]-3-oxo-propenyl}-phenyl)-acrylamide as its bis-hydrochloride salt.
Y= 63%
LC-MS: Method F, rt=1.14; (ES+) MH⁺: 406.25
¹H NMR (DMSO-d₆ 353K +TFA) δ (ppm): 7.77 (m, 2 H), 7.61 (m, 2 H), 7.47 - 7.59 (m, 5 H), 7.43 (d, 1 H), 7.33 (d, 1 H), 6.63 (d, 1 H), 3.91 (s, 2 H), 3.08 - 3.25 (m, 4 H), 2.79 - 2.93 (m, 4 H), 2.71 (s, 3 H).

### Example 36: (E)-N-Hydroxy-3-(5-{(E)-3-[2-(4-methyl-piperazin-1-yl)-phenyl]-3-oxo-propenyl}-pyridin-2-yl)-acrylamide

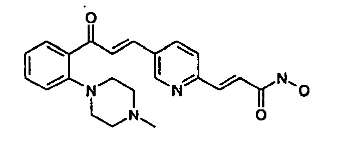

### STEP A

A mixture of (E)-3-(5-formyl-pyridin-2-yl)-acrylic acid *tert*-butyl ester (described in Example 11 STEP A-D, 250 mg, 1.07 mmol), KOH (90 mg, 1.61 mmol) and 1-[2-(4-methyl-piperazin-1-yl)-phenyl]-ethanone (described in Example 1 STEP A-B, 233 mg, 1.07 mmol) in EtOH (10 ml) was stirred at room temperature overnight. The mixture was acidified with 10% HCl and the solvent was removed *in vacuo.* The crude reaction mixture (700 mg) was used in the next step without any further purification.

### STEP B

(E)-3-(5-{(E)-3-[2-(4-Methyl-piperazin-1-yl)-phenyl]-3-oxo-propenyl}-pyridin-2-yl)-acrylic acid hydrochloride (crude mixture from Step A, 400 mg) was suspended in DMF (10 ml). HOBT (240 mg, 1.78 mmol), EDC (340 mg, 1.78 mmol), TEA (0.371 ml, 2.67 mmol) and NH₂OTHP (125 mg, 1.06 mmol) were added to the resulting slurry. The mixture was stirred overnight at room temperature and then partitioned between water and AcOEt. The organic phase was washed with water, dried over Na₂SO₄ and evaporated *in vacuo.* The crude reaction mixture was purified by column chromatography (DCM/MeOH/NH₄OH 95:5:0.2). The collected fractions were evaporated and the resulting product was dissolved in DCM and treated with HCl/Et₂O for 1 h. The precipitate was filtered off and purified by preparative LC-MS to give 23.7 mg of (E)-N-hydroxy-3-(5-{(E)-3-[2-(4-methyl-piperazin-1-yl)-phenyl]-3-oxo-propenyl}-pyridin-2-yl)-acrylamide as its trifluoroacetate salt.
LC-MS: Method C, rt=1.26; (ES+) MH⁺: 393.12
¹H NMR (DMSO-d₆) δ (ppm): 9.81 (bs, 1 H), 8.97 (d, 1H), 8.29 (dd, 1 H), 7.67 (d, 1 H), 7.62 (d, 1 H), 7.48 - 7.60 (m, 4 H), 7.30 (d, 1 H), 7.21 (td, 1 H), 7.00 (d, 1 H), 3.47 (d, 2 H), 3.22 - 3.40 (m, 2 H), 3.02 - 3.21 (m, 2 H), 2.82 - 3.02 (m, 2 H), 2.75 (s, 3 H).

### Example 37: (E)-N-Hydroxy-3-(5-{(E)-3-[3-(4-methyl-piperazin-1-yl)-phenyl]-3-oxo-propenyl}-pyridin-2-yl)-acrylamide

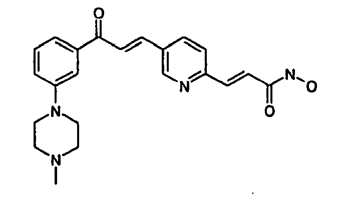

### STEP A

A mixture of (E)-3-(5-formyl-pyridin-2-yl)-acrylic acid *tert*-butyl ester (described in Example 11 STEP A-D, 190 mg, 0.81 mmol), KOH 1.7 M (0.716 ml, 1.22 mmol) and 1-[3-(4-methyl-piperazin-1-yl)-phenyl]-ethanone (177 mg, 0.812 mmol) in EtOH (8 ml) was stirred at 0°C for 4 h. The resulting slurry was partitioned between water and AcOEt and the organic phase was dried over Na₂SO₄ and evaporated *in vacuo.* The crude reaction mixture was purified by column chromatography (eluent: DCM/MeOH/NH₄OH 98:2:0.2). The collected fractions were evaporated and the resulting powder was dissolved in DCM (10 ml) and TFA (1 ml). The solution was stirred at room temperature for 72 h. The solvent was then removed and the residue was triturated in Et₂O to give 164 mg (E)-3-(5-{(E)-3-[3-(4-methyl-piperazin-1-yl)-phenyl]-3-oxo-propenyl}-pyridin-2-yl)-acrylic acid as its bis-trifluoroacetate salt.
Y= 32%

### STEP B

(E)-3-(5-{(E)-3-[3-(4-Methyl-piperazin-1-yl)-phenyl]-3-oxo-propenyl}-pyridin-2-yl)-acrylic acid bis-trifluoroacetate (164 mg, 0.27 mmol) was dissolved in DMF (7 ml). HOBT (90 mg, 0.668 mmol), EDC (127 mg, 0.668 mmol), TEA (0.139 ml, 1 mmol) and NH₂OTH (47 mg, 0.400 mmol) were added to the resulting solution. The mixture was stirred overnight at room temperature and then partitioned between water and AcOEt. Then the organic phase was dried over Na₂SO₄, evaporated *in vacuo* and the crude product was purified by column chromatography (eluent: DCM/MeOH/NH₄OH 98:2:0.2). The collected fractions were evaporated and the resulting product was dissolved in DCM and treated with HCl/Et₂O for 1 h. The precipitate was filtered off and purified by preparative LC-MS to give 23.6 mg of (E)-N-hydroxy-3-(5-{(E)-3-[3-(4-methyl-piperazin-1-yl)-phenyl]-3-oxo-propenyl}-pyridin-2-yl)-acrylamide as its bis-trifluoroacetate salt.
Y= 14%
LC-MS: Method C, rt=1.17; (ES+) MH⁺: 393.25
¹H NMR (DMSO-d₆) δ (ppm): 10.96 (bs, 1 H), 9.66 (bs, 1 H), 9.05 (d, 1 H), 8.39 (dd, 1 H), 8.06 (d, 1 H), 7.78 (d, 1 H), 7.62 - 7.73 (m, 3 H), 7.53 (d, 1 H), 7.48 (t, 1 H), 7.34 (dd, 1 H), 7.01 (d, 1 H), 3.95 - 4.05 (m, 2 H), 3.52 - 3.62 (m, 2 H), 2.96 - 3.29 (m, 4 H), 2.89 (dd, 3 H).

### Example 38: (E)-3-(5-{(E)-3-[4-(4-Benzyl-piperazin-1-yl)-phenyl]-3-oxo-propenyl}-pyridin-2-yl)-N-hydroxy-acrylamide

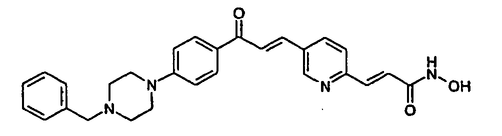

### STEP A

A mixture of 1-(4-piperazin-1-yl-phenyl)-ethanone (500 mg, 2.45 mmol), benzaldehyde (312 mg, 2.94 mmol) and NaBH(OAc)₃ (778 mg, 3.67 mmol) in AcOEt (15 ml) was stirred at room temperature overnight. The resulting solution was partitioned between water and DCM and the organic phase was dried over Na₂SO₄ and evaporated *in vacuo.* The crude product was purified by silica gel chromatography (eluent: DCM/MeOH/NH₄OH 95:5:0.2) to give 390 mg of 1-[4-(4-benzyl-piperazin-1-yl)-phenyl]-ethanone.
Y= 54%

### STEP B

A mixture of 1-[4-(4-benzyl-piperazin-1-yl)-phenyl]-ethanone (319 mg, 1.085 mmol), (E)-3-(5-formyl-pyridin-2-yl)-acrylic acid *tert*-butyl ester (described in Example 11 STEP A-D, 253 mg, 1.08 mmol) and KOH 1.7 M (0.638 ml) in EtOH (10 ml) was stirred at 0°C overnight. The resulting precipitate was filtered washing with hot EtOH and the powder was dissolved in DCM (10 ml) and treated with TFA (2 ml) for 6 h. The solvent was then removed *in vacuo* to give 388 mg of (E)-3-(5-{(E)-3-[4-(4-benzyl-piperazin-1-yl)-phenyl]-3-oxo-propenyl}-pyridin-2-yl)-acrylic acid as its bis-trifluoroacetate salt.
Y= 53%

### STEP C

(E)-3-(5-{(E)-3-[4-(4-Benzyl-piperazin-1-yl)-phenyl]-3-oxo-propenyl}-pyridin-2-yl)-acrylic acid bis-trifluoroacetate (388 mg, 0.569 mmol) was dissolved in DMF (6 ml). HOBT (154 mg, 1.14 mmol), EDC (217.7 mg, 1.14 mmol), TEA (0.238 ml, 1.74 mmol) and NH₂OTH (80 mg, 0.684 mmol) were added to the resulting solution. The mixture was stirred at room temperature overnight and then partitioned between water and AcOEt. The organic phase was washed with water, dried over Na₂SO₄ and evaporated *in vacuo.* The crude reaction mixture was purified by column chromatography (eluent: DCM/MeOH/NH₄OH 98:2:0.2). The collected fractions were evaporated and the resulting product was dissolved in DCM and treated with HCl/Et₂O for 1 h. The precipitate was filtered off and washed with DCM to give 95.4 mg of (E)-3-(5-{(E)-3-[4-(4-benzyl-piperazin-1-yl)-phenyl]-3-oxo-propenyl}-pyridin-2-yl)-N-hydroxy-acrylamide as its bis-hydrochloride salt.
Y= 31%
LC-MS: Method E, rt=2.00; (ES+) MH⁺: 469.33
¹H NMR (DMSO-d₆) δ (ppm): 11.46 (bs, 1 H), 9.05 (d, 1 H), 8.44 (dd, 1 H), 8.12 (d, 2 H), 8.09 (d, 1 H), 7.62 - 7.80 (m, 4 H), 7.53 (d, 1 H), 7.44 - 7.50 (m, 3 H), 7.09 (d, 2 H), 7.03 (d, 1 H), 4.38 (d, 2 H), 4.08 - 4.21 (m, 2 H), 3.30 - 3.51 (m, 4 H), 2.99 - 3.24 (m, 2 H).

### Example 39: (E)-N-Hydroxy-3-(5-{(E)-3-oxo-3-[4-((3R,5S)-3,4,5-trimethyl-piperazin-1-yl)-phenyl]-propenyl}-pyridin-2-yl)-acrylamide

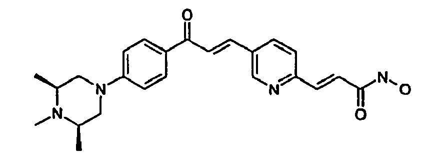

The title compound was obtained starting from 1-[4-((3R,5S)-3,4,5-trimethyl-piperazin-1-yl)-phenyl]-ethanone (prepared as described in Preparation 3) and (E)-3-(5-formyl-pyridin-2-yl)-acrylic acid *tert*-butyl ester (described in Example 11 STEP A-D), following procedure described for Example 38 (STEP B-C). The title compound was purified by preparative LC-MS and was obtained as its bis-trifluoroacetate salt.
LC-MS: Method C, rt=1.61; (ES+) MH⁺: 421.23
¹H NMR (DMSO-d₆) δ (ppm): 10.95 (bs, 1 H), 9.26 (bs, 1 H), 9.02 (d, 1 H), 8.38 (dd, 1 H), 8.03 - 8.18 (m, 3 H), 7.72 (d, 1 H), 7.68 (d, 1 H), 7.53 (d, 1 H), 7.16 (d, 2 H), 7.00 (d, 1 H), 4.24 (d, 2 H), 3.51 (bs, 2 H), 2.96 (dd, 2 H), 2.88 (d, 3 H), 1.39 (d, 6 H).

### Example 40: (E)-N-Hydroxy-3-(5-[(E)-3-(4-morpholin-4-ylmethyl-phenyl)-3-oxo-propenyl]-pyridin-2-yl)-acrylamide

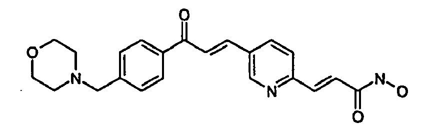

The title compound was obtained starting from 1-(4-morpholin-4-ylmethyl-phenyl)-ethanone (described in Example 7 STEP A and B) and (E)-3-(5-formyl-pyridin-2-yl)-acrylic acid *tert*-butyl ester (described in Example 11 STEP A-D), following the procedure described for Example 38 (STEP B-C). The title compound was obtained as its bis-hydrochloride salt.
LC-MS: Method C, rt=1.06; (ES+) MH⁺: 394.26
¹H NMR (DMSO-d₆) δ (ppm): 9.07 (d, 1 H), 8.44 (dd, 1 H), 8.25 (d, 2 H), 8.13 (d, 1 H), 7.86 (d, 2 H), 7.81 (d, 1 H), 7.72 (d, 1 H), 7.53 (d, 1 H), 7.03 (d, 1 H), 4.44 (m, 2 H), 3.75 - 4.01 (m, 4 H), 3.02 - 3.34 (m, 4 H).

### Example 41: (E)-3-(5-{(E)-3-[4-(4-Ethyl-piperazin-1-yl)-phenyl]-3-oxo-propenyl}-pyridin-2-yl)-N-hydroxy-acrylamide

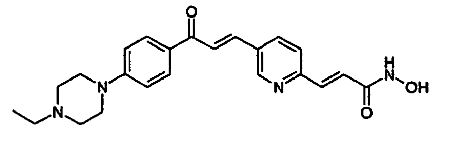

### STEP A

A mixture of 1-(4-piperazin-1-yl-phenyl)-ethanone (500 mg, 2.45 mmol), CH₃CHO (161 mg, 3.67 mmol) and NaBH(OAc)₃ (778 mg, 3.67 mmol) in DCM (15 ml) was stirred at room temperature overnight. The resulting solution was partitioned between water and DCM and the organic phase was extracted with 1 M HCl. The aqueous layer was brought to basic conditions with NH₄OH, extracted with AcOEt and then the organic phase was dried over Na₂SO₄ and evaporated *in vacuo* to give 250 mg of 1-[4-(4-ethyl-piperazin-1-yl)-phenyl]-ethanone.
Y= 44%

### STEP B

A mixture of 1-[4-(4-ethyl-piperazin-1-yl)-phenyl]-ethanone (250 mg, 1.073 mmol), (E)-3-(5-formyl-pyridin-2-yl)-acrylic acid *tert*-butyl ester (described in Example 11 STEP A-D, 250 mg, 1.073 mmol) and KOH 1.7 M (1.26 ml) in EtOH (10 ml) was stirred at 0°C for 2 h and then at 4°C overnight. The solution was then acidified with HCl/Et₂O and the solvent was removed *in vacuo.* The crude reaction mixture was dissolved in DCM (10 ml) and TFA (2 ml) and the solution was stirred at room temperature for 6 h. The solvent was removed under vacuo and the resulting solid was triturated with EtOH to give 391 mg of (E)-3-(5-{(E)-3-[4-(4-ethyl-piperazin-1-yl)-phenyl]-3-oxo-propenyl}-pyridin-2-yl)-acrylic acid bis-trifluoroacetate as a yellow powder.
Y= 59%

### STEP

(E)-3-(5-{(E)-3-[4-(4-Ethyl-piperazin-1-yl)-phenyl]-3-oxo-propenyl}-pyridin-2-yl)-acrylic acid bis-trifluoroacetate (391 mg, 0.631 mmol) was dissolved in DMF (7 ml) and TEA (0.263 ml, 1.89 mmol). HOBT (170 mg, 1.26 mmol), EDC (240 mg, 1.26 mmol), and NH₂OTHP (89 mg, 0.758 mmol) were added to the resulting solution. The mixture was stirred at room temperature overnight and then partitioned between water and AcOEt. The organic phase was washed with water, dried over Na₂SO₄ and evaporated *in vacuo.* The crude reaction mixture was purified by column chromatography (eluent: DCM/MeOH/NH₄OH 99:1:0.2). The collected fractions were evaporated and the resulting product was dissolved in DCM and treated with HCl/Et₂O for 1 h. The precipitate was filtered off, washed with DCM and Et₂O to give 112 mg of (E)-3-(5-{(E)-3-[4-(4-ethyl-piperazin-1-yl)-phenyl]-3-oxo-propenyl}-pyridin-2-yl)-N-hydroxy-acrylamide bis-hydrochloride.
Y= 37%
LC-MS: Method C, rt=1.09; (ES+) MH⁺: 407.32
¹H NMR (DMSO-d₆ + TFA) δ (ppm): 11.31 (bs, 1 H), 9.09 (d, 1 H), 8.52 (dd, 1 H), 8.13 (m, 2 H), 8.14 (d, 1 H), 7.80 (d, 1 H), 7.72 (d, 1 H), 7.56 (d, 1 H), 7.12 (m, 2 H), 7.07 (d, 1 H), 4.13 (d, 2 H), 3.55 (d, 2 H), 3.26 - 3.46 (m, 2 H), 2.96 - 3.23 (m, 4 H), 1.30 (t, 3 H).

### Example 42: (E)-3-(5-{(E)-3-[4-(4-Acetyl-piperazin-1-yl)-phenyl]-3-oxo-propenyl}-pyridin-2-yl)-N-hydroxy-acrylamide

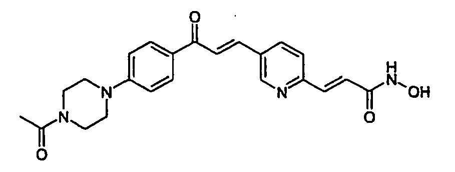

The title compound was obtained starting from 1-[4-(4-acetyl-piperazin-1-yl)-phenyl]-ethanone (prepared following the procedure described in Example 16 STEP A) and (E)-3-(5-formyl-pyridin-2-yl)-acrylic acid *tert*-butyl ester (described in Example 11 STEP A-D), following the procedure described for Example 38 (STEP B-C). The title compound was purified by preparative LC-MS and was obtained as its trifluoroacetate salt.
LC-MS: Method C, rt=1.32; (ES+) MH⁺: 421.19
¹H NMR (DMSO-d₆) δ (ppm): 9.01 (d, 1 H), 8.37 (dd, 1 H), 8.09 (m, 2 H), 8.08 (d, 1 H), 7.69 (d, 1 H), 7.67 (d, 1 H), 7.52 (d, 1 H), 7.04 (m, 2 H), 7.00 (d, 1 H), 3.57 - 3.67 (m, 4 H), 3.33 - 3.49 (m, 4 H), 2.05 (s, 3 H).

### Example 43: (E)-N-Hydroxy-3-(5-{(E)-3-oxo-3-[3-((3R,5S)-3,4,5-trimethyl-piperazin-1-yl)-phenyl]-propenyl}-pyridin-2-yl)-acrylamide

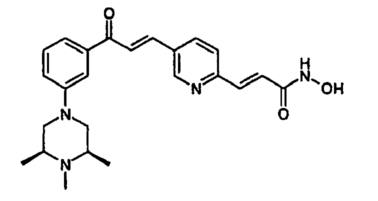

### STEP A

A mixture of (E)-3-(5-formyl-pyridin-2-yl)-acrylic acid *tert*-butyl ester (described in Example 11 STEP A-D, 168 mg, 0.718 mmol), 1.7 M KOH (0.674 ml) and 1-[3-((3R,5S)-3,4,5-trimethyl-piperazin-1-yl)-phenyl]-ethanone (described in Preparation 8, 188 mg, 0.764 mmol) in EtOH (7 ml) was stirred at 0°C for 6 h. The resulting precipitate was filtered off and dissolved in DCM (5 ml) and TFA (1 ml). The mixture was stirred at room temperature for 4 h and then the solvent was removed *in vacuo* to give 282 mg of (E)-3-(5-{(E)-3-oxo-3-[3-((3R,5S)-3,4,5-trimethyl-piperazin-1-yl)-phenyl]-propenyl}-pyridin-2-yl)-acrylic acid as its bis-trifluoroacetate salt.
Y= 58%

### STEP B

A mixture of (E)-3-(5-{(E)-3-oxo-3-[3-((3R,5S)-3,4,5-trimethyl-piperazin-1-yl)-phenyl]-propenyl}-pyridin-2-yl)-acrylic acid bis-trifluoroacetate (282 mg, 0.445 mmol), HOBT (120 mg, 0.890 mmol), EDC (170 mg, 0.890 mmol), TEA (0.186 ml, 1.33 mmol) and NH₂OTHP (62 mg, 0.534 mmol) in DMF (5 ml), was stirred at room temperature overnight and then partitioned between water and AcOEt. The organic extract was dried over Na₂SO₄, evaporated *in vacuo* and the crude was purified by column chromatography (eluent: DCM/MeOH/NH₄OH 96:4:0.2). The collected fractions were evaporated and the resulting product was dissolved in DCM and treated with HCl/Et₂O for 1 h. The precipitate was filtered off and washed with DCM to give 26.6 mg of (E)-N-hydroxy-3-(5-{(E)-3-oxo-3-[3-((3R,5S)-3,4,5-trimethyl-piperazin-1-yl)-phenyl]-propenyl}-pyridin-2-yl)-acrylamide as its bis-hydrochloride salt.
Y= 12%
LC-MS: Method C, rt=1.25; (ES+) MH⁺: 421.2
¹H NMR (DMSO-d₆) δ (ppm): 10.84 (bs, 2 H), 9.08 (d, 1 H), 8.46 (dd, 1 H), 8.09 (d, 1 H), 7.78 (d, 1 H), 7.63 - 7.77 (m, 3 H), 7.54 (d, 1 H), 7.47 (dd, 1 H), 7.36 (dd, 1 H), 7.04 (d, 1 H), 3.97 - 4.12 (m, 2 H), 3.27 - 3.49 (m, 2 H), 3.06 (dd, 2 H), 2.82 (d, 3 H), 1.44 (d, 6 H).

### Example 44: (E)-N-Hydroxy-3-(5-{(E)-3-[4-(1-methyl-piperidin-4-ylmethyl)-phenyl]-3-oxo-propenyl}-pyridin-2-yl)-acrylamide

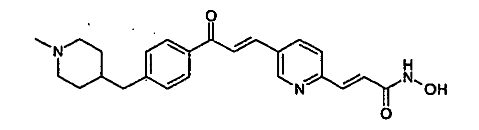

The product was obtained starting from (E)-3-(5-formyl-pyridin-2-yl)-acrylic acid *tert*-butyl ester (described in Example 11 STEP A-D) and 1-[4-(1-methyl-piperidin-4-ylmethyl)-phenyl]-ethanone (prepared as described in Preparation 2) following the experimental procedure described for Example 43.
The title compound was obtained as its bis-hydrochloric salt.
LC-MS: Method C, rt=1.24; (ES+) MH⁺: 406.18
¹H NMR (DMSO-d₆ + TFA 353 K) δ (ppm): 9.91 (s, 1 H), 8.99 (d, 1 H), 8.30 (dd, 1 H), 8.07 (m, 2 H), 7.95 (d, 1 H), 7.73 (d, 1 H), 7.65 (d, 1 H), 7.51 (d, 1 H), 7.40 (m, 2 H), 7.05 (d, 1 H), 3.39 (d, 2 H), 2.82 - 2.96 (m, 2 H), 2.71 (s, 3 H), 2.70 (d, 2 H), 1.74 - 1.95 (m, 3 H), 1.46 - 1.67 (m, 2 H).

### Example 45: (E)-N-Hydroxy-3-{5-[(E)-3-oxo-3-(4-piperazin-1-yl-phenyl)-propenyl]-pyridin-2-yl}-acrylamide

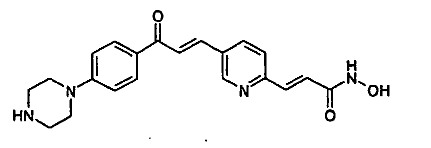

### STEP A

A mixture of (E)-3-(5-formyl-pyridin-2-yl)-acrylic acid *tert*-butyl ester (described in Example 11 STEP A-D, 250 mg, 1.07 mmol) and TFA (1 ml) in DCM (4 ml) was stirred at room temperature for 6 h. The solvent was then removed *in vacuo* and the solid was triturated with Et₂O to give 272 mg of (E)-3-(5-formyl-pyridin-2-yl)-acrylic acid as its trifluoroacetate salt.
Y= 87%

### STEP B

A mixture of (E)-3-(5-formyl-pyridin-2-yl)-acrylic acid trifluoroacetate (272 mg, 0.93 mmol), 4-(4-acetyl-phenyl)-piperazine-1-carboxylic acid tert-butyl ester (obtained as described in Preparation 9, 283 mg, 0.93 mmol) and 1.7 M KOH (0.820 ml) in EtOH (10 ml) was stirred at 0°C for 8 h and then at room temperature for 6 days. The mixture was acidified with 10% HCl until reaching a pH value of 3 and the resulting precipitate was filtered to give 184 mg of 4-(4-{(E)-3-[6-((E)-2-carboxyvinyl)-pyridin-3-yl]-acryloyl}-phenyl)-piperazine-1-carboxylic acid tert-butyl ester hydrochloride.
Y= 40%

### STEP C

4-(4-{(E)-3-[6-((E)-2-carboxy-vinyl)-pyridin-3-yl]-acryloyl}-phenyl)-piperazine-1-carboxylic acid tert-butyl ester hydrochloride (184 mg, 0.363 mmol) was dissolved in DMF (5 ml), THF (5 ml) and TEA (0.190 ml, 1.47 mmol). Then EDC (140 mg, 0.736 mmol), HOBT (99 mg, 0.736 mmol) and NH₂OTHP (51.6 mg, 0.441 mmol) were added to the resulting solution. The mixture was stirred at room temperature overnight and then partitioned between water and AcOEt. The organic phase was dried over Na₂SO₄ and evaporated *in vacuo.* The crude reaction mixture was purified by column chromatography (eluent: DCM/MeOH/NH₄OH 98:2:0.2) and the resulting compound was dissolved in DCM and treated with HCl/Et₂O for 1 h. The precipitate was filtered off and washed with DCM. The hygroscopic powder was dissolved in water and freeze dried to give 53 mg of (E)-N-hydroxy-3-{5-[(E)-3-oxo-3-(4-piperazin-1-yl-phenyl)-propenyl]-pyridin-2-yl}-acrylamide as its bis-hydrochloride salt.
Y= 32%
LC-MS: Method C, rt=1.08; (ES+) MH⁺: 379.18
¹H NMR (DMSO-d₆ + TFA 353 K) δ (ppm): 9.20 (bs, 2 H), 8.98 (d, 1 H), 8.29 (dd, 1 H), 8.07 (m, 2 H), 7.95 (d, 1 H), 7.68 (d, 1 H), 7.65 (d, 1 H), 7.51 (d, 1 H), 7.08 (m, 2 H), 7.05 (d, 1 H), 3.59 - 3:70 (m, 4 H), 3.16 - 3.33 (m, 4 H).

### Example 46: (E)-3-(4-{(E)-3-[5-Chloro-2-(4-methyl-piperazin-1-yl)-phenyl]-3-oxo-propenyl}-phenyl)-N-hydroxy-acrylamide

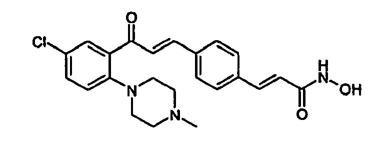

### STEP A

A mixture of 4-formylcinnamic acid (397 mg, 2.25 mmol), 1-[5-chloro-2-(4-methyl-piperazin-1-yl)-phenyl]-ethanone (obtained as described in Preparation 10, 570 mg, 2.25 mmol) and 1.7 M KOH (2.66 ml) in EtOH (25 ml) was stirred at 0°C for 3 h and then acidified with 10% HCl. The solution was concentrated until formation of a yellow precipitate. The solid was filtered to give 823 mg of (E)-3-(4-{(E)-3-[5-chloro-2-(4-methyl-piperazin-1-yl)-phenyl]-3-oxo-propenyl}-phenyl)-acrylic acid hydrochloride.
Y=82%

### STEP B

A mixture of (E)-3-(4-{(E)-3-[5-chloro-2-(4-methyl-piperazin-1-yl)-phenyl]-3-oxo-propenyl}-phenyl)-acrylic acid hydrochloride (250 mg, 0.56 mmol), HOBT (139 mg, 1.03 mmol), EDC (196 mg, 1.03 mmol), TEA (0.568 ml, 4.08 mmol) and NH₂OTHP (89 mg, 0.765 mmol) in DCM (5 ml) was stirred overnight at room temperature. The solvent was evaporated and the residue was partitioned between water and AcOEt. The phases were separated and the aqueous layer was brought to basic conditions with NH₄OH and extracted with DCM. The collected organic extract were dried over Na₂SO₄ and evaporated *in vacuo.* The crude mixture was purified by silica gel chromatography (eluent: DCM/MeOH/NH₄OH 97:3:0.1) and the resulting product was dissolved in DCM and treated with HCl/Et₂O for 2.5 h. The precipitate was filtered and triturated with isopropanol and di-isopropylether to give 61 mg of (E)-3-(4-{(E)-3-[5-chloro-2-(4-methyl-piperazin-1-yl)-phenyl]-3-oxo-propenyl}-phenyl)-N-hydroxy-acrylamide hydrochloride.
Y= 23%
LC-MS: Method F, rt=1.61; (ES+) MH⁺: 426.17
1H NMR (DMSO-d₆) δ (ppm): 10.83 (bs, 1 H), 7.85 (m, 2 H), 7.64 (m, 2 H), 7.42 - 7.62 (m, 5 H), 7.31 (d, 1 H), 6.57 (d, 1 H), 3.43 (d, 2 H), 3.08 - 3.35 (m, 4 H), 2.78 - 3.01 (m, 2 H), 2.67 (d, 3 H).

### Example 47: (E)-N-Hydroxy-3-(5-{(E)-3-[2-(4-methyl-piperazin-1-ylmethyl)-phenyl]-3-oxo-propenyl}-pyridin-2-yl)-acrylamide

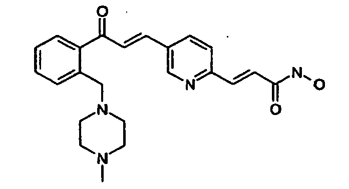

### STEP A

A mixture of 1-[2-(4-methyl-piperazin-1-ylmethyl)-phenyl]-ethanone (obtained as described in Preparation 11, 250 mg, 1.07 mmol), (E)-3-(5-formyl-pyridin-2-yl)-acrylic acid *tert*-butyl ester (described in Example 11 STEP A-D, 251 mg, 1.07 mmol) and 1.7 M KOH (0.633 ml) in EtOH (10 ml) was stirred at 4°C overnight. The resulting solution was diluted with water and extracted with AcOEt. The organic phase was dried over Na₂SO₄ and evaporated *in vacuo.* The crude reaction mixture was purified by column chromatography (eluent: DCM/MeOH/NH₄OH 95:5:0.2) and the resulting product was dissolved in DCM (10 ml) and TFA (2 ml) and stirred at room temperature for 4 h. The solvent was evaporated to give 558 mg of (E)-3-(5-{(E)-3-[2-(4-methyl-piperazin-1-ylmethyl)-phenyl]-3-oxo-propenyl}-pyridin-2-yl)-acrylic acid as its tris-trifluoroacetate salt.
Y= 71%

### STEP B

A mixture of (E)-3-(5-{(E)-3-[2-(4-methyl-piperazin-1-ylmethyl)-phenyl]-3-oxo-propenyl}-pyridin-2-yl)-acrylic acid tris-trifluoroacetate (558 mg, 0.76 mmol), HOBT (205 mg, 1.52 mmol), EDC (290 mg, 1.52 mmol), TEA (0.423 ml, 3.04 mmol) and NH₂OTHP (107 mg, 0.913 mmol) in DMF (10 ml) was stirred overnight at room temperature. The solution was diluted with water, brought to basic conditions with NH₄OH and extracted with AcOEt and DCM. The collected organic extracts were dried over Na₂SO₄ and evaporated *in vacuo.* The crude mixture was purified by silica gel chromatography (eluent: DCM/MeOH/NH₄OH 96:4:0.2) and the resulting product was dissolved in DCM and treated with HCl/Et₂O for 2 h. The hygroscopic precipitate was filtered and freeze dried to give 130 mg of the title compound. 70 mg were purified by preparative LC-MS to give 35 mg of (E)-N-hydroxy-3-(5-{(E)-3-[2-(4-methyl-piperazin-1-ylmethyl)-phenyl]-3-oxo-propenyl}-pyridin-2-yl)-acrylamide as its fomate salt.
LC-MS: Method F, rt=0.93; (ES+) MH⁺: 407.20
¹H NMR (DMSO-d₆) δ (ppm): 10.90 (bs, 1 H), 8.90 (d, 1 H), 8.24 (dd, 1 H), 8.15 (s, 1 H), 7.63 (d, 1 H), 7.50 (d, 1 H), 7.33 - 7.47 (m, 5 H), 7.20 - 7.33 (m, 1 H), 6.98 (d, 1 H), 3.58 (s, 2 H), 2.27 (m, 4 H), 2.18 (m, 4 H), 2.05 (s, 3 H).

### Example 48: (E)-N-Hydroxy-3-{5-[(E)-3-oxo-3-(4-piperazin-1-ylmethyl-phenyl)-propenyl]-pyridin-2-yl}-acrylamide

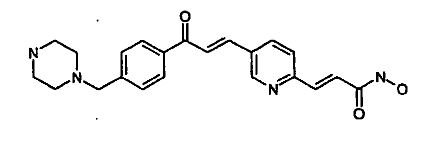

### STEP A

A mixture of (E)-3-(5-formyl-pyridin-2-yl)-acrylic acid trifluoroacetate (described in Example 45 STEP A, 206 mg, 0.71 mmol), 4-(4-acetyl-benzyl)-piperazine-1-carboxylic acid tert-butyl ester (described in Preparation 12, 368 mg, 1.16 mmol) and 1.7 M KOH (2 ml) in EtOH (12 ml) was stirred at 4°C overnight. The resulting precipitate was filtered and washed with AcOEt to give 250 mg of 4-(4-{(E)-3-[6-((E)-2-carboxy-vinyl)-pyridin-3-yl]-acryloyl}-benzyl)-piperazine-1-carboxylic acid tert-butyl ester as its potassium salt.
Y= 69%

### STEP B

A mixture of 4-(4-{(E)-3-[6-((E)-2-carboxy-vinyl)-pyridin-3-yl]-acryloyl}-benzyl)-piperazine-1-carboxylic acid tert-butyl ester potassium salt (250 mg, 0.48 mmol), HOBT (130 mg, 0.96 mmol), EDC (184 mg, 0.96 mmol), TEA (0.134 ml, 0.96 mmol) and NH₂OTHP (84 mg, 0.72 mmol) in DMF (4 ml) and DCM (4 ml) was stirred at room temperature for 5 h. The solution was diluted with DCM and washed with water and brine. The organic phase was dried over Na₂SO₄ and evaporated *in vacuo.* The crude mixture was purified by column chromatography (eluent: DCM/MeOH/NH₄OH 95:5:0.1) and the resulting product was dissolved in DCM and treated with HCl/Et₂O for 4 h. The resulting precipitate was filtered and purified by preparative LC-MS to give 40 mg of (E)-N-hydroxy-3-{5-[(E)-3-oxo-3-(4-piperazin-1-ylmethyl-phenyl)-propenyl]-pyridin-2-yl}-acrylamide as its formate salt.
Y= 19%
LC-MS: Method F, rt=0.91; (ES+) MH⁺: 393.18
1H NMR (DMSO-d₆ 353K) δ (ppm): 8.99 (d, 1 H), 8.29 (dd, 1 H), 8.18 (s, 1 H), 8.08 (m, 2 H), 7.87 - 8.01 (m, 1 H), 7.68 - 7.80 (m, 1 H), 7.64 (d, 1 H), 7.51 (d, 1 H), 7.51 (m, 2 H), 7.05 (d, 1 H), 3.58 (s, 2 H), 2.75 - 2.86 (m, 4 H), 2.33 - 2.46 (m, 4 H).

### Example 49: (E)-3-(5-{(E)-3-[4-(4-Acetyl-piperazin-1-ylmethyl)-phenyl]-3-oxo-propenyl}-pyridin-2-yl)-N-hydroxy-acrylamide

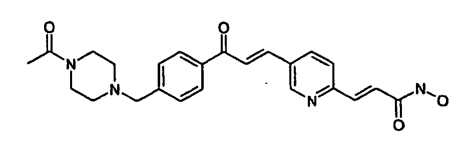

### STEP A

A mixture of 1-[4-(4-acetyl-benzyl)-piperazin-1-yl]-ethanone (prepared following the procedure described in Preparation 12, 500 mg, 1.92 mmol), (E)-3-(5-formyl-pyridin-2-yl)-acrylic acid *tert*-butyl ester (described in Example 11 STEP A-D, 448 mg, 1.92 mmol) and 1.7 M KOH (1.2 ml) in EtOH (19 ml) was stirred at room temperature for 6 h and then partitioned between water and AcOEt. The organic phase was dried over Na₂SO₄ and evaporated *in vacuo.* The crude reaction mixture was purified by column chromatography (eluent: DCM/MeOH/NH₄OH 97:3:0.1) and the resulting product was dissolved in DCM (1.5 ml) and TFA (0.560 ml). The solution was stirred at room temperature for 3 h and then the solvent was evaporated to give 300 mg (E)-3-(5-{(E)-3-[4-(4-acetyl-piperazin-1-ylmethyl)-phenyl]-3-oxo-propenyl}-pyridin-2-yl)-acrylic acid as its bis-trifluoroacetate salt (the compound was used without further purification in the next step).

### STEP B

A mixture of (E)-3-(5-{(E)-3-[4-(4-acetyl-piperazin-1-ylmethyl)-phenyl]-3-oxo-propenyl}pyridin-2-yl)-acrylic acid bis-trifluoroacetate (300 mg), HOBT (105 mg, 0.78 mmol), EDC (149 mg, 0.78 mmol), TEA (0.271 ml, 1.95 mmol) and NH₂OTH (69 mg, 0.58 mmol) in DMF (2 ml) and DCM (20 ml) was stirred at room temperature overnight. Further NH₂OTHP (69 mg, 0.58 mmol) was added and after stirring at room temperature overnight the solution was diluted with DCM and washed with water and brine. The organic phase was dried over Na₂SO₄ and evaporated *in vacuo.* The crude mixture was purified by silica gel chromatography (eluent: DCM/MeOH/NH₄OH 96:4:0.1) and the resulting product was dissolved in DCM and treated with HCl/Et₂O for 3 h. The resulting precipitate was filtered and purified by preparative LC-MS to give 9.4 mg of (E)-3-(5-{(E)-3-[4-(4-acetyl-piperazin-1-ylmethyl)-phenyl]-3-oxo-propenyl}-pyridin-2-yl)-N-hydroxy-acrylamide as its bis-trifluoroacetate salt.
LC-MS: Method F, rt=0.94; (ES+) MH⁺: 435.21
¹H NMR (DMSO-d₆ 353K +TFA) δ (ppm): 9.01 (d, 1 H), 8.31 (dd, 1 H), 8.21 (m, 2 H), 7.97 (d, 1 H), 7.77 (d, 1 H), 7.71 (m, 2 H), 7.66 (d, 1 H), 7.52 (d, 1 H), 7.05 (d, 1 H), 4.42 (s, 2 H), 3.71 (m, 4 H), 3.03 - 3.35 (m, 4 H), 2.05 (s, 3 H).

### Example 50: (E)-N-Hydroxy-3-(5-{(E)-3-[4-(4-methyl-piperazin-1-ylmethyl)-phenyl]-3-oxo-propenyl}-pyridin-2-yl)-acrylamide

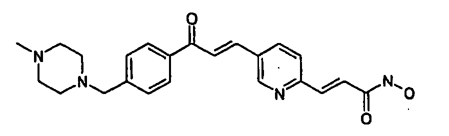

### STEP A

A solution of 1-(4-hydroxymethyl-phenyl)-ethanone (750 mg, 5 mmol) in EtOH (15 ml) was added dropwise at 0°C to a stirred solution of (E)-3-(5-formyl-pyridin-2-yl)-acrylic acid *tert*-butyl ester (described in Example 11 STEP A-D, 1.16 g, 5 mmol) and 1.7 M KOH (4.4 ml) in EtOH (10 ml). The mixture was stirred at 0°C for 5 h and the resulting precipitate was filtered and triturated with di-isopropylether to give 630 mg of desired (E)-3-{5-[(E)-3-(4-hydroxymethyl-phenyl)-3-oxo-propenyl]-pyridin-2-yl}-acrylic acid tert-butyl ester. The mother liquors were diluted with water and extracted with AcOEt. The organic layer was dried over Na₂SO₄ and evaporated *in vacuo.* The residue was triturated with EtOH to give additional 70 mg of (E)-3-{5-[(E)-3-(4-hydroxymethyl-phenyl)-3-oxo-propenyl]-pyridin-2-yl}-acrylic acid tert-butyl ester.
Y= 39%

### STEP B

A solution of (E)-3-{-[(E)-3-(4-hydroxymethyl-phenyl)-3-oxo-propenyl]-pyridin-2-yl}-acrylic acid tert-butyl ester (700 mg, 1.92 mmol) and TFA (2.9 ml) in DCM (10 ml) was stirred at room temperature for 3 h and then the solvent was evaporated *in vacuo.* The resulting yellow solid was treated with a solution of KOH (240 mg, 4.28 mmol) in EtOH (30 ml) for 30 min. The mixture was acidified with HCl/Et₂O and the solid was filtered with a buchner funnel. The powder was dissolved in DCM (10 ml), DMF (10 ml) and TEA (1.1 ml, 7.76 mmol), HOBT (524 mg, 3.88 mmol), EDC (741 mg, 3.88 mmol) and NH₂OTHP (227 mg, 1.94 mmol) were added. The solution was stirred at room temperature overnight and then diluted with water and extracted twice with DCM. The organic layer was washed with water, dried over Na₂SO₄ and evaporated *in vacuo.* The crude reaction mixture was purified by column chromatography (eluent: DCM/MeOH/NH₄OH 95:5:0.1) to give 230 mg of (E)-3-{5-[(E)-3-(4-hydroxymethyl-phenyl)-3-oxo-propenyl]-pyridin-2-yl}-N-(tetrahydro-pyran-2-yloxy)-acrylamide.
Y= 30%

### STEP C

Methanesulfonyl chloride (0.133 ml, 1.72 mmol) was added dropwise to a stirred solution of (E)-3-{5-[(E)-3-(4-hydroxymethyl-phenyl)-3-oxo-propenyl]-pyridin-2-yl}-N-(tetrahydro-pyran-2-yloxy)-acrylamide (230 mg, 0.56 mmol) and TEA (0.48 ml, 3.44 mmol) in DCM (2.5 ml) and DMF (2.5 ml). The resulting mixture was stirred at room temperature for 40 min. Then further methanesulfonyl chloride (0.067 ml, 0.86 mmol) and TEA (0.120 ml, 0.86 mmol) were added. After stirring for additional 1 h the solution was treated with 5% NaHCO₃ and extracted twice with DCM. The collected organic phases were washed with water, dried over Na₂SO₄ and evaporated *in vacuo.* The crude mixture was purified by column chromatography (eluent: DCM/MeOH/NH₄OH 97:3:0.1) to give 275 mg of a mixture of methanesulfonic acid 4-((E)-3-{6-[(E)-2-(tetrahydro-pyran-2-yloxycarbamoyl)-vinyl]-pyridin-3-yl}acryloyl)-benzyl ester and (E)-3-{5-[(E)-3-(4-chloromethyl-phenyl)-3-oxo-propenyl]-pyridin-2-yl}-N-(tetrahydro-pyran-2-yloxy)-acrylamide. 80 mg of this mixture were dissolved in CH₃CN (10 ml) and TEA (0.046 ml, 0.33 mmol) and N-methyl piperazine (0.022 ml, 0.198 mmol) were added. The solution was stirred at 80°C for 1 h and then at room temperature overnight. Further N-methyl piperazine (0.022 ml, 0.198 mmol) and TEA (0.046 ml, 0.33 mmol) were added and the mixture was stirred at 80°C for additional 1 h. The solvent was removed *in vacuo* and the residue was partitioned between water and AcOEt. The organic phase was dried over Na₂SO₄ and evaporated. The crude reaction mixture was purified by column chromatography (eluent: DCM/MeOH/NH₄OH 97:3:0.1) and the product was dissolved in DCM and treated with HCl/Et₂O for 2 h. The resulting precipitate was filtered and rinsed with DCM to give 14.5 mg of (E)-N-Hydroxy-3-(5-{(E)-3-[4-(4-methyl-piperazin-1-ylmethyl)-phenyl]-3-oxo-propenyl}-pyridin-2-yl)-acrylamide as its tris-hydrochloride salt.
Y= 3.3%
LC-MS: Method F, rt=1.07; (ES+) MH⁺: 407.26
1H NMR (DMSO-d₆ 353K +TFA) δ (ppm): 9.01 (d, 1 H), 8.31 (dd, 1 H), 8.14 (m, 2 H), 7.89 - 8.05 (m, 1 H), 7.75 (d, 1 H), 7.63 - 7.71 (m, 3 H), 7.52 (d, 1 H), 7.06 (d, 1 H), 4.05 (s, 2 H), 3.26 - 3.47 (m, 4 H), 2.97 - 3.21 (m, 4 H), 2.79 (s, 3 H).

### Example 51: (E)-3-(4-{(E)-3-[2-Chloro-5-(4-methyl-piperazin-1-yl)-phenyl]-3-oxo-propenyl}-phenyl)-N-hydroxy-acrylamide

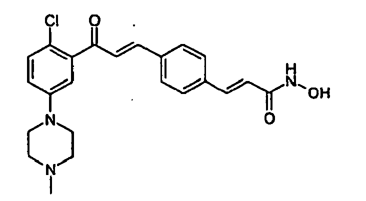

### STEP A

A mixture of 4-formylcinnamic acid (250 mg, 1.42 mmol), 1-[2-chloro-5-(4-methyl-piperazin-1-yl)-phenyl]-ethanone (obtained as described in Preparation 13, 358 mg, 1.42 mmol) and 1.7 M KOH (1.67 ml) in EtOH (10 ml) was stirred at 0°C for 6 h and then acidified with 10% HCl until reaching a pH value of 6. The resulting yellow precipitate was filtered and rinsed with EtOH and Et₂O to give 519 mg of (E)-3-(4-{(E)-3-[2-chloro-5-(4-methyl-piperazin-1-yl)-phenyl]-3-oxo-propenyl}-phenyl)-acrylic acid. The reaction product was used in the next step without any further purification.

### STEP B

A mixture of (E)-3-(4-{(E)-3-[2-chloro-5-(4-methyl-piperazin-1-yl)-phenyl]-3-oxo-propenyl}-phenyl)-acrylic acid (compound from STEP A, 205 mg), HOBT (134 mg, 0.99 mmol), EDC (190 mg, 0.99 mmol), TEA (0.138 ml, 0.99 mmol) and NH₂OTHP (70 mg, 0.60 mmol) in DMF (10 ml) was stirred at room temperature overnight. The reaction mixture was diluted with water and extracted with AcOEt twice. The organic phases were washed with water, brine and then dried over Na₂SO₄ and evaporated *in vacuo.* The crude mixture was purified by silica gel chromatography (eluent: DCM/MeOH/NH₄OH 95:5:0.2) and the resulting product was dissolved in DCM and treated with HCl/Et₂O for 3 h. The precipitate was filtered and rinsed with DCM and Et₂O to give 124 mg of (E)-3-(4-{(E)-3-[2-chloro-5-(4-methyl-piperazin-1-yl)-phenyl]-3-oxo-propenyl}-phenyl)-N-hydroxy-acrylamide hydrochloride.
LC-MS: Method F, rt=1.40; (ES+) MH⁺: 426.19
¹H NMR (DMSO-d₆ 353K) δ (ppm): 7.76 (m, 2 H), 7.61 (m, 2 H), 7.48 (d, 1 H), 7.45 (d, 1 H), 7.42 (d, 1 H), 7.22 (d, 1 H), 7.16 (dd, 1 H), 7.12 (d, 1 H), 6.62 (d, 1 H), 3.86 (m, 4 H), 3.22 (m, 4 H), 2.83 (s, 3 H).

### Example 52: (E)-3-(5-{(E)-3-[3-Chloro-5-(4-methyl-piperazin-1-yl)-phenyl]-3-oxo-propenyl}-pyridin-2-yl)-N-hydroxy-acrylamide

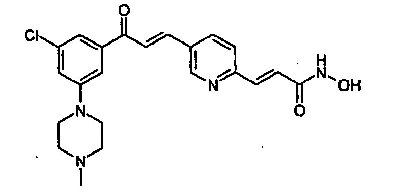

### STEP A

A solution of 1-[3-chloro-5-(4-methyl-piperazin-1-yl)-phenyl]-ethanone (obtained as described in Preparation 4, 360 mg, 1.43 mmol) in EtOH (20 ml) was added dropwise to a stirred solution of (E)-3-(5-formyl-pyridin-2-yl)-acrylic acid *tert*-butyl ester (described in Example 11 STEP A-D, 332 mg, 1.43 mmol) and 1.7 M KOH (0.85 ml) in EtOH (15 ml), cooled down to -15 °C. The mixture was stirred at -15 °C for 3 h and then partitioned between water and AcOEt. The organic layer was dried over Na₂SO₄ and evaporated *in vacuo.* The crude reaction mixture was purified by column chromatography (eluent: DCM/MeOH/NH₄OH 96:4:0.1) and the resulting product was dissolved in DCM (2 ml) and TFA (0.6 ml). The solution was stirred at room temperature for 4 h and then the solvent was removed *in vacuo* to give 192 mg of (E)-3-(5-{(E)-3-[3-chloro-5-(4-methyl-piperazin-1-yl)-phenyl]-3-oxo-propenyl}-pyridin-2-yl)-acrylic acid bis-trifluoroacetate. The crude mixture was used in the next step without any further purification.

### STEP B

A mixture of (E)-3-(5-{(E)-3-[3-chloro-5-(4-methyl-piperazin-1-yl)-phenyl]-3-oxo-propenyl}-pyridin-2-yl)-acrylic acid bis-trifluoroacetate (obtained in STEP A, 190 mg), HOBT (81 mg, 0.60 mmol), EDC (115 mg, 0.60 mmol), TEA (0.335 ml, 2.4 mmol) and NH₂OTH (52.6 mg, 0.45 mmol) in DCM (7 ml) was stirred at room temperature for 4 h. The solvent was evaporated and the residue was partitioned between water and AcOEt. The organic phase was dried over Na₂SO₄ and evaporated *in vacuo.* The crude mixture was purified by silica gel chromatography (eluent: DCM/MeOH/NH₄OH from 97:3:0.1 to 95:5:0.1) and the resulting product was dissolved in DCM and treated with HCl/Et₂O at room temperature for 3 h. The precipitate was filtered and triturated with isopropanol. The crude mixture was purified by preparative LC-MS to give 25 mg (E)-3-(5-{(E)-3-[3-chloro-5-(4-methyl-piperazin-1-yl)-phenyl]-3-oxo-propenyl}-pyridin-2-yl)-N-hydroxy-acrylamide as its bis-trifluoroacetate salt.
LC-MS: Method F, rt=1.83; (ES+) MH⁺: 427.13
¹H NMR (DMSO-d₆) δ (ppm): 9.72 (bs 1 H), 9.06 (d, 1 H), 8.42 (dd, 1 H), 8.06 (d,1 H), 7.80 (d, 1 H), 7.64 - 7.76 (m, 2 H), 7.47 - 7.64 (m, 2 H), 7.39 (t, 1 H), 7.01 (d, 1 H), 3.94 - 4.16 (m, 2 H), 3.40 - 3.65 (m, 2 H), 3.05 - 3.23 (m, 4 H), 2.88 (s, 3 H).

### Example 53: (E)-N-Hydroxy-3-(5-{(E)-3-[3-(4-methyl-piperazin-1-ylmethyl)-phenyl]-3-oxo-propenyl}-pyridin-2-yl)-acrylamide

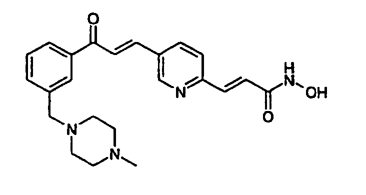

### STEP A

1.7 M KOH (0.634 ml) was added dropwise to a stirred mixture of (E)-3-(5-formyl-pyridin-2-yl)-acrylic acid *tert*-butyl ester (see Example 11 STEP A-D, 250 mg, 1.078 mmol) and 1-[3-(4-methyl-piperazin-1-ylmethyl)-phenyl]-ethanone (obtained as described in Preparation 14, 250 mg, 1.078 mmol) in EtOH (15 ml). The resulting solution was stirred at 0°C for 7 h and then diluted with water and extracted with AcOEt. The organic phase was dried over Na₂SO₄ and evaporated *in vacuo.* The crude product was purified by chromatographic column (eluent: DCM/MeOH/NH₄OH from 97:3:0.1 to 95:5:0.2) and the desired intermediate was dissolved in DCM (4 ml) and TFA (1 ml). The mixture was stirred at room temperature for 6 h and then the solvent was removed *in vacuo* to give 200 mg of (E)-3-(5-{(E)-3-[3-(4-methyl-piperazin-1-ylmethyl)-phenyl]-3-oxo-propenyl}-pyridin-2-yl)-acrylic acid as its tris-trifluoroacetate salt. The crude mixture was used in the next step without further purifications.

### STEP B

A mixture of (E)-3-(5-{(E)-3-[3-(4-methyl-piperazin-1-ylmethyl)-phenyl]-3-oxo-propenyl}-pyridin-2-yl)-acrylic acid tris-trifluoroacetate (crude compound from STEP A, 194 mg), HOBT (83 mg, 0.616 mmol), EDC (118 mg, 0.616 mmol), TEA (0.127 ml, 0.92 mmol) and NH₂OTHP (44 mg, 0.376 mmol) in DMF (10 ml) was stirred at room temperature for 5 h. The resulting solution was diluted with water and extracted with AcOEt and DCM. The collected organic phases were washed with brine and then dried over Na₂SO₄ and evaporated *in vacuo.* The crude mixture was purified by column chromatography (eluent: DCM/MeOH/NH₄OH from 97:3:0.1 to 95:5:0.2) and the resulting product was dissolved in DCM and treated with HCl/Et₂O for 3 h. The precipitate was filtered and purified by preparative LC-MS to give 9 mg of (E)-N-hydroxy-3-(5-{(E)-3-[3-(4-methyl-piperazin-1-ylmethyl)-phenyl]-3-oxo-propenyl}-pyridin-2-yl)-acrylamide as its tris-trifluoroacetate salt.
LC-MS: Waters Acquity UPLC, Micromass ZQ Single quadrupole (Waters). Column Acquity UPLC HSS T3, 2.1x100 mm, 1.8 µm;
flow rate: 0.6 ml/min splitting ratio MS: waste/1:4;
mobile phase: A phase= water/CH₃CN 95/5 + 0.1 % TFA; B phase= water/CH₃CN 5/95 +0.1% TFA. 0-0.5 min (A: 95%, B: 5%), 0.5-6 min (A: 0%, B: 100%), 6.00-7.00 min (A: 0%, B: 100%), 7.00-7.10 min (A: 95%, B: 5%); 7.10-8.50 min (A: 95%, B: 5%) UV detection wavelength 254 nm or BPI; Injection volume: 5µl
rt=2.00; (ES+) MH⁺: 407.21
¹H NMR (DMSO-d₆ 353K +TFA) δ (ppm): 9.00 (d, 1 H), 8.29 (dd, 1 H), 8.08 (dt, 1 H), 8.04 (t, 1 H), 7.94 (d, 1 H), 7.75 (d, 1 H), 7.62 - 7.69 (m, 2 H), 7.58 (t, 1 H), 7.52 (d, 1 H), 7.05 (d, 1 H), 3.79 (s, 2 H), 3.23 (bs, 4 H), 2.80 (s, 3 H), 2.68 - 2.85 (m, 4 H).

### 2. BIOLOGICAL TESTING

### Methods and results

### 2.1 Histone acetylation assay

In order to assess the ability of the compounds to modify histone acetylation levels, a dose-response study was carried out using the cell line K562 (derived from human lymphoma). Briefly, the cells were incubated with the compound for 3 hours, then fixed with 1% formaldehyde in PBS and permeabilized with a solution containing 0.1% Triton X-100 in PBS. After washing, the cells were pre-incubated with 10% goat serum in PBS for 30 minutes at 4°C, exposed for 1 hour at room temperature to a monoclonal antibody against acetylated histones and then incubated for one hour with a secondary antibody conjugated with FITC. Histone acetylation levels were measured by cytofluorometry (FACS) (Ronzoni, S. et al. Cytometry A. 2005, 66, 52-61).

The compounds of the present invention showed a remarkable histone deacetylase inhibitory activity (calculated on increase in acetylation) at low micromolar concentrations or even below.

### 2.2 Assay of enzyme inhibition of HDAC

The *in-vitro* activity of HDAC inhibitors was assayed by means of a biochemical assay using a BIOMOL Kit, according to the recommended experimental conditions. In a first step, 5 µg of a nuclear extract of HeLa cells were added to a solution containing the HDAC inhibitor and the substrate (lysine with acetylated amino on the side chain) at a concentration of 116 µM. The samples were incubated for 10 minutes at room temperature and then exposed to a developer (15 minutes at room temperature). In this last step a fluorophore was produced, whose fluorescence was measured using an excitation wavelength of 355 nm and an emission at 460 nm.

The obtained results are illustrated in the following tables 4-6, wherein the compounds of the invention are grouped together depending on their belonging to formula (**Ia**), (**Ib**) or (**Ic**). In tables 4-6, the reference HDAC inhibitors marked with (*) are those disclosed in the patent application PCT/EP2005/054949.

As evident from comparison with the reference HDAC inhibitors of the prior art, the compounds of the invention showed a significant enhancement of activity.

**Table 4: Compounds of formula (Ia)**

| Example no. | Mol. structure | Activity IC₅₀[µM] |
|---|---|---|
| 26 | 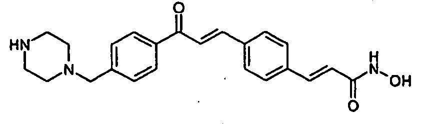 | 0.0267 |
| 30 | 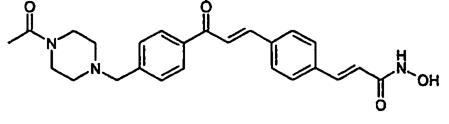 | 0.025 |
| Ref (*) 48 | 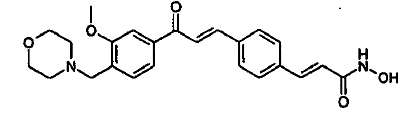 | 0.0733 |

**Table 5: Compounds of formula (Ib)**

| Example no. | Mol. structure | Activity IC₅₀[µM] |
|---|---|---|
| 25 | 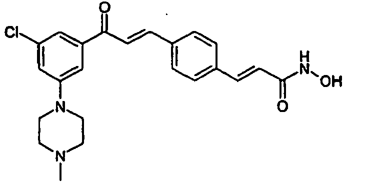 | 0.035 |
| Ref (*) 29 | 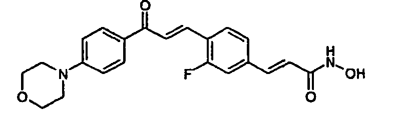 | 0.2825 |
| Ref (*) 41 | 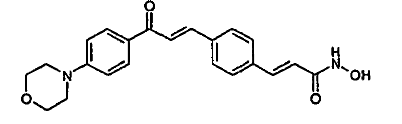 | 0.2375 |
| Ref (*) 59 | 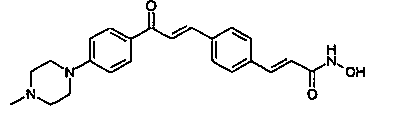 | 1.995 |

**Table 6: Compounds of formula (Ic)**

| Example no. | Mol. structure | Activity IC₅₀[µM] |
|---|---|---|
| 39 | 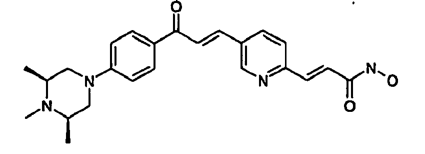 | 0.0061 |
| 42 | 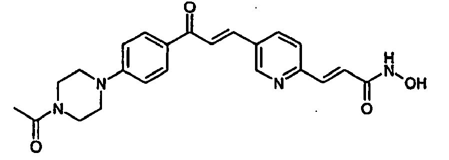 | 0.0048 |
| 43 | 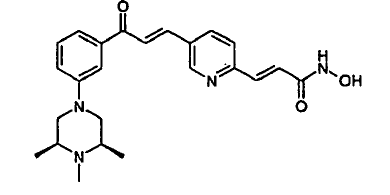 | 0.0040 |
| 44 | 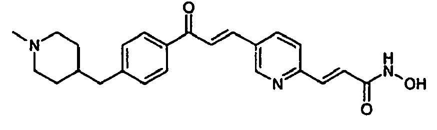 | 0.0013 |
| 45 | 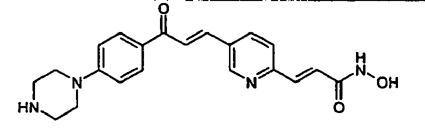 | 0.0007 |
| 47 | 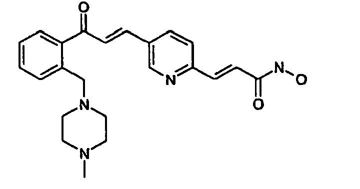 | 0.0118 |
| Ref (*) 69 | 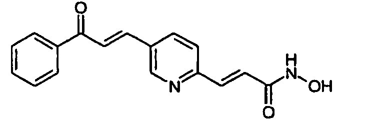 | 0.0225 |
| Ref (*) 71 | 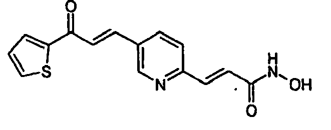 | 0.0675 |
| Ref (*) 72 | 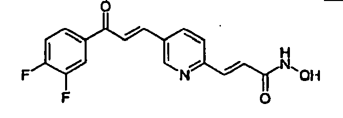 | 0.0200 |

### 2.3 Cell growth: MTT assay

The MTT [3-(4,5-dimethylthiazolyl)-2,5-diphenyltetrazolium bromide] test is a colorimetric test able to measure cell viability and proliferation, based on the capacity of cells to metabolise tetrazolium salt to form formazan crystals, by means of a mitochondrial dehydrogenase. The cells in exponential growth phase are exposed to the inhibitors. The activity of the mitochondrial dehydrogenase and the quantity of formazan salts produced are proportional to the number of survived cells. The quantity of formazan produced is detected by UV-VIS spectrophotometry.

K562 cells were incubated for 72 hours with different concentrations of the inhibitors. 5 mg/ml of MTT in PBS were added at different time points and the solution was incubated for 3-4 hours at 37°C. The supernatant was then removed and the formazan crystals were dissolved in a mixture of DMSO and absolute ethanol (1:1, v:v) and the solution analysed with a spectrophotometer at a wavelength between 550 and 570 nm. The IC₅₀ is calculated using GraphPad Software.

### 2.4 Cell cycle and apoptosis

A suspension of K562 or HT29 cells was treated with increasing amounts of HDAC inhibitors in order to assess the biological response. In order to establish the effect of the HDAC inhibitors on the cell cycle and apoptosis the cells were fixed in 70% ethanol for 30 minutes, re-suspended in propidium iodide (PI: 50 µg/ml) with added RNAse (250 µg/ml) and incubated for 24 hours at room temperature. The samples were analysed using a FACScan Cytometer (Beckton Dickinson). The tested compounds were able to determine a clear cell cycle modification and to induce apoptosis evaluated as sub-G1 analysis.

### 2.5 Metabolic stability in hepatic microsomes

### Experimental procedure

The test compound was dissolved in DMSO at the final concentration of 1 µM and pre-incubated for 10 min at 37°C in potassium phosphate buffer pH 7.4 together with mouse or human hepatic microsomes (Xenotech) at the final concentration of 0.5 mg/ml.

After the pre-incubation the reaction was started by adding the cofactor mixture (NADP, G6P, G6P-DH); aliquots were taken at time 0 and 30 min, added to acetonitrile in order to stop the reaction. After centrifugation the supernatant was separated and analyzed by LC-MS/MS.

A control sample without cofactor was always studied in parallel in order to check the chemical stability of the test compound.

Two reference compounds of known metabolic stability 7-ethoxycoumarin and propranolol were present each time to access the validity of the experiment.

A fixed concentration of verapamil was added in each sample as internal standard for the LC-MS/MS analysis.

### Data analysis

The percentage of the compound remaining after 30 min incubation period was calculated according the following equation: [area at time 30 min]/ [area at time 0 min]*100%.

### Sample analysis

### HPLC conditions

Samples were analyzed on an Acquity UPLC (Waters) coupled with a Sample Organizer and interfaced with a triple quadrupole Premiere XE (Waters). Eluents were:
Phase A: 95% H₂O, 5% CH₃CN + 0.1% HCOOH
Phase B: 5% H₂O, 95% CH₃CN + 0.1% HCOOH
Column: Acquity BEH C18 50x2.1 mm 1.7 µm at 40°C. Flow 0.45 ml/min, alternatively Acquity BEH C18 50x1 mm 1.7 µm at 40°C. Flow 0.2 ml/min, Chromatographic method is reported below.

**Table 7 Chromatographic method**

| **Time (min)** | **%A** | **%B** |
|---|---|---|
| 0 | 98 | 2 |
| 0.2 | 98 | 2 |
| 0.21 | 0 | 100 |
| 1.5 | 0 | 100 |
| 1.6 | 98 | 2 |
| 2 | 98 | 2 |

### MS method

Samples were analyzed in MRM (Multiple Reaction Monitoring) ESI Pos mode. MS Conditions: Capillary Voltage 3.4kV, Source Temp. 115°C, Desolvation Temp. 450°C, Desolvation gas 900 l/h, Cell Pressure 3.3 10⁻³ mbar.

Cone Voltage and Collision Energy were optimized for each compound. The acquisition of each compound was performed together with the internal standard verapamil.

The obtained results are illustrated in the following tables 8-10, wherein the compounds of the invention are grouped together depending on their belonging to formula (Ia), (Ib) or (Ic). The reference HDAC inhibitors marked with (*) are those disclosed in the patent application PCT/EP2005/054949.

As evident from comparison with the reference HDAC inhibitors of the prior art, the compounds of the invention showed a significant enhancement in metabolic stability.

**Table 8: Compounds of formula (Ia)**

| Example no. | Mol. structure | Met. mouse | Met. human |
|---|---|---|---|
| 7 | 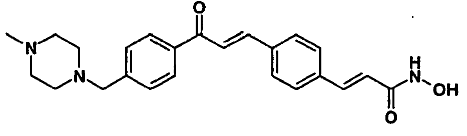 | 16.75 | 61.16 |
| 26 | 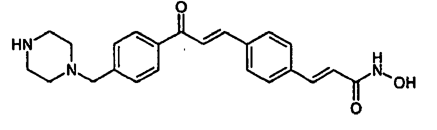 | 42.41 | 50.84 |
| Ref (*) 48 | 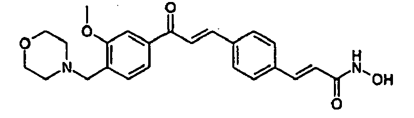 | 2.8 | 35.28 |

**Table 9: Compounds of formula (Ib)**

| Example no. | Mol. structure | Met. mouse | Met. human |
|---|---|---|---|
| 1 | 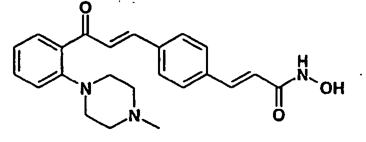 | 33.81 | 38.16 |
| 4 | 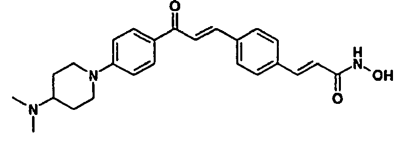 | 14.00 | 41.88 |
| 15 | 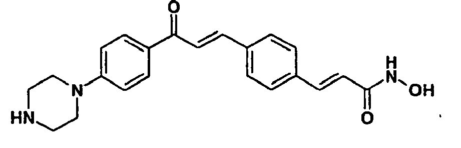 | 30.5 | 42.58 |
| 25 | 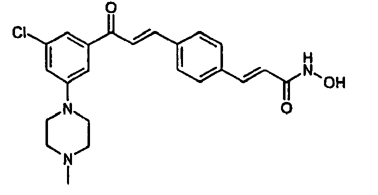 | 84.89 | 57.28 |
| Ref (*) 29 | 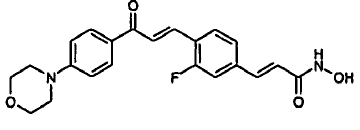 | 11.17 | 20.86 |
| Ref (*) 41 | 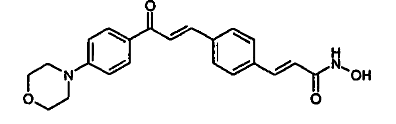 | 2.76 | 8.66 |
| Ref (*) 59 | 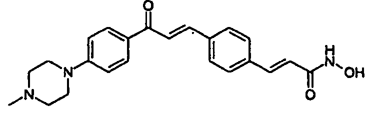 | 9.12 | 33.29 |

**Table 10: Compounds of formula (Ic)**

| Example no. | Mol. structure | Met. mouse | Met. human |
|---|---|---|---|
| 39 | 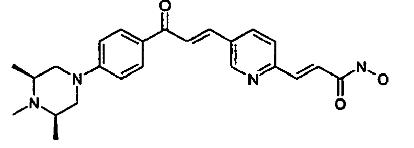 | 20.29 | 44.35 |
| 42 | 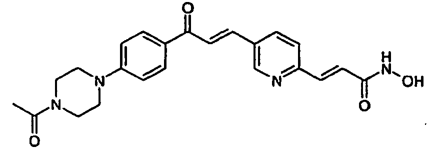 | 50.47 | 35.27 |
| 43 | 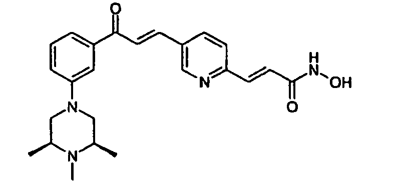 | 42.45 | 37.29 |
| 44 | 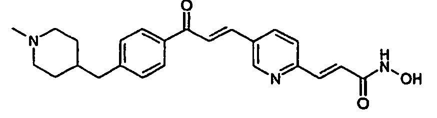 | 54.56 | 34.38 |
| 45 | 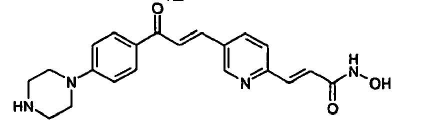 | 43.41 | 53.65 |
| 47 | 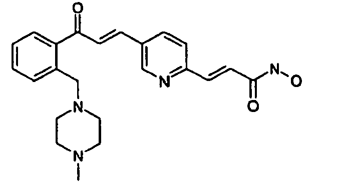 | 52.66 | 93.48 |
| Ref (*) 69 | 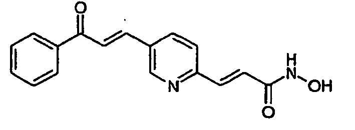 | 5.65 | 13.74 |
| Ref (*) 71 | 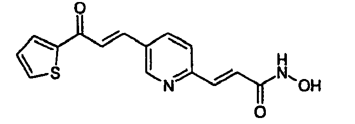 | 6.04 | 21.82 |
| Ref (*) 72 | 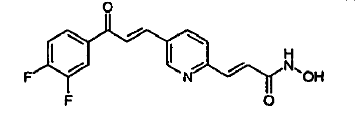 | 10.83 | 15.32 |

## Claims

1. A compound of formula (**I**) 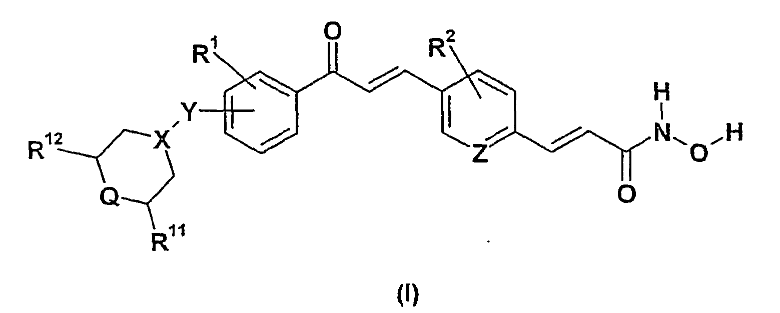 wherein:
Q is a bond, CH₂, CH-NR³R⁴, NR⁵ or oxygen;
X is CH or nitrogen;
Y is a bond, CH₂, oxygen or NR⁶;
Z is CH or nitrogen;
R¹, R² are, independently, hydrogen, halogen, C₁-C₆ alkyl, or C₁-C₆ haloalkyl;
R³, R⁴ are, independently, hydrogen, C₁-C₆ alkyl, phenyl or benzyl;
R⁵ is hydrogen, C₁-C₆ alkyl, (CO)R⁷, SO₂-C₁-C₆ alkyl, phenyl or benzyl;
R⁶ is hydrogen, C₁-C₆ alkyl or benzyl;
R⁷ is hydrogen, C₁-C₆ alkyl, phenyl, benzyl, OR⁸ or NR⁹R¹⁰;
R⁸ is C₁-C₆ alkyl;
R⁹ is hydrogen, C₁-C₆ alkyl, phenyl or benzyl;
R¹⁰ is hydrogen, C₁-C₆ alkyl or benzyl;
R¹¹, R¹² are, independently, hydrogen or C₁-C₆ alkyl;
and the pharmaceutically acceptable salts thereof;
with the proviso that when X is nitrogen, Y cannot be oxygen or NR⁶;
and with the exclusion of the following compounds:
(E)-N-Hydroxy-3-(4-{(E)-3-[4-(4-methyl-piperazin-1-yl)-phenyl]-3-oxo-propenyl}-phenyl)-acrylamide;
(E)-N-Hydroxy-3-{4-[(E)-3-(4-morpholin-4-yl-phenyl)-3-oxo-propenyl]-phenyl}-acrylamide;
(E)-3-{3-Fluoro-4-[(E)-3-(4-morpholin-4-yl-phenyl)-3-oxo-propenyl]-phenyl}-N-hydroxy-acrylamide.

2. Compound according to claim 1, wherein one or more of the aforesaid alkyls is a C₁-C₄ alkyl group.

3. Compound according to claim 1, wherein:
**R¹, R²** are, independently, hydrogen, fluorine, chlorine, C₁-C₂ alkyl, or CF₃;
**R³, R⁴** are, independently, hydrogen, C₁-C₂ alkyl, phenyl or benzyl;
**R⁵** is hydrogen, C₁-C₂ alkyl, (CO)R⁷, SO₂-C₁-C₂ alkyl, phenyl or benzyl;
**R⁶** is hydrogen, C₁-C₂ alkyl or benzyl;
**R⁷** is hydrogen, C₁-C₂ alkyl, phenyl, benzyl, O**R⁸** or N**R⁹R¹⁰**;
**R⁸** is C₁-C₂ alkyl;
**R⁹** is hydrogen, C₁-C₂ alkyl, phenyl or benzyl;
**R¹⁰** is hydrogen, C₁-C₂ alkyl or benzyl;
**R¹¹, R¹²** are, independently, hydrogen or C₁-C₂ alkyl;

4. Compound according to claim 1, having formula (**Ia**), 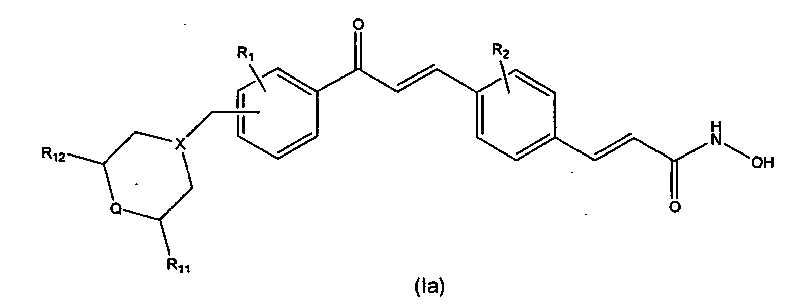 wherein:
**Q** is CH₂, CH-NR³**R⁴**, or N**R⁵**;
**X** is CH or nitrogen;
**R¹, R²** are, independently, hydrogen, halogen, C₁-C₄ alkyl, or C₁-C₄ haloalkyl;
**R³, R⁴** are, independently, hydrogen or C₁-C₄ alkyl;
**R⁵** is hydrogen, C₁-C₄ alkyl, (CO)R⁷, phenyl or benzyl;
**R⁷** is hydrogen, C₁-C₆ alkyl, phenyl, benzyl, O**R⁸** or N**R⁹R¹⁰**;
**R⁸** is C₁-C₄ alkyl;
**R⁹, R¹⁰** are, independently, hydrogen or C₁-C₄ alkyl;
**R¹¹, R¹²** are, independently, hydrogen or C₁-C₄ alkyl.

5. Compound according to claim 4, wherein:
**Q** is CH₂, CH-N**R³R⁴**, or N**R⁵**;
**X** is CH or nitrogen;
**R¹, R²** are, independently, hydrogen, fluoro, chloro, or CF₃;
**R³, R⁴** are, independently, hydrogen or C₁-C₂ alkyl;
**R⁵** is hydrogen, C₁-C₄ alkyl, (CO)R⁷, phenyl or benzyl;
**R⁷** is hydrogen, C₁-C₄ alkyl, phenyl, benzyl, O**R⁸** or N**R⁹R¹⁰**;
**R⁸** is C₁-C₄ alkyl;
**R⁹, R¹⁰** are, independently, hydrogen or C₁-C₂ alkyl;
**R¹¹, R¹²** are, independently, hydrogen or C₁-C₂ alkyl.

6. Compound according to claim 4, wherein:
**Q** is N**R⁵**;
**X** is nitrogen;
**R¹, R²** are, independently, hydrogen, fluoro, chloro or CF₃;
**R⁵** is hydrogen, C₁-C₄ alkyl, (CO)R⁷, phenyl or benzyl;
**R⁷** is hydrogen, C₁-C₄ alkyl, phenyl, benzyl, O**R⁸** or N**R⁹R¹⁰**;
**R8** is C₁-C₄ alkyl;
**R⁹, R¹⁰** are, independently, hydrogen or C₁-C₂ alkyl;
**R¹¹, R¹²** are, independently, hydrogen or C₁-C₂ alkyl.

7. Compound according to claim 4, selected from:
(E)-N-Hydroxy-3-(4-{(E)-3-[4-(4-methyl-piperazin-1-yl-methyl)-phenyl]-3-oxo-propenyl}-phenyl)-acrylamide;
(E)-3-(4-{(E)-3-[4-(4-Dimethylamino-piperidin-1-yl-methyl)-phenyl]-3-oxo-propenyl}-phenyl)-N-hydroxy-acrylamide;
(E)-N-Hydroxy-3-(4-{(E)-3-[4-(1-methyl-piperidin-4-ylmethyl)-phenyl]-3-oxo-propenyl}-phenyl)-acrylamide;
(E)-N-Hydroxy-3-{4-[(E)-3-oxo-3-(4-piperazin-1-ylmethyl-phenyl)-propenyl]-phenyl}-acrylamide;
(E)-3-(4-{(E)-3-[4-(4-Benzyl-piperazin-1-ylmethyl)-phenyl]-3-oxo-propenyl}-phenyl)-N-hydroxy-acrylamide;
(E)-3-(4-{(E)-3-[4-((3R,5S)-3,5-Dimethyl-piperazin-1-ylmethyl)-phenyl]-3-oxo-propenyl}-phenyl)-N-hydroxy-acrylamide;
(E)-3-(4-{(E)-3-[4-(4-Acetyl-piperazin-1-ylmethyl)-phenyl]-3-oxo-propenyl}-phenyl)-N-hydroxy-acrylamide;
(E)-3-(4-{(E)-3-[4-((3R,5S)-4-Acetyl-3,5-dimethyl-piperazin-1-ylmethyl)-phenyl]-3-oxo-propenyl}-phenyl)-N-hydroxy-acrylamide;
(E)-3-(4-{(E)-3-[4-(4-Ethyl-piperazin-1-ylmethyl)-phenyl]-3-oxo-propenyl}-phenyl)-N-hydroxy-acrylamide;
(E)-N-Hydroxy-3-(4-{(E)-3-[3-(4-methyl-piperazin-1-ylmethyl)-phenyl]-3-oxo-propenyl}-phenyl)-acrylamide;
(E)-N-Hydroxy-3-(4-{(E)-3-[2-(4-methyl-piperazin-1-ylmethyl)-phenyl]-3-oxo-propenyl}-phenyl)-acrylamide.

8. Compound according to claim 1, having formula (Ib) 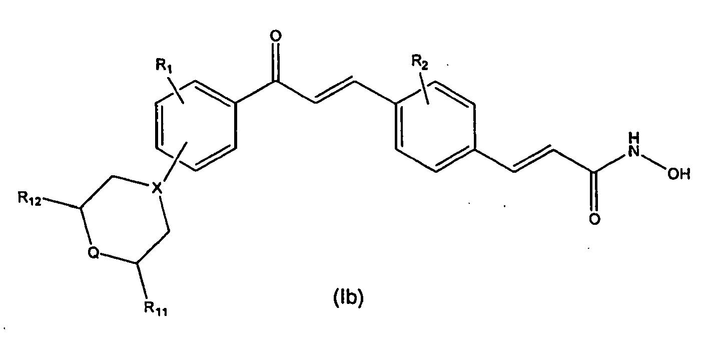 wherein:
**Q** is CH₂, CH-N**R³R⁴**, or N**R⁵**;
**X** is CH or nitrogen;
**R¹, R²** are, independently, hydrogen, halogen, C₁-C₄ alkyl or C₁-C₄ haloalkyl;
**R³, R⁴** are, independently, hydrogen or C₁-C₄ alkyl;
**R⁵** is hydrogen, C₁-C₄ alkyl, (CO)R⁷, SO₂-C₁-C₄ alkyl, phenyl or benzyl;
**R⁷** is hydrogen, C₁-C₆ alkyl, phenyl, benzyl, O**R⁸** or N**R⁹R¹⁰**;
**R⁸** is C₁-C₄ alkyl;
**R⁹, R¹⁰** are, independently, hydrogen or C₁-C₄ alkyl;
**R¹¹, R¹²** are, independently, hydrogen or C₁-C₄ alkyl;
with the exclusion of the following compound:
(E)-N-Hydroxy-3-(4-{(E)-3-[4-(4-methyl-piperazin-1-yl)-phenyl]-3-oxo-propenyl}-phenyl)-acrylamide.

9. Compound according to claim 8, wherein, within said formula **(Ib)**, the group: 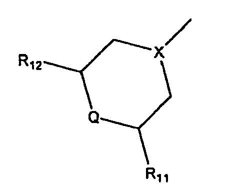 is in ortho or meta position with respect to the 3-oxo-propenyl moiety, and:
**Q** is CH₂ or N**R⁵**;
**X** is CH or nitrogen;
**R¹, R²** are, independently, hydrogen, fluoro, chloro or CF₃;
**R⁵** is hydrogen, C₁-C₄ alkyl, (CO)**R⁷**, phenyl or benzyl;
**R⁷** is hydrogen, C₁-C₄ alkyl, phenyl, benzyl, O**R⁸** or N**R⁹R¹⁰**;
**R⁸** is C₁-C₄ alkyl;
**R⁹, R¹⁰** are, independently, hydrogen or C₁-C₂ alkyl;
**R¹¹, R¹²** are, independently, hydrogen or C₁-C₂ alkyl.

10. Compound according to claim 8, selected from:
(E)-3-(4-{(E)-3-[5-Chloro-2-(4-methyl-piperazin-1-yl)-phenyl]-3-oxo-propenyl}-phenyl)-N-hydroxy-acrylamide;
(E)-N-Hydroxy-3-(4-{(E)-3-[2-(4-methyl-piperazin-1-yl)-phenyl]-3-oxo-propenyl}-phenyl)-acrylamide;
(E)-N-Hydroxy-3-(4-{(E)-3-[3-(4-methyl-piperazin-1-yl)-phenyl]-3-oxo-propenyl}-phenyl)-acrylamide;
(E)-N-Hydroxy-3-(4-{(E)-3-[4-(4-methylamino-piperidin-1-yl)-phenyl]-3-oxo-propenyl}-phenyl)-acrylamide;
(E)-3-(4-{(E)-3-[4-(4-Dimethylamino-piperidin-1-yl)-phenyl]-3-oxo-propenyl}-phenyl)-N-hydroxy-acrylamide;
(E)-N-Hydroxy-3-(4-{(E)-3-[4-(1-methyl-piperidin-4-yl)-phenyl]-3-oxo-propenyl}-phenyl)-acrylamide;
(E)-N-Hydroxy-3-(4-{(E)-3-[4-(4-isobutyl-piperazin-1-yl)-phenyl]-3-oxo-propenyl}-phenyl)-acrylamide;
(E)-3-(4-{(E)-3-[4-(4-Ethyl-piperazin-1-yl)-phenyl]-3-oxo-propenyl}-phenyl)-N-hydroxy-acrylamide;
(E)-3-(4-{(E)-3-[4-(4-Benzyl-piperazin-1-yl)-phenyl]-3-oxo-propenyl}-phenyl)-N-hydroxy-acrylamide;
(E)-N-Hydroxy-3-{4-[(E)-3-(4-piperazin-1-yl-phenyl)-3-oxo-propenyl]-phenyl}-acrylamide;
(E)-3-(4-{(E)-3-[4-(4-Benzoyl-piperazin-1-yl)-phenyl]-3-oxo-propenyl}-phenyl)-N-hydroxy-acrylamide;
(E)-3-(4-{(E)-3-[4-(4-Acetyl-piperazin-1-yl)-phenyl]-3-oxo-propenyl}-phenyl)-N-hydroxy-acrylamide;
(E)-N-Hydroxy-3-(4-{(E)-3-[4-(4-methanesulfonyl-piperazin-1-yl)-phenyl]-3-oxo-propenyl}-phenyl)-acrylamide;
4-(4-{(E)-3-[4-((E)-2-Hydroxycarbamoyl-vinyl)-phenyl]-acryloyl}-pheny)-piperazine-1-carboxylic acid dimethylamide;
4-(4-{(E)-3-[4-((E)-2-Hydroxycarbamoyl-vinyl)-phenyl]-acryloyl}-phenyl)-piperazine-1-carboxylic acid amide;
4-(4-{(E)-3-[4-((E)-2-Hydroxycarbamoyl-vinyl)-phenyl]-acryloyl}-phenyl)-piperazine carboxylic acid ethyl ester;
(E)-N-Hydroxy-3-(4-{(E)-3-oxo-3-[4-((3R,5S)-3,4,5-trimethyl-piperazin-1-yl)-phenyl]-propenyl}-phenyl)-acrylamide;
(E)-3-(4-{(E)-3-[3-Chloro-5-(4-methyl-piperazin-1-yl)-phenyl]-3-oxo-propenyl}-phenyl)-N-hydroxy-acylamide;
(E)-3-(4-{(E)-3-[2-Chloro-5-(4-methyl-piperazin-1-yl)-phenyl]-3-oxo-propenyl}-phenyl)-N-hydroxy-acrylamide.

11. Compound according to claim 1, having formula **(Ic)** 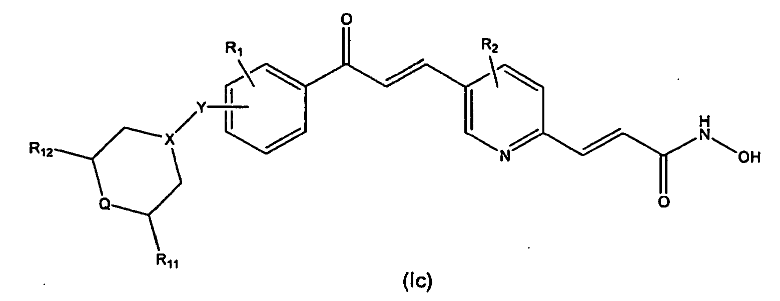 wherein:
**Q** is CH₂, CH-N**R³R⁴**, N**R⁶** or oxygen;
**X** is CH or nitrogen;
**Y** is a bond, CH₂, oxygen or N**R⁶**;
**R¹, R²** are, independently, hydrogen, halogen, C₁-C₄ alkyl, or C₁-C₄ haloalkyl;
**R³, R⁴** are, independently, hydrogen or C₁-C₄ alkyl;
**R⁵** is hydrogen, C₁-C₄ alkyl, (CO)R⁷, phenyl or benzyl;
**R⁶** is hydrogen or C₁-C₄ alkyl;
**R⁷** is hydrogen, C₁-C₆ alkyl, phenyl, benzyl, OR⁸ or N**R⁹R¹⁰**;
**R⁸** is C₁-C₄ alkyl;
**R⁹, R¹⁰** are, independently, hydrogen or C₁-C₄ alkyl;
**R¹¹, R¹²** are, independently, hydrogen or C₁-C₄ alkyl;
provided that when **X** is nitrogen, **Y** cannot be oxygen or N**R⁶**.

12. Compound according to claim 11, wherein:
**Q** is CH₂, N**R⁵** or oxygen;
**X** is CH or nitrogen;
**Y** is a bond or CH₂;
**R¹, R²** are, independently, hydrogen, fluoro, chloro or CF₃;
**R⁵** is hydrogen, C₁-C₂ alkyl, (CO)R⁷, phenyl or benzyl;
**R⁷** is hydrogen, C₁-C₄ alkyl, phenyl, benzyl, O**R⁸** or N**R⁹R¹⁰**;
**R⁸** is C₁-C₄ alkyl;
**R⁹, R¹⁰** are, independently, hydrogen or C₁-C₂ alkyl;
**R¹¹, R¹²** are, independently, hydrogen or C₁-C₂ alkyl.

13. Compound according to claim 11, selected from:
(E)-N-Hydroxy-3-(5-{(E)-3-[4-(4-methyl-piperazin-1-yl)-phenyl]-3-oxo-propenyl}-pyridin-2-yl)-acrylamide;
(E)-N-Hydroxy-3-(5-{(E)-3-[2-(4-methyl-piperazin-1-yl)-phenyl]-3-oxo-propenyl}-pyridin-2-yl)-acrylamide;
(E)-N-Hydroxy-3-(5-{(E)-3-[3-(4-methyl-piperazin-1-yl)-phenyl]-3-oxo-propenyl}-pyridin-2-yl)-acrylamide;
(E)-3-(5-{(E)-3-[4-(4-Benzyl-piperazin-1-yl)-phenyl]-3-oxo-propenyl}-pyridin-2-yl)-N-hydroxy-acrylamide;
(E)-N-Hydroxy-3-(5-{(E)-3-oxo-3-[4-((3R,5S)-3,4,5-trimethyl-piperazin-1-yl)-phenyl]-propenyl}-pyridin-2-yl)-acrylamide;
(E)-N-Hydroxy-3-{5-[(E)-3-(4-morpholin-4-ylmethyl-phenyl)-3-oxo-propenyl]-pyridin-2-yl}-acrylamide;
(E)-3-(5-{(E)-3-[4-(4-Ethyl-piperazin-1-yl)-phenyl]-3-oxo-propenyl}-pyridin-2-yl)-N-hydroxy-acrylamide;
(E)-3-(5-{(E)-3-[4-(4-Acetyl-piperazin-1-yl)-phenyl]-3-oxo-propenyl}-pyridin-2-yl)-N-hydroxy-acrylamide;
(E)-N-Hydroxy-3-(5-{(E)-3-oxo-3-[3-((3R,5S)-3,4,5-trimethyl-piperazin-1-yl)-phenyl]-propenyl}-pyridin-2-yl)-acrylamide;
(E)-N-Hydroxy-3-(5-{(E)-3-[4-(1-methyl-piperidin-4-ylmethyl)-phenyl]-3-oxo-propenyl}-pyridin-2-yl)-acrylamide;
(E)-N-Hydroxy-3-{5-[(E)-3-oxo-3-(4-piperazin-1-yl-phenyl)-propenyl]- pyridin-2-yl}-acrylamide;
(E)-N-Hydroxy-3-(5-{(E)-3-[2-(4-methyl-piperazin-1-ylmethyl)-phenyl]-3-oxo-propenyl}-pyridin-2-yl)-acrylamide;
(E)-N-Hydroxy-3-{5-[(E)-3-oxo-3-(4-piperazin-1-ylmethyl-phenyl)-propenyl]- pyridin-2-yl}-acrylamide;
(E)-3-(5-{(E)-3-[4-(4-Acetyl-piperazin-1-ylmethyl)-phenyl]-3-oxo-propenyl}-pyridin-2-yl)-N-hydroxy-acrylamide;
(E)-N-Hydroxy-3-(5-{(E)-3-[4-(4-methyl-piperazin-1-ylmethyl)-phenyl]-3-oxo-propenyl}-pyridin-2-yl)-acrylamide;
(E)-3-(5-{(E)-3-[3-Chloro-5-(4-methyl-piperazin-1-yl)-phenyl]-3-oxo-propenyl}-pyridin-2-yl)-N-hydroxy-acrylamide;
(E)-N-Hydroxy-3-(5-{(E)-3-[3-(4-methyl-piperazin-1-ylmethyl)-phenyl]-3-oxo-propenyl}-pyridin-2-yl)-acrylamide.

14. A process for obtaining the compounds of formula (**I**) as defined in claims 1-13, **characterized by** treating a compound of formula **(II)** 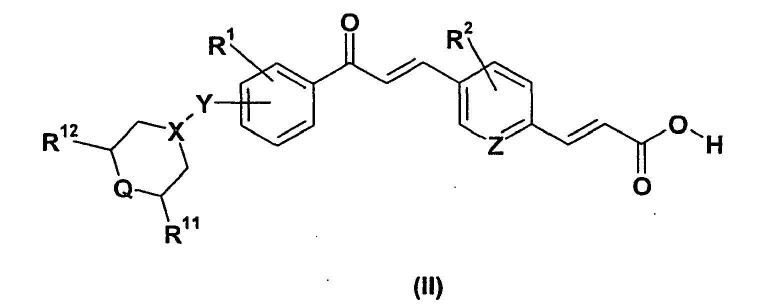 where **Q, X, Y, Z, R¹, R², R¹¹** and **R¹²** have the same meanings as described for formula (I), with a protected hydroxylamine, followed by a hydroxylamine deprotection step.

15. A process according to claim 14, wherein the compound of formula (**II**) is obtained from a compound of formula (**V**) or from a compound of formula (**XI**) 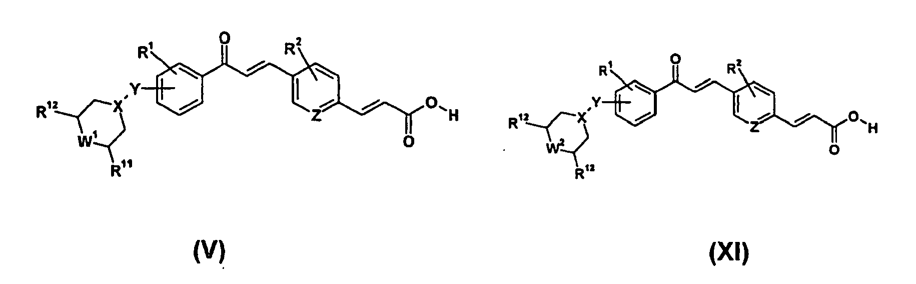 where **X, Y, R¹, R², R¹¹, R¹²** and **Z** have the same meanings as indicated for formula (I) and **W¹** is NH and **W²** is CO, treated with reagents able to convert the **W¹** or **W²** group into **Q**, as defined in formula (**I**).

16. A process according to claim 14, wherein the compound of formula (**II**) is obtained by reacting a compound of formula (**IV**) with a compound of formula (**III**), 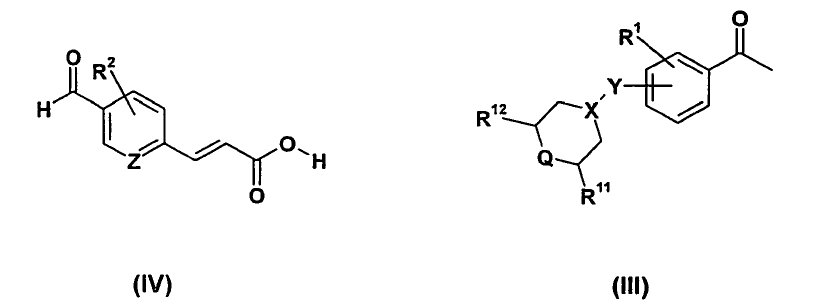 where **Q, X, Y, R¹, R², R¹¹, R¹²** and **Z** have the same meanings as indicated for formula (I).

17. A compound of formula (**I**), (**Ia**), (**Ib**), (**Ic**) as defined in claims 1-13, for use in therapy.

18. A pharmaceutical composition comprising one or more compounds of formula (**I**), (**Ia**), (**Ib**); (**Ic**) as defined in claims 1-13, in association with pharmaceutically acceptable excipients.

19. A composition according to claim 18 in the form of a tablet, capsule, pill, oral preparation, powder, granular preparation, injectable or infusible solution or suspension, suppository, aqueous or oily suspension, solution, emulsion, syrup, elixir, cream, ointment, paste, gel, solution, oil or lotion, membrane or medicated patch.

20. Use of one or more compounds of formula (**I**), (**Ia**), (**Ib**), (**Ic**) as defined in claims 1-13, in the preparation of a medicament for preventing and/or treating diseases linked to the disregulation of histone deacetylase activity.

21. Use according to claim 20, wherein said disease is cancer.

## Patentansprüche

1. Eine Verbindung der Formel (I) 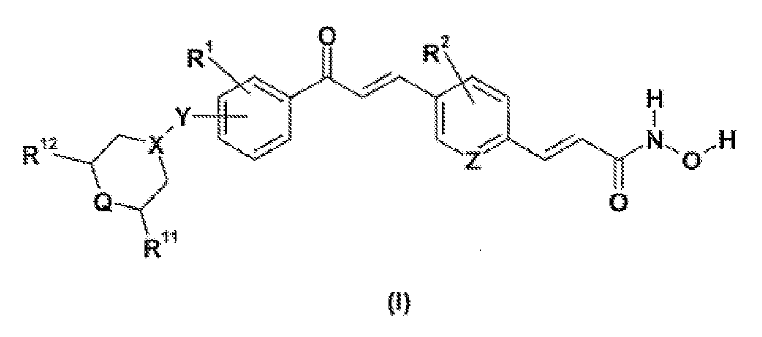 worin
Q für eine Bindung, CH₂, CH-NR³R⁴, NR⁵ oder Sauerstoff steht;
X für CH oder Stickstoff steht;
Y für eine Bindung, CH₂, Sauerstoff oder NR⁶ steht;
Z für CH oder Stickstoff steht;
R¹, R² unabhängig voneinander für Wasserstoff, Halogen, C₁- bis C₆-Alkyl oder C₁- bis C₆-Haloalkyl stehen;
R³, R⁴ unabhängig voneinander für Wasserstoff, C₁- bis C₆-Alkyl, Phenyl oder Benzyl stehen;
R⁵ für Wasserstoff, C₁- bis C₆-Alkyl, (CO)R⁷, SO₂-C₁- bis C₆-Alkyl, Phenyl oder Benzyl steht;
R⁶ für Wasserstoff, C₁- bis C₆-Alkyl oder Benzyl steht;
R⁷ für Wasserstoff, C₁- bis C₆-Alkyl, Phenyl, Benzyl, OR⁸ oder NR⁹R¹⁰ steht;
R⁸ für C₁- bis C₆-Alkyl steht;
R⁹ für Wasserstoff, C₁- bis C₆-Alkyl, Phenyl oder Benzyl steht;
R¹⁰ für Wasserstoff, C₁- bis C₆-Alkyl oder Benzyl steht;
R¹¹, R¹² unabhängig voneinander für Wasserstoff oder C₁- bis C₆-Alkyl stehen; und die pharmazeutisch annehmbaren Salze davon;
mit der Maßgabe, dass dann, wenn X Stickstoff ist, Y nicht Sauerstoff oder NR⁶ sein kann; und mit dem Ausschluss der folgenden Verbindungen:
(E)-N-Hydroxy-3-(4-{(E)-3-[4-(4-methyl-piperazin-1-yl-)phenyl-]3-oxo-propenyl-}phenyl-)acrylamid;
(E)-N-Hydroxy-3-{4-[(E)-3-(4-morpholin-4-yl-phenyl-)3-oxo-propenyl-]phenyl-}acrylamid;
(E)-3-{3-Fluor-4-[(E)-3-(4-morpholin-4-yl-phenyl-)3-oxo-propenyl-]phenyl-} N-hydroxy-acrylamid;

2. Verbindung nach Anspruch 1, worin eine oder mehrere der vorgenannten AlkylReste eine C₁- bis C₄-Alkyl-Gruppe ist/sind.

3. Verbindung nach Anspruch 1, worin
R¹, R² unabhängig voneinander für Wasserstoff, Fluor, Chlor, C₁- bis C₂-Alkyl oder CF₃ stehen;
R³, R⁴ unabhängig voneinander für Wasserstoff, C₁- bis C₂-Alkyl, Phenyl oder Benzyl stehen;
R⁵ für Wasserstoff, C₁- bis C₂-Alkyl, (CO)R⁷, SO₂-C₁- bis C₂-Alkyl, Phenyl oder Benzyl steht;
R⁶ für Wasserstoff, C₁- bis C₂-Alkyl oder Benzyl steht;
R⁷ für Wasserstoff, C₁- bis C₂-Alkyl, Phenyl, Benzyl, OR⁸ oder NR⁹R¹⁰ steht;
R⁸ für C₁- bis C₂-Alkyl steht;
R⁹ für Wasserstoff, C₁- bis C₂-Alkyl, Phenyl oder Benzyl steht;
R¹⁰ für Wasserstoff, C₁- bis C₂-Alkyl oder Benzyl steht;
R¹¹, R¹² unabhängig voneinander für Wasserstoff oder C₁- bis C₂-Alkyl stehen.

4. Verbindung nach Anspruch 1, die die Formel (Ia) aufweist 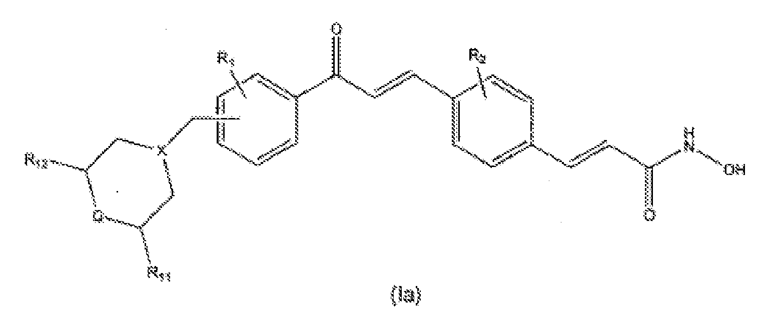 worin
Q für CH₂, CH-NR³R⁴ oder NR⁵ steht;
X für CH oder Stickstoff steht;
R¹, R² unabhängig voneinander für Wasserstoff, Halogen, C₁- bis C₄- Alkyl oder C₁- bis C₄-Haloalkyl stehen;
R³, R⁴ unabhängig voneinander für Wasserstoff oder C₁- bis C₄-Alkyl stehen;
R⁵ für Wasserstoff, C₁- bis C₄-Alkyl, (CO)R⁷, Phenyl oder Benzyl steht;
R⁷ für Wasserstoff, C₁- bis C₆-Alkyl, Phenyl, Benzyl, OR⁸ oder NR⁹R¹⁰ steht;
R⁸ für C₁- bis C₄-Alkyl steht;
R⁹, R¹⁰ unabhängig voneinander für Wasserstoff oder C₁- bis C₄-Alkyl stehen;
R¹¹, R¹² unabhängig voneinander für Wasserstoff oder C₁- bis C₄-Alkyl stehen;

5. Verbindung nach Anspruch 4, worin
Q für CH₂, CH-NR³R⁴ oder NR⁵ steht;
X für CH oder Stickstoff steht;
R¹, R² unabhängig voneinander für Wasserstoff, Fluor, Chlor oder CF₃
R³, R⁴ stehen; unabhängig voneinander für Wasserstoff oder C₁- bis C₂-Alkyl stehen;
R⁵ für Wasserstoff, C₁- bis C₄-Alkyl, (CO)R⁷, Phenyl oder Benzyl steht;
R⁷ für Wasserstoff, C₁- bis C₄-Alkyl, Phenyl, Benzyl, OR⁸ oder NR⁹R¹⁰ steht;
R⁸ für C₁- bis C₄-Alkyl steht;
R⁹,R¹⁰ unabhängig voneinander für Wasserstoff oder C₁- bis C₂-Alkyl stehen;
R¹¹, R¹² unabhängig voneinander für Wasserstoff oder C₁- bis C₂-Alkyl stehen;

6. Verbindung nach Anspruch 4, worin
Q für NR⁵ steht;
X für Stickstoff steht;
R¹, R² unabhängig voneinander für Wasserstoff, Fluor, Chlor oder CF₃ stehen;
R⁵ für Wasserstoff, C₁- bis C₄-Alkyl, (CO)R⁷, Phenyl oder Benzyl steht;
R⁷ für Wasserstoff, C₁- bis C₄-Alkyl, Phenyl, Benzyl, OR⁸ oder NR⁹R¹⁰ steht;
R⁸ für C₁- bis C₄-Alkyl steht;
R⁹, R¹⁰ unabhängig voneinander für Wasserstoff oder C₁- bis C₂-Alkyl stehen;
R¹¹, R¹² unabhängig voneinander für Wasserstoff oder C₁- bis C₂-Alkyl stehen.

7. Verbindung nach Anspruch 4, ausgewählt aus:
(E)-N-Hydroxy-3-(4-{(E)-3-[4-(4-methyl-piperazin-1-yl-methyl-)phenyl-]3-oxo-propenyl-}phenyl-)acrylamid;
(E)-3-(4-{(E)-3-[4-(4-Dimethylamino-piperidin-1-yl-methyl-)-phenyl-]3-oxo-propenyl-}phenyl-)-N-hydroxy-acrylamid;
(E)-N-Hydroxy-3-(4-{(E)-3[4-(1-methyl-piperidin-4-ylmethyl-)phenyl-]3-oxo-propenyl-}phenyl-)acrylamid;
(E)-N-Hydroxy-3-{4-[(E)-3-oxo-3-(4-piperazin-1-ylmethyl-phenyl-)propenyl-] phenyl-}acrylamid;
(E)-3-(4-{(E)-3-[4-(4-Benzyl-piperazin-1-ylmethyl-)phenyl-]3-oxo-propenyl-}-phenyl-)N-hydroxy-acrylamid;
(E)-3-(4-{(E)-3-[4-((3R,5S)-3,5-Dimethyl-piperazin-1-ylmethyl-)phenyl-]3-oxo-propenyl-}phenyl-)N-hydroxy-acrylamid;
(E)-3-(4-{(E)-3-[4-(4-Acetyl-piperazin-1-ylmethyl-)phenyl-]3-oxo-propenyl-} phenyl-)N-hydroxy-acrylamid;
(E)-3-(4-{(E)-3-[4-((3R,5S)-4-Acetyl-3,5-dimethyl-piperazin-1-ylmethyl-) phenyl-]3-oxo-propenyl-}phenyl-)N-hydroxy-acrylamid;
(E)-3-(4-{(E)-3-[4-(4-Ethyl-piperazin-1-ylmethyl-)phenyl-]3-oxo-propenyl-} phenyl-)N-hydroxy-acrylamid;
(E)-N-Hydroxy-3-(4-{(E)-3-[3-(4-methyl-piperazin-1-ylmethyl-)phenyl-]3-oxo-propenyl-}phenyl-acrylamid;
(E)-N-Hydroxy-3-(4-{(E)-3-[2-(4-methyl-piperazin-1-ylmethyl-)phenyl-]3-oxo-propenyl-}phenyl-)acrylamid;

8. Verbindung nach Anspruch 1, die die Formel (Ib) aufweist 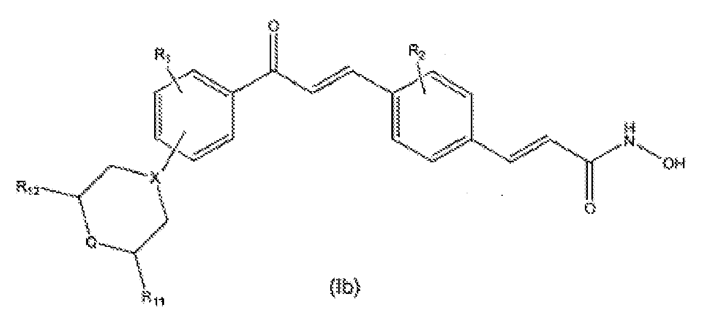 worin
Q für CH₂, CH-NR³R⁴ oder NR⁵ steht;
X für CH oder Stickstoff steht;
R¹, R² unabhängig voneinander für Wasserstoff, Halogen, C₁- bis C₄- Alkyl oder C₁- bis C₄-Haloalkyl stehen;
R³, R⁴ unabhängig voneinander für Wasserstoff oder C₁- bis C₄-Alkyl stehen;
R⁵ für Wasserstoff, C₁- bis C₄-Alkyl, (CO)R⁷, SO₂-C₁- bis C₄-Alkyl, Phenyl oder Benzyl steht;
R⁷ für Wasserstoff, C₁- bis C₆-Alkyl, Phenyl, Benzyl, OR⁸ oder NR⁹R¹⁰ steht;
R⁸ für C₁- bis C₄-Alkyl steht;
R⁹, R¹⁰ unabhängig voneinander für Wasserstoff oder C₁- bis C₄-Alkyl stehen;
R¹¹, R¹² unabhängig voneinander für Wasserstoff oder C₁- bis C₄-Alkyl stehen.
Mit dem Ausschluss der folgenden Verbindung:
(E)-N-Hydroxy-3-(4-{(E)-3-[4-(4-methyl-piperazin-1-yl-)phenyl-]3-oxo-propenyl-}phenyl-)acrylamid.

9. Verbindung nach Anspruch 8, worin in der Formel (Ib) die Gruppe: 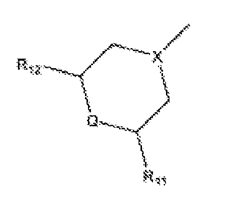 in ortho- oder meta-Position ist, bezogen auf den 3-oxo-Propenyl-Rest und:
Q für CH₂ oder NR⁵ steht;
X für CH oder Stickstoff steht;
R¹, R² unabhängig voneinander für Wasserstoff, Fluor, Chlor oder CF₃ stehen;
R⁵ für Wasserstoff, C₁- bis C₄-Alkyl, (CO)R⁷, Phenyl oder Benzyl steht;
R⁷ für Wasserstoff, C₁- bis C₄-Alkyl, Phenyl, Benzyl, OR⁸ oder NR⁹R¹⁰ steht;
R⁸ für C₁- bis C₄-Alkyl steht;
R⁹,R¹⁰ unabhängig voneinander für Wasserstoff oder C₁- bis C₂-Alkyl stehen;
R¹¹, R¹² unabhängig voneinander für Wasserstoff oder C₁- bis C₂-Alkyl stehen.

10. Verbindung nach Anspruch 8, ausgewählt aus:
(E)-3-(4-{(E)-3-[5-Chlor-2-(4-methyl-piperazin-1-yl-)phenyl-]3-oxo-propenyl-} phenyl-)N-hydroxy-acrylamid;
(E)-N-Hydroxy-3-(4-{ (E)-3-[2-(4-methyl-piperazin-1-yl-)phenyl-]3-oxo-propenyl-}phenyl-)acrylamid;
(E)-N-Hydroxy-3-(4-{(E)-3-[3-(4-methyl-piperazin-1-yl-)phenyl-]3-oxo-propenyl-}phenyl-)acrylamid;
(E)-N-Hydroxy-3-(4-{(E)-3-[4-(4-methylamino-piperidin-1-yl-)phenyl-]3-oxo-propenyl-}phenyl-)acrylamid;
(E)-3-(4-{(E)-3-[4-(4-Dimethylamino-piperidin-1-yl-)phenyl-]3-oxo-propenyl-} phenyl-)N-hydroxy-acrylamid;
(E)-N-Hydroxy-3-(4-{(E)-3-[4-(1-methyl-piperidin-4-yl-)phenyl-]3-oxo-propenyl-}phenyl-)acrylamid;
(E)-N-Hydroxy-3-(4-{(E)-3-[4-(4-isobutyl-piperazin-1-yl-)phenyl-]3-oxo-propenyl-}phenyl-)acrylamid;
(E)-3-(4-{(E)-3-[4-(4-Ethyl-piperazin-1-yl-)phenyl-]3-oxo-propenyl-}phenyl-) N-hydroxy-acrylamid;
(E)-3-(4-{(E)-3-[4-(4-Benzyl-piperazin-1-yl-)phenyl-]3-oxo-propenyl-}phenyl-) N-hydroxy-acrylamid;
(E)-N-Hydroxy-3-{4-[(E)-3-(4-piperazin-1-yl-phenyl-)3-oxo-propenyl-]phenyl-}acrylamid;
(E)-3-(4-{(E)-3-[4-(4-Benzoyl-piperazin-1-yl-)phenyl-]3-oxo-propenyl-}phenyl-)N-hydroxy-acrylamid;
(E)-3-(4-{(E)-3-[4-(4-Acetyl-piperazin-1-yl-)phenyl-]3-oxo-propenyl-}phenyl-) N-hydroxy-acrylamid;
(E)-N-Hydroxy-3-(4-{(E)-3-[4-(4-methansulfonyl-piperazin-1-yl-)phenyl-]3-oxo-propenyl-}phenyl-)acrylamid;
4-(4-{(E)-3-[4-((E)-2-Hydroxycarbamoyl-vinyl-)phenyl-]acryloyl-}phenyl-) piperazin-1-carbonsäure-dimethylamid;
4-(4-{(E)-3-[4-((E)-2-Hydroxycarbamoyl-vinyl-)phenyl-]acryloyl-}phenyl-) piperazin-1-carbonsäure-amid;
4-(4-{(E)-3-[4-((E)-2-Hydroxycarbamoyl-vinyl-)phenyl-]acryloyl-}phenyl-) piperazin-1-carbonsäure-ethylester;
(E)-N-Hydroxy-3-(4-{(E)-3-oxo-3-[4-((3R,5S)-3,4,5-trimethyl-piperazin-1-yl-) phenyl-]propenyl-}phenyl-)acrylamid;
(E)-3-(4-{(E)-3-(3-Chlor-5-(4-methyl-piperazin-1-yl-)phenyl-]3-oxo-propenyl-} phenyl-)N-hydroxy-acylamid;
(E)-3-(4-{(E)-3-[2-Chlor-5-(4-methyl-piperazin-1-yl-)phenyl-]3-oxo-propenyl-} phenyl-)N-hydroxy-acrylamid.

11. Verbindung nach Anspruch 1, die die Formel (Ic) aufweist 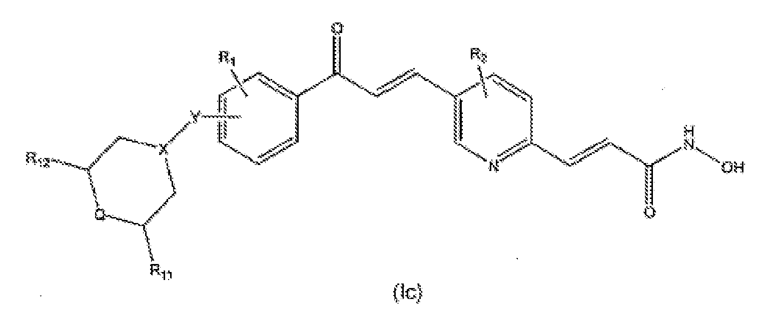 worin
Q für CH₂, CH-NR³R⁴, NR⁵ oder Stauerstoff steht;
X für CH oder Stickstoff steht;
Y für eine Bindung, CH₂, Sauerstoff oder NR⁶ steht;
R¹,R² unabhängig voneinader für Wasserstoff, Halogen, C₁- bis C₄- Alkyl oder C₁- bis C₄-Haloalkyl stehen;
R³,R⁴ unabhängig voneinander für Wasserstoff oder C₁- bis C₄-Alkyl stehen;
R⁵ für Wasserstoff, C₁- bis C₄-Alkyl, (CO)R⁷, Phenyl oder Benzyl steht;
R⁶ für Wasserstoff oder C₁- bis C₄-Alkyl steht;
R⁷ für Wasserstoff, C₁- bis C₆-Alkyl, Phenyl, Benzyl, OR⁸ oder NR⁹R¹⁰ steht;
R⁸ für C₁- bis C₄-Alkyl steht;
R⁹,R¹⁰ unabhängig voneinander für Wasserstoff oder C₁- bis C₄-Alkyl stehen;
R¹¹, R¹² unabhängig voneinander für Wasserstoff oder C₁- bis C₄-Alkyl stehen;
mit der Maßgabe, dass dann, wenn X Stickstoff ist, Y nicht Sauerstoff oder NR⁶ sein kann.

12. Verbindung nach Anspruch 11, worin:
Q für CH₂, NR⁵ oder Sauerstoff steht;
X für CH oder Stickstoff steht;
Y für eine Bindung oder CH₂ steht;
R¹, R² unabhängig voneinander für Wasserstoff, Fluor, Chlor oder CF₃ stehen;
R⁵ für Wasserstoff, C₁- bis C₂-Alkyl, (CO)R⁷, Phenyl oder Benzyl steht;
R⁷ für Wasserstoff, C₁- bis C₄-Alkyl, Phenyl, Benzyl, OR⁸ oder NR⁹R¹⁰ steht;
R⁸ für C₁- bis C₄-Alkyl steht;
R⁹,R¹⁰ unabhängig voneinander für Wasserstoff oder C₁- bis C₂-Alkyl stehen;
R¹¹, R¹² unabhängig voneinander für Wasserstoff oder C₁- bis C₂-Alkyl stehen;

13. Verbindung nach Anspruch 11, ausgewählt aus:
(E)-N-Hydroxy-3-(5-{(E)-3-[4-(4-methyl-piperazin-1-yl-)phenyl-]3-oxo-propenyl-}pyridin-2-yl-)acrylamid;
(E)-N-Hydroxy-3-(5-{(E)-3-[2-(4-methyl-piperazin-1-yl-)phenyl-]3-oxo-propenyl-}pyridin-2-yl-)acrylamid;
(E)-N-Hydroxy-3-(5-{(E)-3-[3-(4-methyl-piperazin-1-yl-)phenyl-]3-oxo-propenyl-}pyridin-2-yl-)acrylamid;
(E)-3-(5-{(E)-3-[4-(4-Benzyl-piperazin-1-yl-)phenyl-]3-oxo-propenyl-}pyridin-2-yl-)N-hydroxy-acrylamid;
(E)-N-Hydroxy-3-(5-{(E)-3-oxo-3-[4-((3R,5S)-3,4,5-trimethyl-piperazin-1-yl-) phenyl-]propenyl-}pyridin-2-yl-)acrylamid;
(E)-N-Hydroxy-3-{5-[(E)-3-[4-morpholin-4-ylmethyl-phenyl-]3-oxo-propenyl-] pyridin-2-yl-}acrylamid;
(E)-3-(5-{(E)-3-[4-(4-Ethyl-piperazin-1-yl-)phenyl-]3-oxo-propenyl-}pyridin-2-yl-)N-hydroxy-acrylamid;
(E)-3-(5-{(E)-3-[4-(4-Acetyl-piperazin-1-yl-)phenyl-]3-oxo-propenyl-}pyridin-2-yl-)N-hydroxy-acrylamid;
(E)-N-Hydroxy-3-(5-{(E)-3-oxo-3-[3-((3R,5S)-3,4,5-trimethyl-piperazin-1-yl-) phenyl-]propenyl-}pyridin-2-yl-)acrylamid;
(E)-N-Hydroxy-3-(5-{(E)-3-[4-(1-methyl-piperidin-4-ylmethyl-)phenyl-]3-oxo-propenyl-}pyridin-2-yl-)acrylamid;
(E)-N-Hydroxy-3-{5-[(E)-3-oxo-3-(4-piperazin-1-yl-phenyl-)propenyl-]pyridin-2-yl-}acrylamid;
(E)-N-Hydroxy-3-(5-{(E)-3-[2-(4-methyl-piperazin-1-ylmethyl-)phenyl-]-3-oxo-propenyl-}pyridin-2-yl-)acrylamid;
(E)-N-Hydroxy-3-{5-[(E)-3-oxo-3-(4-piperazin-1-ylmethyl-phenyl-)propenyl-] pyridin-2-yl-}acrylamid;
(E)-3-(5-{(E)-3-[4-(4-Acetyl-piperazin-1-ylmethyl-)phenyl-]3-oxo-propenyl-} pyridin-2-yl-)N-hydroxy-acrylamid;
(E)-N-Hydroxy-3-(5-{(E)-3-[4-(4-methyl-piperazin-1-ylmethyl-)phenyl-]3-oxo-propenyl-}pyridin-2-yl-)acrylamid;
(E)-3-(5-{(E)-3-[3-Chlor-5-(4-methyl-piperazin-1-yl-)phenyl-]3-oxo-propenyl-} pyridin-2-yl-)N-hydroxy-acrylamid;
(E)-N-Hydroxy-3-(5-{(E)-3-[3-(4-methyl-piperazin-1-ylmethyl-)phenyl-]3-oxo-propenyl-}pyridin-2-yl-)acrylamid;

14. Verfahren zum Erhalten der Verbindungen der Formel (I), wie in den Ansprüchen 1 bis 13 definiert, **gekennzeichnet durch** Behandeln einer Verbindung der Formel (II) 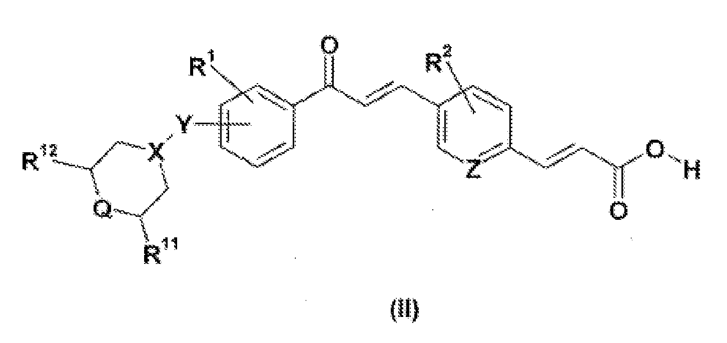 worin Q, X, Y, Z, R¹, R², R¹¹ und R¹² dieselben Bedeutungen haben, wie dies für die Formel (I) beschrieben wurde, mit einem geschützten Hydroxylamin, gefolgt von einem Schritt einer Hydroxylamin-Deprotektion.

15. Verfahren nach Anspruch 14, worin die Verbindung der Formel (II) erhalten wird von einer Verbindung der Formel (V) oder von einer Verbindung der Formel (XI) 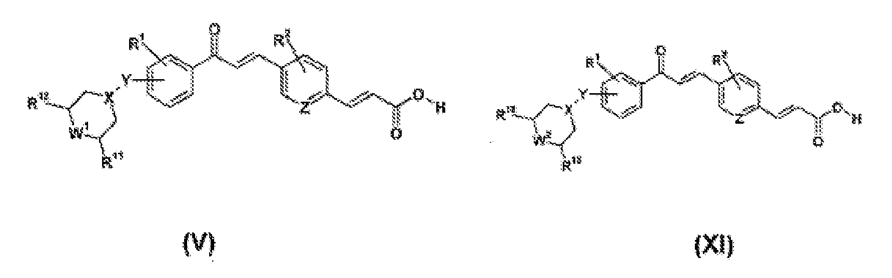 worin X, Y, R¹, R², R¹¹, R¹² und Z dieselben Bedeutungen haben, wie dies für die Formel (I) angegeben wurde, und W¹ für NH und W² für CO steht, behandelt mit Reagienzen, die in der Lage sind, die W¹- oder W²-Gruppe in Q zu überführen, wie in Formel (I) definiert.

16. Verfahren nach Anspruch 14, worin die Verbindung der Formel (II) erhalten wird durch Umsetzen einer Verbindung der Formel (IV) mit einer Verbindung der Formel (III) 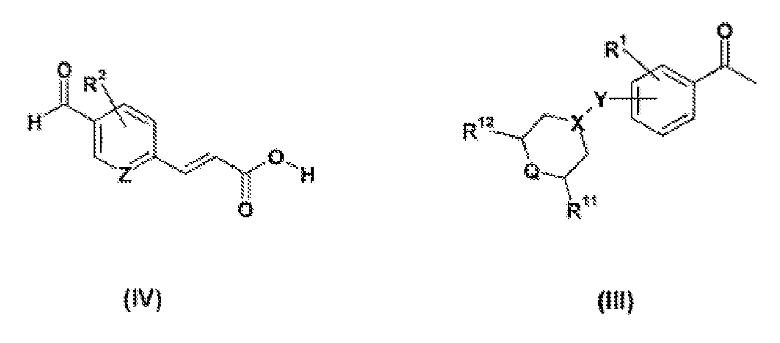 worin Q, X, Y, R¹, R², R¹¹, R¹² und Z dieselben Bedeutungen haben, wie dies für Formel (I) angegeben ist.

17. Verbindung aus den Formeln (I), (Ia), (Ib), (Ic), wie in den Ansprüchen 1 bis 13 definiert, zur Verwendung in der Therapie.

18. Pharmazeutische Zusammensetzung, umfassend eine oder mehrere Verbindung(en) der Formel (I), (Ia), (Ib), (Ic), wie in den Ansprüchen 1 bis 13 definiert, in Verbindung mit pharmazeutisch annehmbaren Trägerstoffen.

19. Zusammensetzung nach Anspruch 18 in Form einer Tablette, Kapsel, Pille, einer oralen Zubereitung, eines Pulvers, einer granulären Zubereitung, einer injizierbaren oder infundierbaren Lösung oder Suspension, eines Suppositoriums, einer wässrigen oder öligen Suspension, einer Lösung, einer Emulsion, eines Sirups, eines Elixiers, einer Creme, einer Salbe, einer Paste, eines Gels, einer Lösung, eines Öls oder einer Lotion, einer Membran oder eines medizinischen Pflasters.

20. Verwendung einer oder mehrerer Verbindung(en) der Formel (I), (Ia), (Ib), (Ic), wie in den Ansprüchen 1 bis 13 definiert, zur Herstellung eines Medikaments zur Vorbeugung und/oder Behandlung von Erkrankungen, die mit der Disregulation der Histon-Deacetylase-Aktivität verbunden sind.

21. Verwendung nach Anspruch 20, worin die Erkrankung Krebs ist.

## Revendications

1. Un composé de la formule (I) 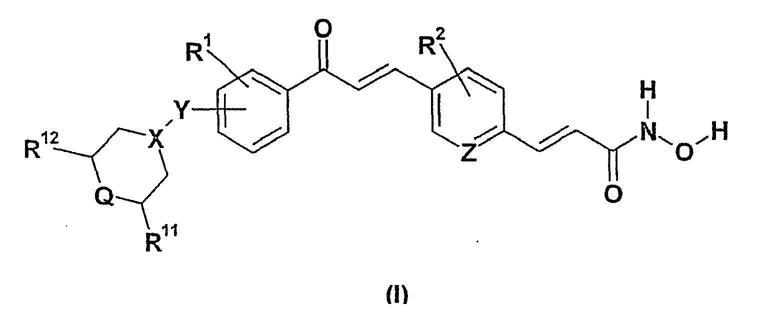 où:
Q est une liaison, CH₂, CH-NR³R⁴, NR⁵ ou oxygène;
X est CH ou azote;
Y est une liaison, CH₂, oxygène ou NR⁶;
Z est CH ou azote;
R¹, R² sont, indépendamment, hydrogène, halogène, alkyleC₁-C₆ ou haloalkyleC₁-C₆;
R³, R⁴ sont indépendamment, hydrogène, alkyleC₁-C₆, phényle ou benzyle;
R⁵ est hydrogène, alkyleC₁-C₆, (CO)R⁷, SO₂-alkyleC₁-C₆, phényle ou benzyle;
R⁶ est hydrogène, alkyleC₁-C₆ ou benzyle;
R⁷ est hydrogène, alkyleC₁-C₆, phényle, benzyle, OR⁸ ou NR⁹R¹⁰;
R⁸ est alkyle C₁-C₆;
R⁹ est hydrogène, alkyleC₁-C₆, phényle ou benzyle;
R¹⁰ est hydrogène, alkyleC₁-C₆ ou benzyle;
R¹¹, R¹² sont indépendamment hydrogène ou alkyleC₁-C₆;
et les sels pharmaceutiquement acceptables de celui-ci;
à condition que lorsque X est azote, Y ne peut pas être oxygène ou NR⁶;
et à l'exclusion des composés suivants:
(E)-N-Hydroxy-3-(4-{(E)-3-[4-(4-méthyl-pipérazin-1-yl)-phényl]-3-oxo-propényl}-phényl)-acrylamide;
(E)-N-Hydroxy-3-(4-[(E)-3-(4-morpholin-4-yl-phényl)-3-oxo-propényl-phényl}-acrylamide;
(E)-3-(3-Fluoro-4-[(E)-3-(4-morpholin-4-yl-phényl)-3-oxo-propényl]-phényl}-N-hydroxy-acrylamide.

2. Composé selon la revendication 1, dans lequel un ou plusieurs desdits alkyles précités est un groupe alkyle C₁-C₄.

3. Composé selon la revendication 1, dans lequel:
**R¹, R²** sont indépendamment hydrogène, fluore, chlore, alkyleC₁-C₂ ou CF₃;
**R³, R⁴** sont indépendamment hydrogène, alkyleC₁-C₂, phényle ou benzyle;
**R⁵** est hydrogène, alkyleC₁-C₂, (CO)R⁷, SO₂-alkyle C₁-C₂, phényle ou benzyle;
**R⁶** est hydrogène, alkyleC₁-C₂ ou benzyle;
**R⁷** est hydrogène, alkyleC₁-C₂, phényle, benzyle, OR⁸ ou NR⁹R¹⁰;
**R⁸** est alkyleC₁-C₂;
**R⁹** est hydrogène, alkyleC₁-C₂, phényle ou benzyle;
**R¹⁰** est hydrogène, alkyleC₁-C₂ ou benzyle;
**R¹¹, R¹²** sont indépendamment hydrogène ou alkyleC₁-C₂.

4. Composé selon la revendication 1, ayant la formule (Ia) 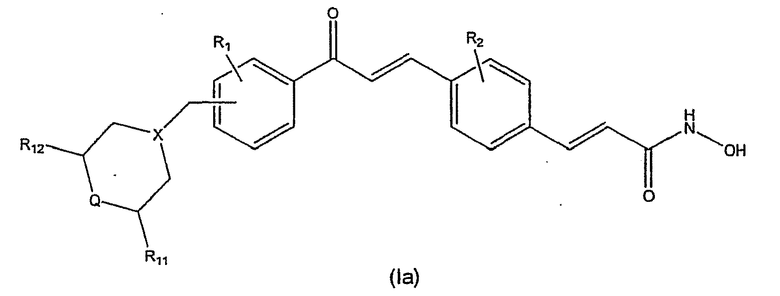 où:
**Q** est CH₂, CH-NR³R⁴ ou NR⁵;
**X** est CH ou azote;
**R¹, R²** sont indépendamment hydrogène, halogène, alkyle C₁-C₄ ou haloalkyleC₁-C₄;
**R³, R⁴** sont indépendamment hydrogène ou alkyleC₁-C₄;
**R⁵** est hydrogène, alkyleC₁-C₄, (CO)R⁷, phényle ou benzyle;
**R⁷** est hydrogène, alkyleC₁-C₆, phényle, benzyle, OR⁸ ou NR⁹R¹⁰;
**R⁸** est alkyleC₁-C₄;
**R⁹, R¹⁰** sont indépendamment hydrogène ou alkyleC₁-C₄;
**R¹¹, R¹²** sont indépendamment hydrogène ou alkyleC₁-C₄;

5. Composé selon la revendication 4, dans lequel:
**Q** est CH₂, CH-NR³R⁴ ou NR⁵;
**X** est CH ou azote;
**R¹, R²** sont indépendamment hydrogène, fluoro, chloro ou CF₃;
**R³, R⁴** sont indépendamment hydrogène ou alkyleC₁-C_{2;}
**R⁵** est hydrogène, alkyleC₁-C₄, (CO)R⁷, phényle ou benzyle;
**R⁷** est hydrogène, alkyleC₁-C₄, phényle, benzyle, OR⁸ ou NR⁹R¹⁰;
**R⁸** est alkyleC₁-C₄;
**R⁹, R¹⁰** sont indépendamment hydrogène ou alkyleC₁-C₂;
**R¹¹, R¹²** sont indépendamment hydrogène ou alkyleC₁-C₂.

6. Composé selon la revendication 4, où:
**Q** est NR⁵;
**X** est azote;
**R¹, R²** sont indépendamment hydrogène, fluoro, chloro ou CF₃;
**R⁵** est hydrogène, alkyleC₁-C₄, (CO)R⁷, phényle ou benzyle;
**R⁷** est hydrogène, alkyleC₁-C₄, phényle, benzyle, OR⁸ ou NR⁹R¹⁰;
**R⁸** est alkyle C₁-C₄;
**R⁹, R¹⁰** sont, indépendamment, hydrogène, hydrogène ou alkyleC₁-C₂;
**R¹¹, R¹²** sont, indépendamment, hydrogène ou alkyleC₁-C₂.

7. Composé selon la revendication 4, sélectionné parmi:
(E)-N-Hydroxy-3-(4-{(E)-3-[4-(4-méthyl-pipérazin-1-yl-méthyl)-phényl-3-oxo-propényl}-phényl)-acrylamide;
(E)-3-(4[(E)-3[4-(4-Diméthylamino-pipéridin-1-yl-méthyl)-phényl]-3-oxo-propényl}-phényl)-N-hydroxy-acrylamide;
(E)-N-Hydroxy-3-(4[(E)-3-[4-(1-méthyl-pipéridin-4-ylméthyl)-phényl]-3-oxo-propényl}-phényl)-acrylamide;
(E)-N-Hydroxy-3-{4-[(E)-3-oxo-3-(4-pipérazin-1-ylméthyl-phényl)-propényl}-phényl}-acrylamide;
(E)-3-(4-{(E)-3-[4-(4-Benzyl-pipérazin-1-ylméthyl)-phényl-3-oxo-propényl}-phényl)-N-hydroxy-acrylamide;
(E)-3-(4-{(E)-3-[4-((3R,5S)-3,5-Diméthyl-pipérazin-1-ylméthyl)-phényl]-3-oxo-propényl}-phényl)-N-hydroxy-acrylamide;
(E)-3-(4-{(E)-3-[4-(4-Acétyl-pipérazin-1-ylméthyl)-phényl]-3-oxo-propényl}-phényl)-N-hydroxy-acrylamide;
(E)-3-(4-{(E)-3-[4-((3R,5S)-4-Acétyl-3,5-diméthyl-pipérazin-1-ylméthyl)-phényl]-3-oxo-propényl}-phényl)-N-hydroxy-acrylamide;
(E)-3-(4-{(E)-3-[4-(4-Ethyl-pipérazin-1-ylméthyl)-phényl]-3-oxo-propényl-phényl)-N-hydroxy-acrylamide;
(E)-N-Hydroxy-3-(4-{(E)-3-[3-(4-méthyl-pipérazin-1-ylméthyl)-phényl]-3-oxo-propényl}-phényl)-acrylamide;
(E)-N-Hydroxy-3-(4-[(E)-3-[2-(4-méthyl-pipérazin-1-ylméthyl)-phényl]-3-oxo-propényl}-phényl)-acrylamide.

8. Composé selon la revendication 1, ayant la formule **(Ib)** 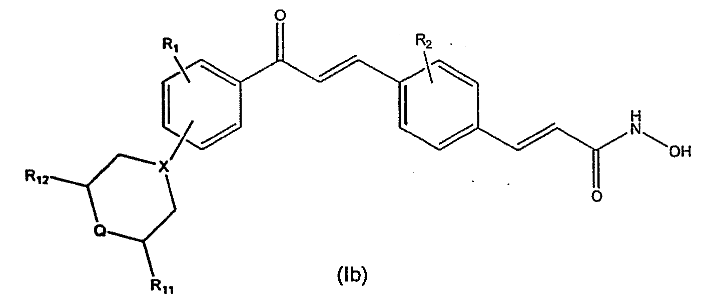 où:
Q est CH₂, CH-NR³R⁴ ou NR⁵;
X est CH ou azote;
R¹, R² sont, indépendamment, hydrogène, halogène, alkyle-C₁-C₄ ou haloalkyle C₁-C₄;
R³, R⁴ sont, indépendamment, hydrogène ou alkyle C₁-C₄;
R⁵ est hydrogène, alkyleC₁-C₄, (CO) R⁷, SO₂alkyle-C₁-C₄, phényle ou benzyle;
R⁷ est hydrogène, alkyleC₁-C₆, phényle, benzyle, OR⁸ ou NR⁹R¹⁰;
R⁸ est alkyleC₁-C₄;
R⁹, R¹⁰ sont indépendamment hydrogène ou alkyleC₁-C₄;
R¹¹, R¹² sont indépendamment hydrogène ou alkyleC₁-C₄;
à l'exclusion du composé suivant: (E)-N-Hydroxy-3-(4-{(E)-3-[4-(4-méthyl-pipérazin-1-yl)-phényl]-3-oxo-propényl}-phényl)-acrylamide.

9. Composé selon la revendication 8, dans lequel, dans ladite formule (Ib), le groupe: 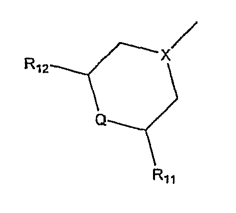 est en position ortho ou méta, par rapport au fragment 3-oxo-propényle, et:
**Q** est CH₂ ou NR⁵;
**X** est CH ou azote;
**R¹, R²** sont indépendamment hydrogène, fluoro, chloro ou CF₃;
**R⁵** est hydrogène, alkyleC₁-C₄, (CO)R⁷, phényle ou benzyle;
**R⁷** est hydrogène, alkyleC₁-C₄, phényle, benzyle, OR⁸ ou NR⁹R¹⁰;
**R⁸** est alkyleC₁-C₄;
**R⁹, R¹⁰** sont indépendamment hydrogène ou alkyleC₁-C₂;
**R¹¹, R¹²** sont indépendamment hydrogène ou alkyleC₁-C₂.

10. Composé selon la revendication 8, sélectionné parmi:
(E)-3-(4-{(E)-3-[5-Chloro-2-(4-méthyl-pipérazin-1-yl)-phényl}-3-oxo-propényl}-phényl)-N-hydroxy-acrylamide;
(E)-N-Hydroxy-3-(4-{(E)-3-[2-(4-méthyl-pipérazin-1-yl)-phényl]-3-oxo-propényl}-phényl)-acrylamide;
(E)-N-Hydroxy-3-(4-{(E)-3-[3-(4-méthyl-pipérazin-1-yl)-phényl]-3-oxo-propényl-phényl)-acrylamide;
(E)-N-Hydroxy-3-(4-{(E)-3-[4-(4-méthylamino-pipéridin-1-yl)-phényl]-3-oxo-propényl}-phényl)-acrylamide;
(E)-3-(4-{(E)-3-[4-(4-Diméthylamino-pipéridin-1-yl)-phényl]-3-oxo-propényl]-phényl)-N-hydroxy-acrylamide;
(E)-N-Hydroxy-3-(4-{(E)-3-[4-(1-méthyl-pipéridin-4-yl)-phényl]-3-oxo-propényl}-phényl)-acrylamide;
(E)-N-Hydroxy-3-(4-{(E)-3-[4-(4-isobutyl-pipérazin-1-yl)-phényl]-3-oxo-propényl}-phényl)-acrylamide;
(E)-3-(4-{(E)-3-[4-(4-Ethyl-pipérazin-1-yl)-phényl]-3-oxo-propényl}-phényl)-N-hydroxy-acrylamide;
(E)-3-(4-{(E)-3-4-(4-Benzyl-pipérazin-1-yl)-phényl]-3-oxo-propényl}-phényl)-N-hydroxy-acrylamide;
(E)-N-Hydroxy-3-{4-[(E)-3-(4-pipérazin-1-yl-phényl)-3-oxo-propényl]-phényl}-acrylamide;
(E)-3-(4-{(E)-3-[4-(4-Benzoyl-pipérazin-1-yl)-phényl]-3-oxo-propényl}-phényl)-N-hydroxy-acrylamide;
(E)-3-(4-{(E)-3-[4-(4-Acétyl-pipérazin-1-yl)-phény]-3-oxo-propényl}-phényl)-N-hydroxy-acrylamide;
(E)-N-Hydroxy-3-(4-{(E)-3-[4-(4-méthanesulfonyl-pipérazin-1-yl)-phényl]-3-oxo-propényl}-phényl)-acrylamide;
Diméthylamide de l'acide 4-(4-{(E)-3-[4-((E)-2-hydroxycarbamoyl-vinyl)-phényl]-acryloyl]-phényl)-pipérazine-1-carboxylique;
Amide de l'acide 4-(4-{(E)-3-[4-((E)-2-hydroxycarbamoyl-vinyl)-phényl]-acryloyl}-phényl)-pipérazine-1-carboxylique;
Ethyl ester de l'acide 4-(4-{(E)-3-[4-((E)-2-hydroxycarbamoyl-vinyl)-phényl-acryloyl}-phényl)-pipérazine carboxylique;
(E)-N-Hydroxy-3-(4-{(E)-3-oxo-3-[4-((3R,5S)-3,4,5-triméthyl-pipérazin-1-yl)-phényl]-propényl}-phényl)-acrylamide,
(E)-3-(4-[(E)-3-[3-Chloro-5-(4-méthyl-pipérazin-1-yl)-phényl]-3-oxo-propényl}-phényl)-N-hydroxy-acylamide;
(E)-3-(4-{(E)-3-[2-Chloro-5-(4-méthyl-pipérazin-1-yl)-phényl]-3-oxo-propényl}-phényl)-N-hydroxy-acrylamide.

11. Composé selon la revendication 1, ayant la formule (Ic) 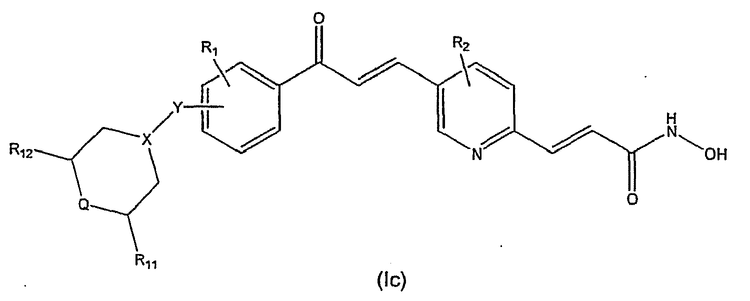 où:
Q est CH₂, CH-NR³R⁴, NR⁵ ou oxygène;
X est CH ou azote;
Y est une liaison, CH₂, oxygène ou NR⁶;
R¹, R² sont, indépendamment, hydrogène, halogène, alkyleC₁-C₄ ou haloalkyleC₁-C₄;
R³, R⁴ sont, indépendamment, hydrogène ou alkyleC₁-C₄;
R⁵ est hydrogène, alkyleC₁-C₄, (CO)R⁷, phényle oubenzyle;
R⁶ est hydrogène ou alkyleC₁-C₄;
R⁷ est hydrogène, alkyleC₁-C₆, phényle, benzyle, OR⁸ ou NR⁹R¹⁰;
R⁸ est alkyleC₁-C₄;
R⁹, R¹⁰ sont, indépendamment, hydrogène ou alkyle C₁-C₄;
R¹¹, R¹² sont, indépendamment, hydrogène ou alkyle C₁-C₄;
à condition que lorsque X est azote, Y ne peut pas être oxygène ou NR⁶.

12. Composé selon la revendication 11, où:
**Q** est CH₂, NR⁵ ou oxygène;
**X** est CH ou azote;
**Y** est une liaison ou CH₂;
**R¹, R²** sont indépendamment hydrogène, fluoro, chloro ou CF₃;
**R⁵** est hydrogène, alkyleC₁-C₂, (CO) R⁷, phényle ou benzyle;
**R⁷** est hydrogène, alkyleC₁-C₄, phényle, benzyle, OR⁸ ou NR⁹R¹⁰;
**R⁸** est alkyleC₁-C₄;
**R⁹, R¹⁰** sont indépendamment hydrogène ou alkyleC₁-C₂;
**R¹¹, R¹²** sont indépendamment hydrogène ou alkyleC₁-C₂.

13. Composé selon la revendication 11, sélectionné parmi:
(E)-N-Hydroxy-3-(5-{(E)-3[4-(4-méthyl-pipérazin-1-yl)-phényl]-3-oxo-propényl}-pyridin-2-yl)-acrylamide;
(E)-N-Hydroxy-3-(5-{(E)-3-[2-(4-méthyl-pipérazin-1-yl)-phényl]-3-oxo-propényl}-pyridin-2-yl)-acrylamide;
(E)-N-Hydroxy-3-(5-{(E)-3-[3-(4-méthyl-pipérazin-1-yl)-phényl]-3-oxo-propényl}-pyridin-2-yl)-acrylamide;
(E)-3-(5-{(E)-3-[4-(4-Benzyl-pipérazin-1-yl)-phényl]-3-oxo-propényl}-pyridin-2-yl)-N-hydroxy-acrylamide;
(E)-N-Hydroxy-3-(5-{(E)-3-oxo-3[4-((3R,5S)-3,4,5-triméthyl-pipérazin-1-yl)-phényl]-propényl}-pyridin-2-yl)-acrylamide,
(E)-N-Hydroxy-3-{5-[(E)-3-(4-morpholin-4-ylméthyl-phényl)-3-oxo-propényl}-pyridin-2-yl}-acrylamide;
(E)-3-(5-{(E)-3-[4-(4-Ethyl-pipérazin-1-yl)-phényl]-3-oxo-propényl}-pyridin-2-yl)-N-hydroxy-acrylamide;
(E)-3-(5-{(E)-3-[4-(4-Acétyl-pipérazin-1-yl)-phényl]-3-oxo-propényl}-pyridin-2-yl)-N-hydroxy-acrylamide;
(E)-N-Hydroxy-3-(5-{(E)-3-oxo-3-[3-((3R,5S)-3,4,5-triméthyl-pipérazin-1-yl)-phényl}-propényl-pyridin-2-yl)-acrylamide;
(E)-N-Hydroxy-3-(5-{(E)-3-[4-(1-méthyl-pipéridin-4-ylméthyl)-phényl]-3-oxo-propényl]-pyridin-2-yl)-acrylamide;
(E)-N-Hydroxy-3-{5-[(E)-3-oxo-3-(4-pipérazin-1-yl-phényl)-propényl-pyridin-2-yl}-acrylamide;
(E)-N-Hydroxy-3-(5-{(E)-3-[2-(4-méthyl-pipérazin-1-ylméthyl)-phényl]-3-oxo-propényl}-pyridin-2-yl)-acrylamide;
(E)-N-Hydroxy-3-{5-[(E)-3-oxo-3-(4-pipérazin-1-ylméthyl-phényl)-propényl]-pyridin-2-yl}-acrylamide;
(E)-3-(5-{(E)-3-[4-(4-Acétyl-pipérazin-1-ylméthyl)-phényl]-3-oxo-propényl]-pyridin-2-yl)-N-hydroxy-acrylamide;
(E)-N-Hydroxy-3-(5-{(E)-3-[4-(4-méthyl-pipérazin-1-ylméthyl)-phényl]-3-oxo-propényl-pyridin-2-yl)-acrylamide;
(E)-3-(5-{(E)-3[3-Chloro-5-(4-méthyl-pipérazin-1-yl)-phényl]-3-oxo-propényl}-pyridin-2-yl)-N-hydroxy-acrylamide;
(E)-N-Hydroxy-3-(5-{(E)-3-[3-(4-méthyl-pipérazin-1-ylméthyl-phényl]-3-oxo-propényl-pyridin-2-yl)-acrylamide.

14. Procédé d'obtention des composés de la formule **(I)** tels que définis dans les revendications 1 à 3, **caractérisé en** traitant un composé de la formule **(II)** 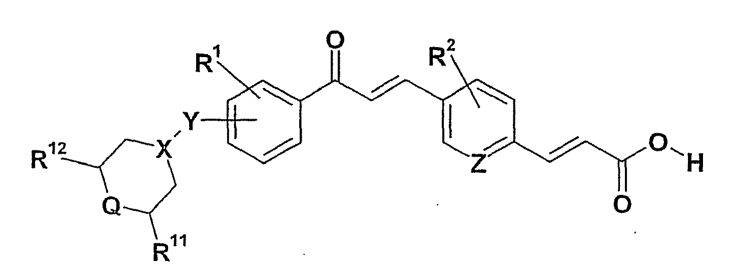 où **Q, X, Y, Z, R¹, R², R¹¹ et R¹²** ont les mêmes significations que décrites pour la formule (I), avec une hydroxylamine protégée, suivie d'une étape de déprotection d'hydroxylamine.

15. Procédé selon la revendication 14, où le composé de la formule **(II)** est obtenu à partir d'un composé de la formule **(V)** ou d'un composé de la formule **(XI)** 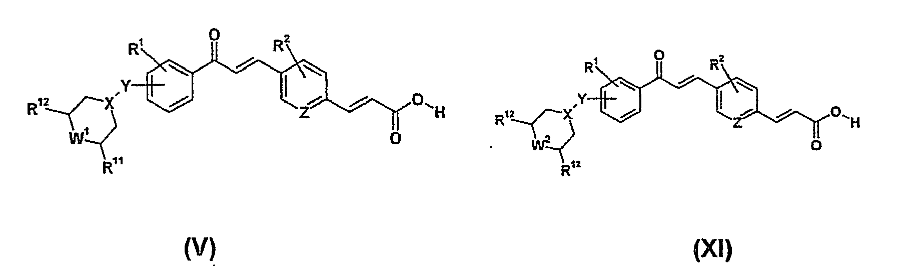 où **X, Y, R¹, R², R¹¹, R¹² et Z** ont les mêmes significations que celles indiquées pour la formule (I), et **W¹** est NH, et **W²** est CO, traité avec des réactifs aptes à convertir le groupe **W¹** ou **W²** en Q, comme défini dans la formule **(I).**

16. Procédé selon la revendication 14, où le composé de la formule **(II)** est obtenu en faisant réagir un composé de la formule **(IV)** avec un composé de la formule **(III).** 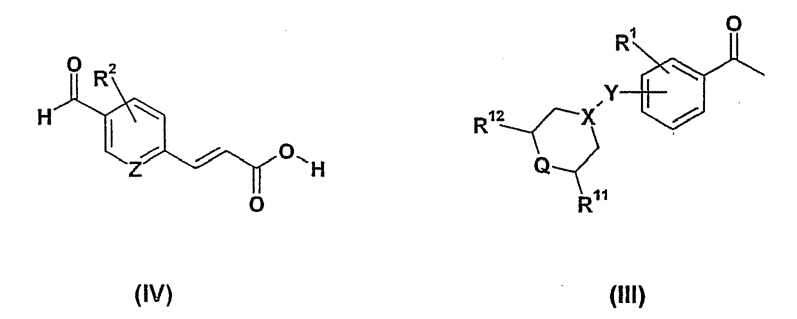 Où **Q, X, Y, R¹, R², R¹¹, R¹²** et **Z** ont les mêmes significations que celles indiquées pour la formule (I).

17. Composé de la formule **(I), (Ia), (Ib), (Ic)** tel que défini dans les revendications 1 à 13, pour utilisation en thérapie.

18. Composition pharmaceutique comprenant un ou plusieurs composés de la formule **(I), (Ia), (Ib), (Ic)** tels que définis dans les revendications 1-13, en association avec des excipients pharmaceutiquement acceptables.

19. Composition selon la revendication 18, sous la forme d'un comprimé, capsule, pilule, préparation orale, poudre, préparation granulaire, solution injectable ou infusible ou suspension, suppositoire, suspension aqueuse ou huileuse, solution, émulsion, sirop, élixir, crème, onguent, pâte, gel, solution, huile ou lotion, membrane ou timbre médicinal.

20. Utilisation d'un ou de plusieurs composés de la formule **(I), (Ia), (Ib), (Ic)** tels que définis dans les revendications 1 à 13, dans la préparation d'un médicament pour prévenir et/ou traiter des maladies liées au dérèglement de l'activité de l'histone désacétylase.

21. Utilisation selon la revendication 20, où ladite maladie est le cancer.
